# EUROPEAN PATENT APPLICATION

(11) **EP 3 124 588 A1**
(43) Date of publication of application: **01.02.2017**
(21) Application number: 15768357.4
(22) Date of filing: 20.03.2015
(51) Int. Cl.: C12M 1/00, C12M 1/34, G01N 21/17

(54) **CELL OBSERVATION INFORMATION PROCESSING SYSTEM, AND CELL OBSERVATION INFORMATION PROCESSING METHOD**

(30) Priority: 27.03.2014 JP 2014066109
(71) Applicant: OLYMPUS CORPORATION, Hachioji-shi Tokyo 192-8507 (JP)
(72) Inventor: IGA, Yasunobu, Tokyo 192-8507 (JP); TAKIMOTO, Shinichi, Tokyo 192-8507 (JP); TANIKAWA, Yohei, Tokyo 192-8507 (JP)
(74) Representative: Schicker, Silvia
(86) International application number: PCT/JP2015/058494
(87) International publication number: WO 2015/146833

(57) **Abstract**

[Object] A cell observation information processing system is provided that can prevent users' errors of inputting the passage number that is one of tags to be assigned to cell observation information, such as of cells.

[Measures for solution] The system includes: a passage presence/absence information acquiring and generating section 11a that acquires or generates previous passage presence/absence information including at least one passage presence/absence value among first to (n-1) -th passage presence/absence values indicating whether work for cells was passage work at first to (n-1)-th observation time points, among first to n-th observation time points (n is an integer equal to or greater than two) at which first to n-th pieces of observation information each indicating a cell observation result including a cell image, activity data such as the number of cells, confluency, morphology and viability, and an observation name are acquired in a time series manner through an observation information acquiring section 20; and a passage number acquiring and generating section 11b that acquires or generates at least the n-th passage number at the n-th observation time point, automatically or through an operation by a user, based on previous passage presence/absence information acquired or generated by the passage presence/absence information acquiring and generating section.

## Description

### TECHNICAL FIELD

The present invention relates to a cell observation information processing system, a cell observation information processing method, a cell observation information processing program, an archive section provided for the cell observation information processing system, and apparatuses provided for the cell observation information processing system, each of which is to be used for condition management of cells and the like to be used for research of living object upon use of observation information on the cells (images and activity data) acquired by an observation information acquiring apparatus such as a microscope.

### BACKGROUND ART

In the fields where experiment and research using cells are to be made, users continue culturing cells on a daily basis and control, through observation of cell images via microscopes and the like, conditions of the cells to be used for the experiment and research.

In culturing cells, passage work and non-passage work, which are maintenance work for maintaining and managing cells, are appropriately performed at appropriate timings.

The passage work is work of transplanting proliferated cells to another container every few days.

The non-passage work is work other than the passage work, and includes cell condition confirmation and culture solution replacement.

Conventionally, cell conditions have been recognized by eye observation, through microscopes, of cells in culture containers by users. Finding variations of the cell conditions has been entrusted to memory and sense of users. However, entrusting it to the users' memory and sense would hinder accurate recognition of a long-term variation of the cell conditions, to lead to failure in enhancing accuracy of experiment and research. For accurate recognition of long-term variations of cell conditions, it is necessary to take images of the cells at time points, generate activity data such as the confluency, morphology and a viability on the basis of the taken images, assign search tags to the images of cells and observation information, such as activity data, and register the tagged information in database files, thereby allowing the information to be compiled into a database and to be searched.

The aforementioned passage number is required to be stored, as a search tag for searching observation information on cells having been complied into the database, in a recording means, such as a magnetic medium.

Conventionally, in fields of managing cell condition, techniques of recording the passage number include, for example, the following Patent Document 1, which describes that identifying information for uniquely identifying the biological specimen, such as the passage number, is stored in an IC chip provided for a biological specimen container useful for managing the traceability of a biological specimen, such as a living object.

### PRIOR ART DOCUMENTS

### PATENT DOCUMENTS

Patent Document 1: Japanese Patent Laid-Open No. 2009-162648

### SUMMARY OF THE INVENTION

### PROBLEMS TO BE SOLVED BY THE INVENTION

In the case of compiling pieces of cell observation information at respective time points into a database in order to manage the condition of cells used for experiment and research, the passage number is incremented by one every time of passage work for cells. Meanwhile, an image of cells at the current observation day when passage work is to be conducted is obtained before the passage work. As a result, an observation day when the passage number of cellular specimens is incremented and stored in a database file deviates from an observation day when the passage work is actually conducted.

Consequently, if a user tries to input the passage number manually through a screen or the like where a search tag to be assigned to observation information is set, on the observation day when the user conducted predetermined maintenance work for cells on the basis of memory and recognition of the maintenance work having been conducted for cells at the last observation time point, misunderstanding on passage number counting and misunderstanding on the details of maintenance work conducted for cells occur, and the passage number is thus prone to be erroneously input.

The problems are hereinafter described in detail.

As stated above, the maintenance work conducted by users for cells is classified into passage work and non-passage work. Timings for conducting passage work and non-passage work are different from each other as follows.

The user would check conditions of cells basically by checking the cells every few days. The checking every few days allows the user to recognize a macro variation of cell conditions. Regarding the cell conditions inside the culture container, when the user captures an image of the cells and confirms that the activity data of the cells acquired from the captured image has reached a predetermined upper threshold, such as the cell confluency and the number of cells, he or she conducts maintenance work for passage. On the next observation day of an observation day when passage work was conducted, the activity data acquired by taking images of cells in a new culture container falls short of a lower threshold, such as the predetermined confluency and the number of cells. On the observation day after the next observation day of that when passage work was conducted, the activity data acquired by capturing images of cells in the culture container becomes equal to or exceeds the predetermined lower threshold, such as the confluency and the number of cells.

Regarding the cell condition in the culture container, during a period in which activity data, such as the predetermined confluency and the number of cells does not reach the upper threshold as to require the passage work, the user conducts non-passage work and checks the daily cell condition using the cell activity data.

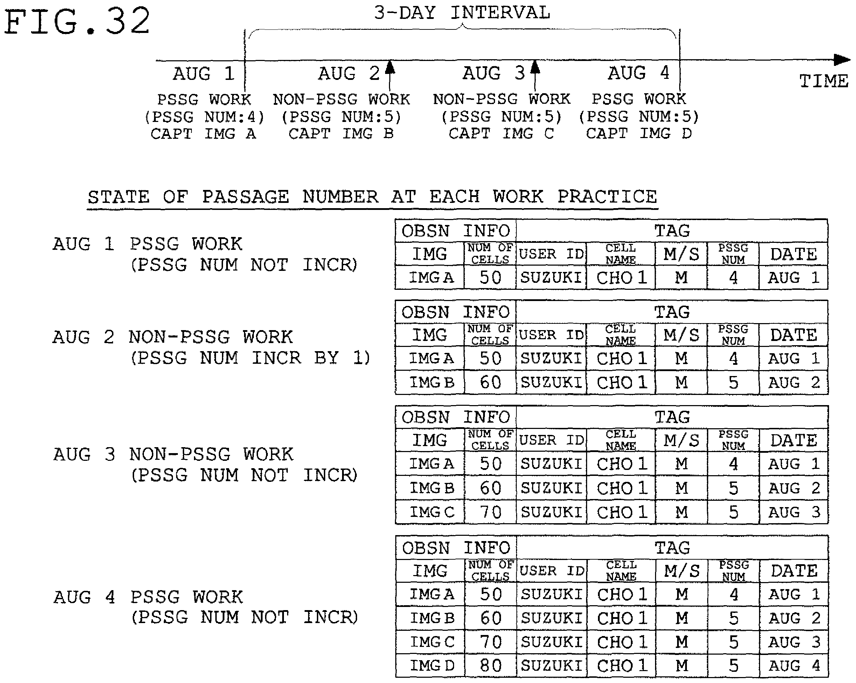

FIG. 32 is an explanatory diagram that shows an example of the relationship between the maintenance work for cells and timing of incrementing the passage number. Here, for convenience' sake, an example is shown, assuming that the cell condition is checked on a daily basis.

The example of FIG. 32 shows maintenance work for cells on each of observation days from Aug. 1 to Aug. 4, and data containing observation information and a passage number assigned as a search tag to the observation information in each of records in a database file corresponding to each practice of the maintenance work.

In the example of FIG. 32, the user conducts the works described below.

On Aug. 1, the user conducts passage work. In this case, the user captures an image of cells before passage work as an image A, assigns a user ID, a cell name, cell level information for identifying whether the cells are of a parent cell line or of a subculture, the passage number, and the date, which serve as search tags for retrieving the observation information, to the observation information containing the image A and the number of cells, and stores the information in a database file. Here, the passage number at the time of storage in the database file is assumed as "4". Subsequently, the user conducts passage work, that is, thins out a certain amount of cells in the culture container, and newly transplant the cells in a new culture container.

On Aug. 2, the user conducts non-passage work. The user takes an image of the cells in the new culture container after acquisition due to the passage work on Aug. 1 as an image B, assigns a search tag similar to that stated above to observation information containing the image B and the number of cells, and stores the information in the database file. This image B is an image of cells after the passage work. Consequently, when the information is stored in the database file, the value of passage number assigned as the tag to the image B is incremented by one to "5".

Also on Aug. 3, the cell activity (the number of cells in this example) does not reach the upper threshold. Consequently, as with Aug. 2, the user conducts non-passage work.

On Aug. 4, the cell activity (the number of cells) reaches the upper threshold. Consequently, as with Aug. 1, the user conducts passage work.

In the case where the passage work and non-passage work are conducted at the timings exemplified in FIG. 32, manual input by the user, for example through an input screen, of the passage number which is one of search tags to be assigned to observation information to be registered in the database file on the corresponding observation day causes the following problem.

As apparent in the example of FIG. 32, on the observation day on which the passage work is conducted, the search tag is assigned to the observation information before passage work and the information is stored in the database file. Consequently, the passage number is not incremented. On the observation day on which the first non-passage work after the passage work is conducted, the passage number is incremented when the search tag is assigned to the cell observation information and the information is registered in the database file.

That is, the observation day on which the passage work is conducted for the cells does not coincide with the observation day on which the passage number is incremented and stored in the database file. The observation days on which the non-passage work is conducted for the cells may include observation days on which the passage number is incremented and observation days on which the passage number is not incremented when the information is stored in the database file. In particular, in the case where the non-passage work was conducted at the last observation time point, the passage number on the current observation day is the same as the passage number at the last observation time point, irrespective of whether the work to the cells is the passage work or non-passage work. On the other hand, in the case where the passage work was conducted at the last observation time point, the passage number on the current observation day has the value obtained by incrementing the value of passage number at the last observation time point by one.

Consequently, manual input of the passage number based on the memory and recognition of the user on the work conducted to the cells on the current observation day in a situation of storing the passage number in the database file makes the user be prone to misunderstanding about the passage number and work details.

For example, in the example of FIG. 32, a situation may occur where even if the user recognizes that the user conducted the non-passage work on Aug. 2, he or she misunderstands about the value of passage number itself and erroneously inputs "4", where "5" should be correctly input instead.

For example, in the example of FIG. 32, a situation may occur where even though the user conducted the passage work on Aug. 1, he or she misunderstands the work details such that he or she has conducted the first non-passage work after the passage work, and erroneously inputs "5" as the value of the passage number, where "4" should be correctly input instead.

For example, a situation may occur where even though the user conducted the non-passage work on Aug. 2, he or she misunderstands such that he or she has conducted the passage work, and erroneously input "4" as the value of the passage number, where "5" should be correctly input instead.

Unfortunately, Patent Document 1 only discloses that the passage number is stored in an IC chip, but does not disclose a problem of erroneous input of the passage number in a situation where the user registers data nor a method of solving the problem.

Consequently, in a field where cell information is compiled into a database for condition management of cells to be used for research of living object, there is a room for improvement in prevention of erroneous input of the passage number by the user during compilation into the database.

Some aspects of the present invention have been proposed to solve the conventionalproblems. The presentinvention hasan object to provide a cell observation information processing system, a cell observation information processing method, a cell observation information processing program, an archive section provided for the cell observation information processing system, and apparatuses provided for the cell observation information processing system that can prevent erroneous input of the passage number, which is one of tags to be assigned to observation information, such as on cells, by the user.

### MEASURES TO SOLVE THE PROBLEMS

To achieve the above object, a cell observation information processing system according to an aspect of the present invention includes: a passage presence/absence information acquiring and generating section that acquires or generates previous passage presence/absence information including at least one passage presence/absence value among first to (n-1)-th passage presence/absence values indicating whether work for cells was passage work at first to (n-1)-th observation time points, among first to n-th observation time points at which first to n-th pieces of observation information including items indicating a cell observation result including a cell image, activity data indicating the cell activity such as the number of cells, confluency, morphology and viability, and an observation name are acquired in a time series manner through an observation information acquiring section; and a passage number acquiring and generating section that acquires or generates at least an n-th passage number at the n-th observation time point automatically or through an operation by a user, based on the previous passage presence/absence information acquired or generated by the passage presence/absence information acquiring and generating section. Note that n is an integer equal to or greater than two, and the current observation time point is represented by the n-th observation time point.

A cell observation information processing method according to another aspect of the present invention includes: a passage presence/absence information acquiring and generating step of acquiring or generating previous passage presence/absence information including at least one passage presence/absence value among first to (n-1) -th passage presence/absence values indicating whether work for cells was passage work at first to (n-1)-th observation time points, among first to n-th observation time points at which first to n-th pieces of observation information each including items indicating a cell observation result including a cell image, activity data indicating the cell activity such as the number of cells, confluency, morphology and viability, and an observation name are acquired in a time series manner through an observation information acquiring section; and a passage number acquiring and generating step of acquiring or generating at least an n-th passage number at the n-th observation time point automatically or through an operation by a user, based on the acquired or generated previous passage presence/absence information. Note that n is an integer equal to or greater than two, and the current observation time point is represented by the n-th observation time point.

A cell observation information processing program according to still another aspect of the present invention causes a computer to function as: a passage presence/absence information acquiring and generating means for acquiring or generating previous passage presence/absence information including at least one passage presence/absence value among first to (n-1)-th passage presence/absence values indicating whether work for cells was passage work at first to (n-1)-th observation time points, among first to n-th observation time points at which first to n-th pieces of observation information each including items indicating a cell observation result including a cell image, activity data indicating the cell activity such as the number of cells, confluency, morphology and viability, and an observation name are acquired in a time series manner through an observation information acquiring section; and a passage number acquiring and generating means for acquiring or generating an n-th passage number at the n-th observation time point automatically or through an operation by a user, based on the previous passage presence/absence information acquired by the passage presence/absence information acquiring and generating means.

### BRIEF DESCRIPTION OF THE DRAWINGS

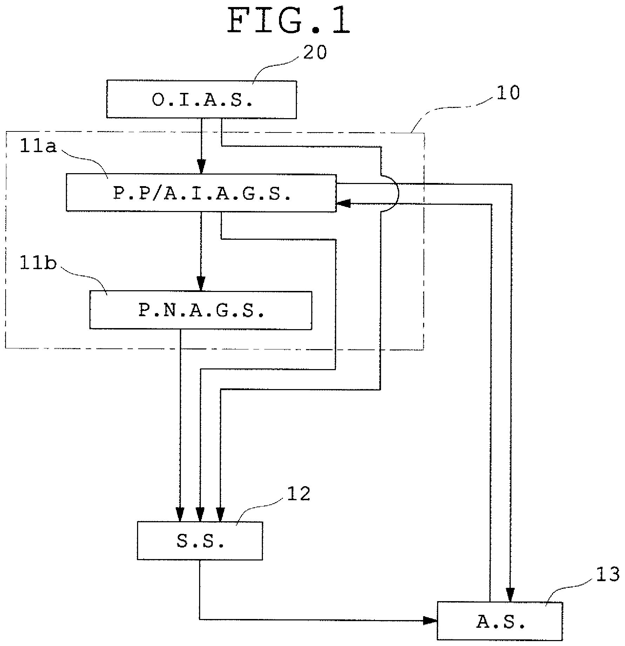

FIG. 1 is a block diagram that schematically shows the basic configuration of a cell observation information processing system according to the present invention.

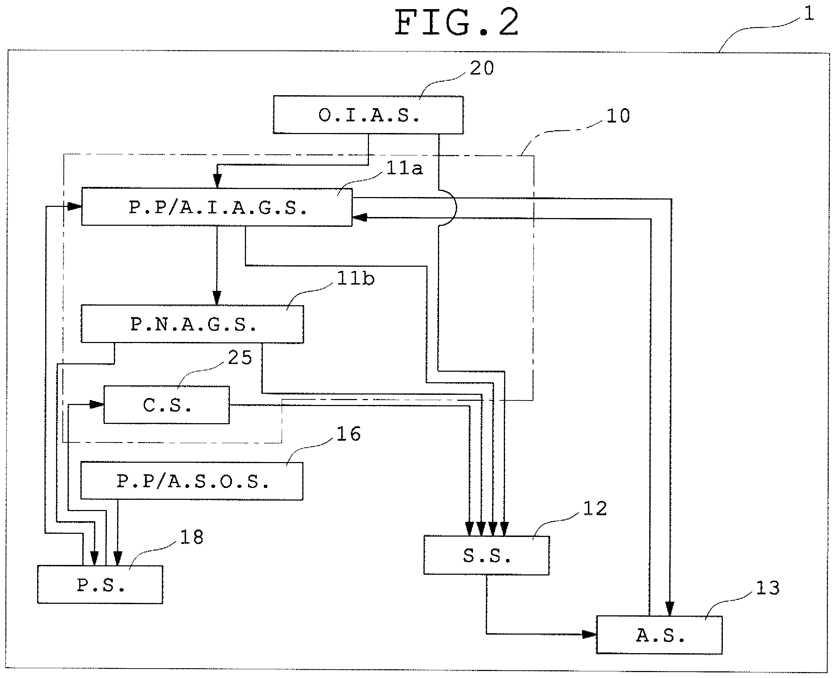

FIG. 2 is an explanatory diagram that shows the configuration of a cell observation information processing system according to a first embodiment mode of the present invention.

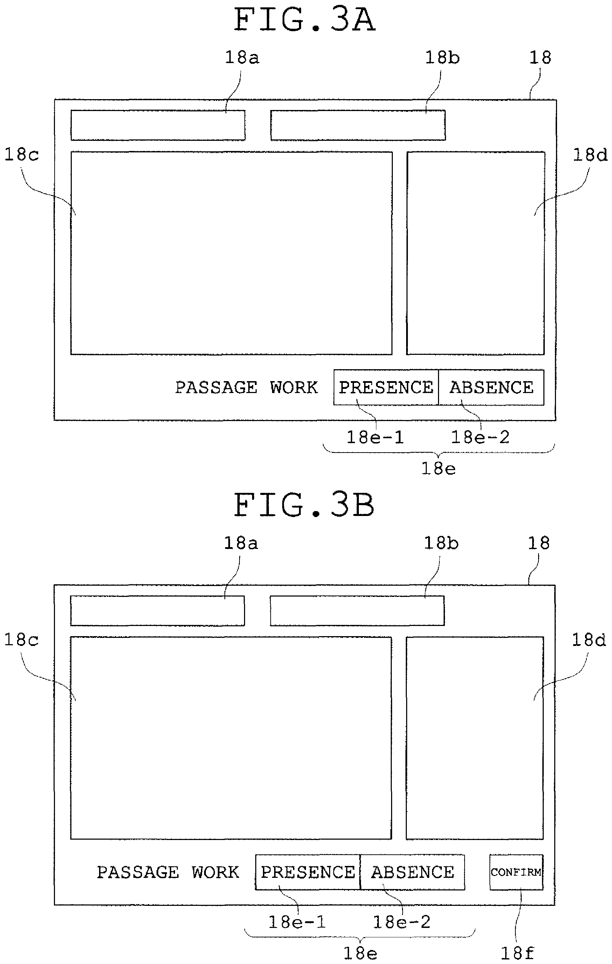

FIG. 3 is an explanatory diagram that shows a configuration example of a presenting section including a passage work presence/absence button for accepting, from a user, a selection order on whether work for cells on the current observation day is passage work in the cell observation information processing system of FIG. 2.

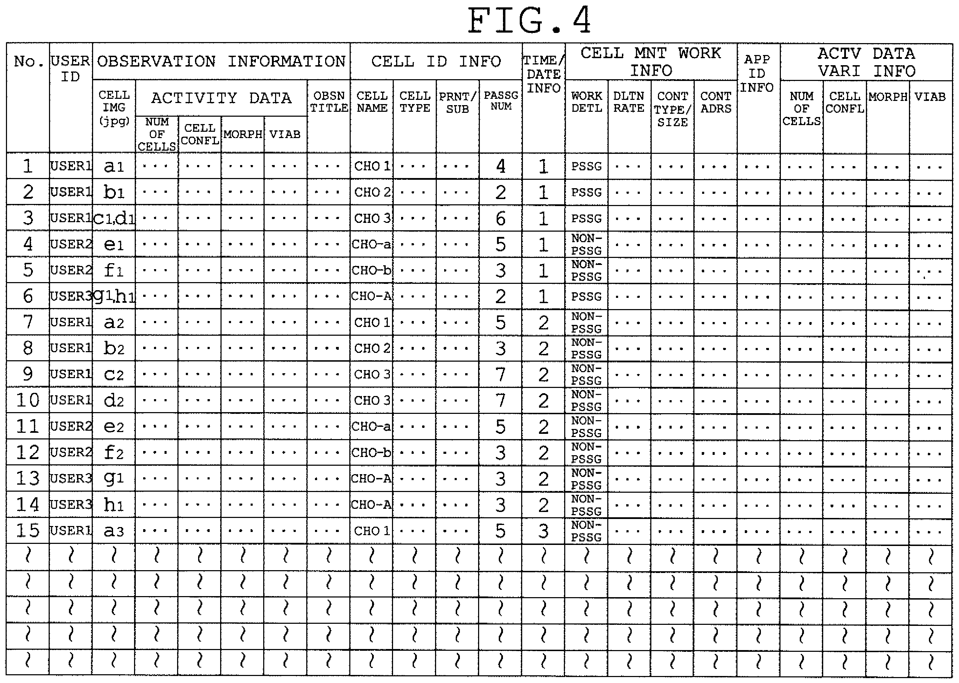

FIG. 4 is an explanatory diagram that shows an example of a data structure of search-tagged observation information to be in an archive section stored by a storing section in the cell observation information processing system of FIG. 2.

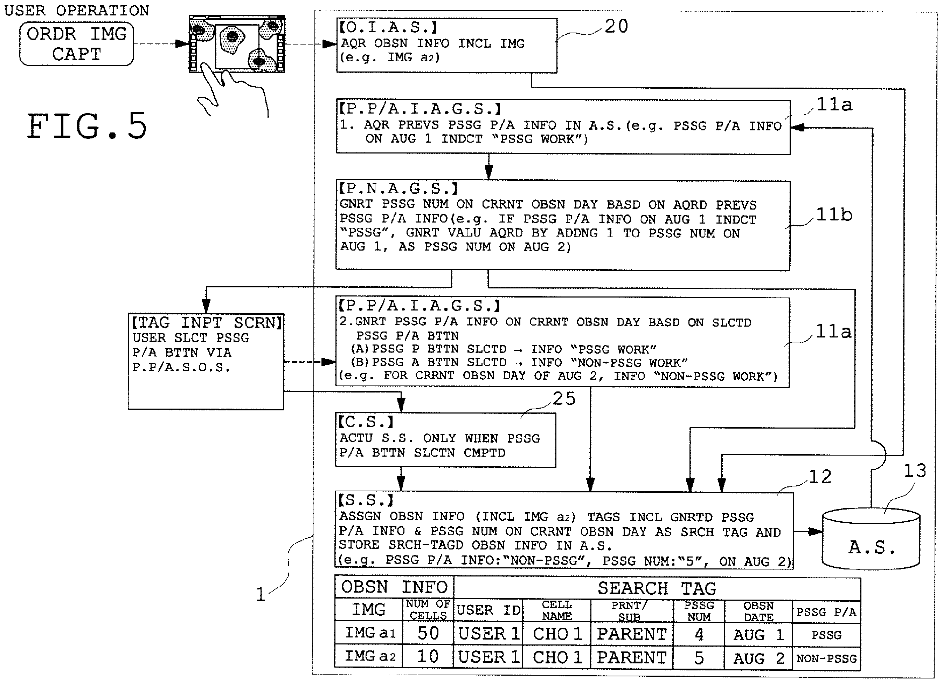

FIG. 5 is an explanatory diagram that schematically shows a flow of processes from acquisition of the observation information, to acquisition of previous passage presence/absence information, generation of the passage number on the current observation day based on the previous passage presence/absence information, a selection order by the user through the presenting section on whether the work to the cells on the current observation day is the passage work, generation of the passage presence/absence value on the current observation day based on the selection order, and storage, in the archive section, of observation information to which the search tags including the passage presence/absence value and the passage number on the day of the observation are assigned, using the cell observation information processing system of FIG. 2.

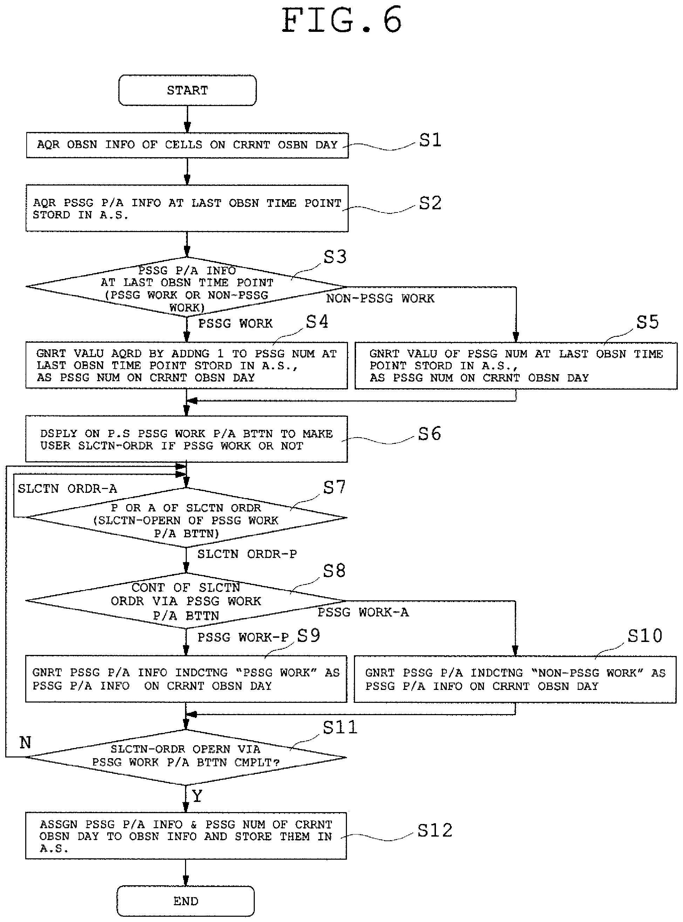

FIG. 6 is a flowchart that shows an example of processing procedures from acquisition of the observation information, to acquisition of previous passage presence/absence information, generation of the passage number on the current observation day based on the previous passage presence/absence information, a selection order by the user through the presenting section on whether the work to the cells on the current observation day is the passage work, generation of the passage presence/absence value on the current observation day based on the selection order, and storage, in the archive section, of observation information to which the search tags including the passage presence/absence value and the passage number on the day of the observation are assigned, using the cell observation information processing system of FIG. 2.

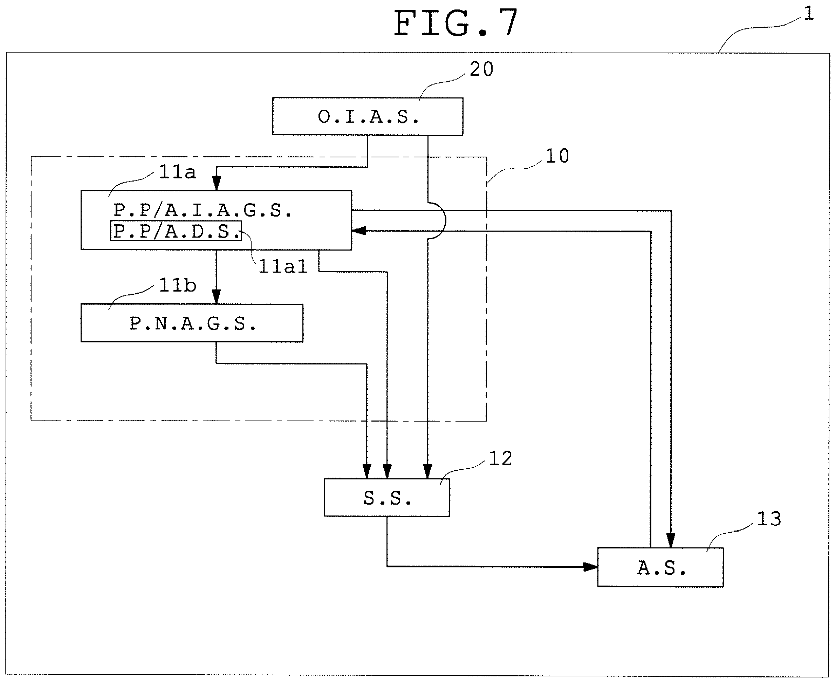

FIG. 7 is an explanatory diagram showing the configuration of a cell observation information processing system according to a second embodiment mode of the present invention.

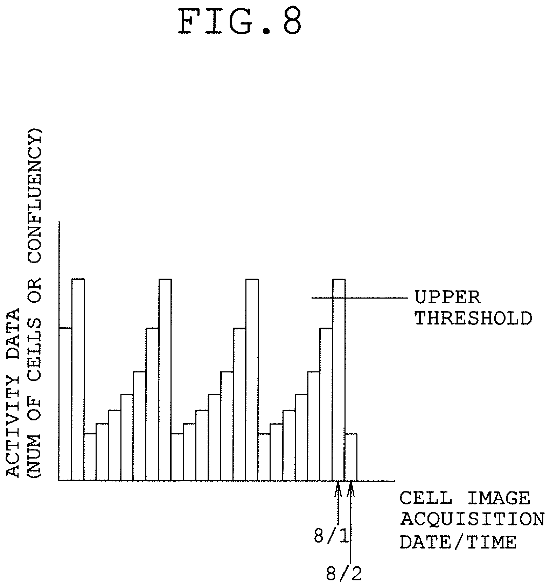

FIG. 8 is an explanatory diagram that conceptually shows a method of determining whether the work for the cells on the current observation day is the passage work by the passage presence/absence determining section in the cell observation information processing system of FIG. 7.

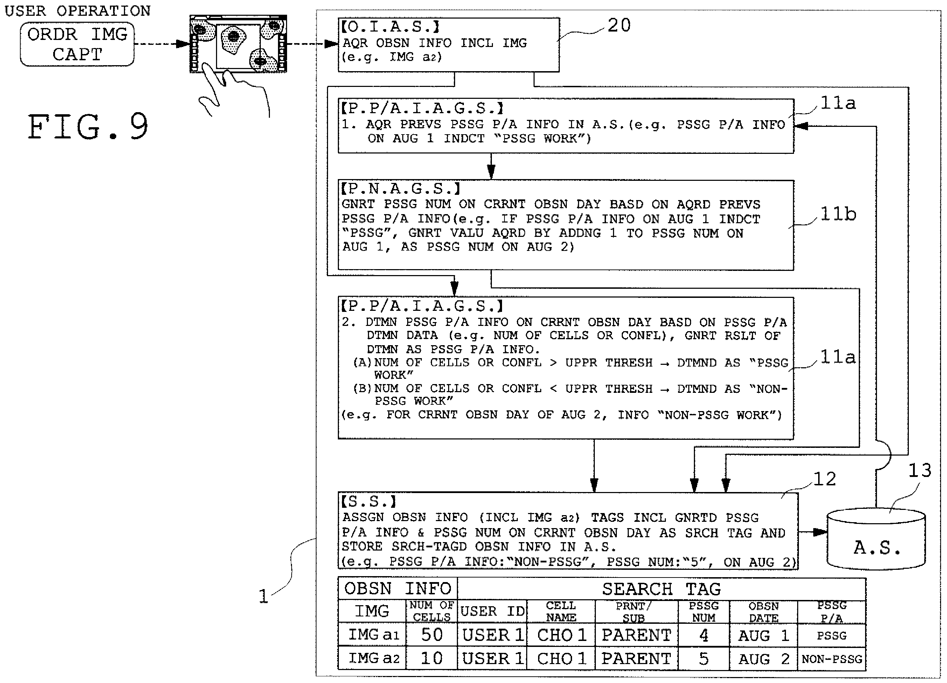

FIG. 9 is an explanatory diagram that schematically shows a flow of processes from acquisition of the observation information, to acquisition of previous passage presence/absence information, generation of the passage number on the current observation day based on the previous passage presence/absence information, determination on whether the work to the cells on the current observation day is the passage work by the passage presence/absence determining section, generation of the passage presence/absence value on the current observation day based on the determination result, and storage, in the archive section, of observation information to which the search tags including the passage presence/absence value and the passage number on the current observation day are assigned, using the cell observation information processing system of FIG. 7.

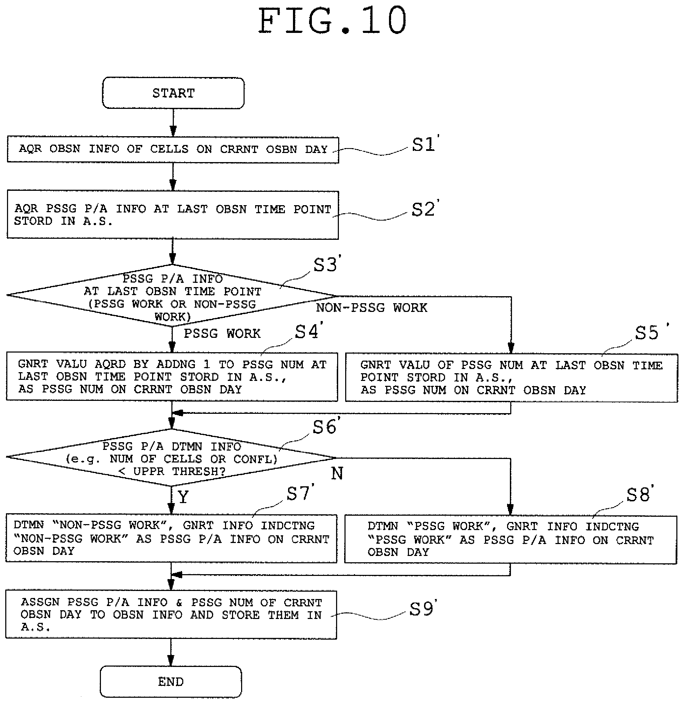

FIG. 10 is a flowchart showing an example of processing procedures from acquisition of the observation information, to acquisition of previous passage presence/absence information, generation of the passage number on the current observation day based on the previous passage presence/absence information, determination on whether the work to the cells on the current observation day is the passage work by the passage presence/absence determining section, generation of the passage presence/absence value on the current observation day based on the determination result, and storage, in the archive section, of observation information to which the search tags including the passage presence/absence value and the passage number on the current observation day are assigned, using the cell observation information processing system of FIG. 7.

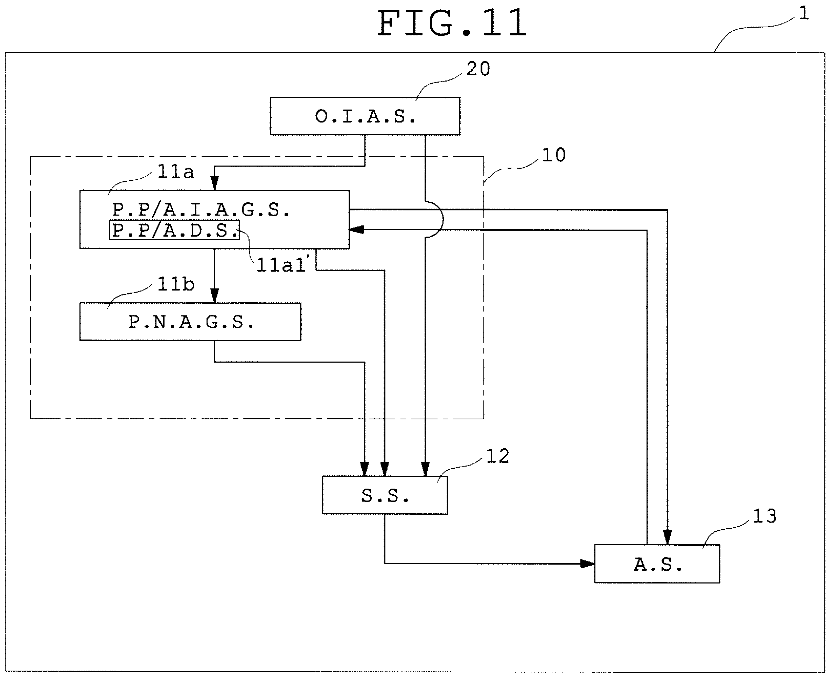

FIG. 11 is an explanatory diagram that shows the configuration of a cell observation information processing system according to a third embodiment mode of the present invention.

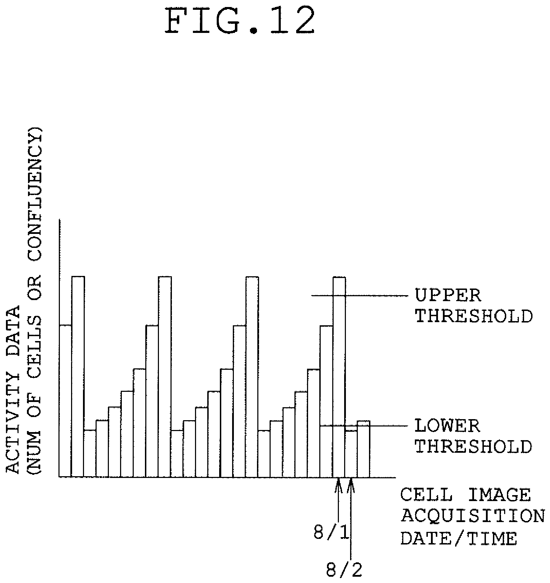

FIG. 12 is an explanatory diagram that conceptually shows a method of determining whether the work for the cells on the last observation time point is the passage work by the passage presence/absence determining section in the cell observation information processing system in FIG. 11.

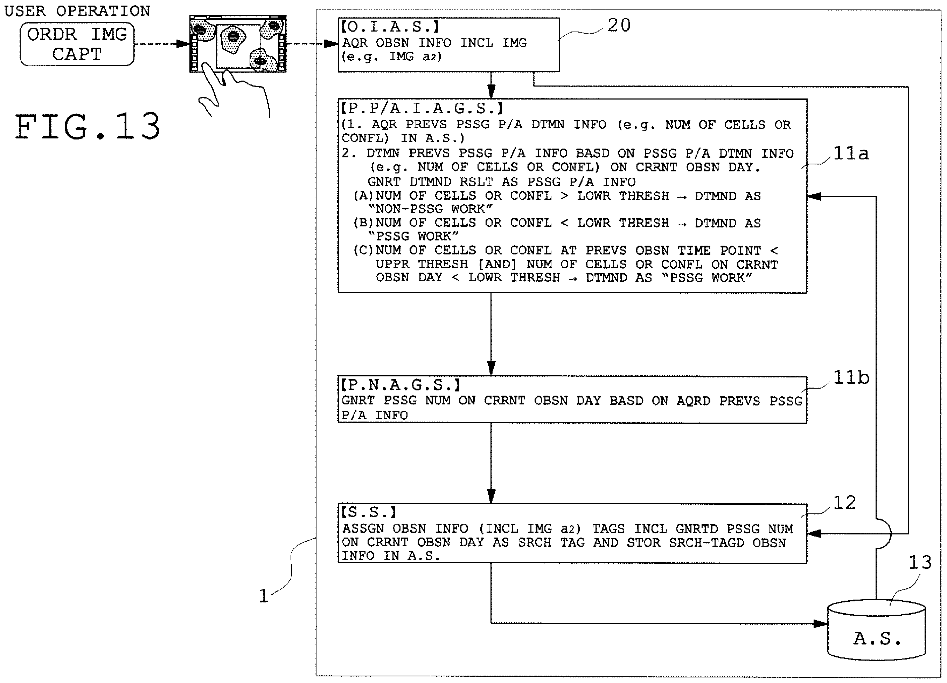

FIG. 13 is an explanatory diagram that schematically shows a flow of processes from acquisition of the observation information, to determination on whether the work to the cells on the previous observation time point is the passage work by the passage presence/absence determining section, generation of the previous passage presence/absence information based on the determination result, generation of the passage number on the current observation day based on the previous passage presence/absence information, and storage, in the archive section, of observation information assigned the search tag including the passage number on the current observation day, using the cell observation information processing system of FIG. 11.

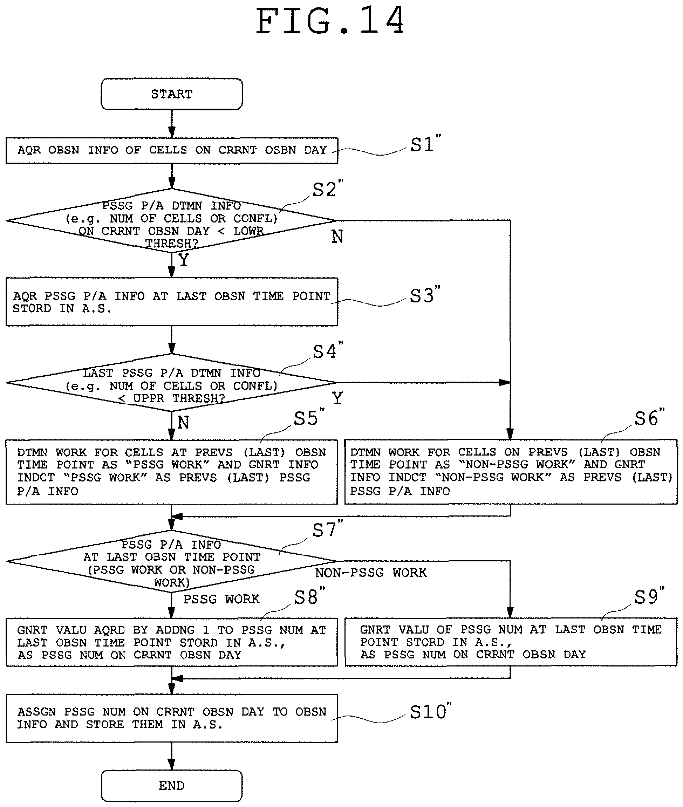

FIG. 14 is a flowchart that shows an example of processing procedures from acquisition of the observation information, to determination on whether the work to the cells on the previous observation time point is the passage work by the passage presence/absence determining section, generation of the previous passage presence/absence information based on the determination result, generation of the passage number on the current observation day based on the previous passage presence/absence information, and storage, in the archive section, of observation information assigned the search tag including the passage number on the current observation day, using the cell observation information processing system of FIG. 11.

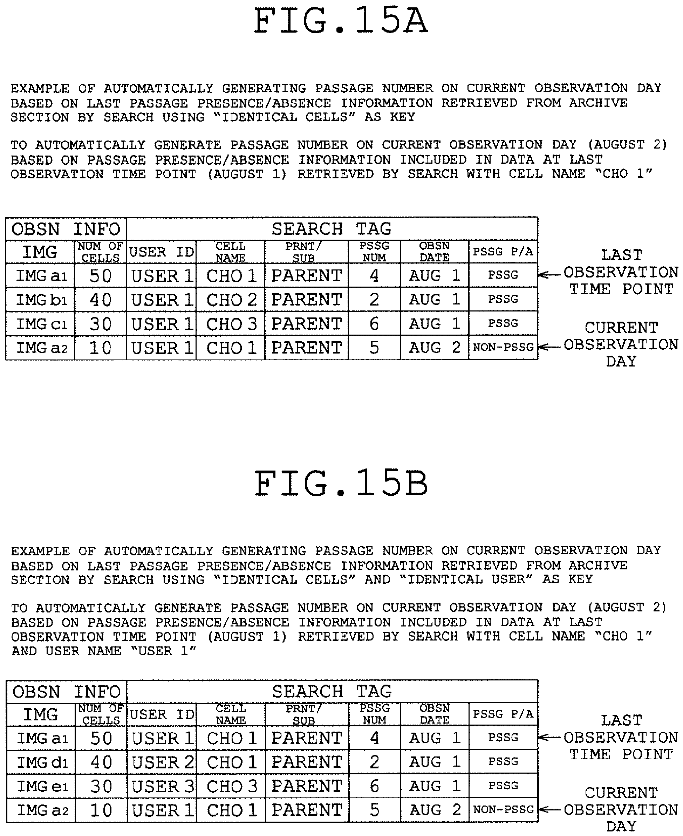

FIGs. 15A and 15B are explanatory diagrams that conceptually show an example of a method of selecting data to be subjected to acquisition or generation of the last passage presence/absence value by the passage presence/absence information acquiring section and to acquisition or generation of the passage number by the passage number acquiring and generating section, in a passage cell observation information processing system according to a fourth embodiment mode of the present invention. FIG. 15A is a data configuration diagram that shows an example thereof. FIG. 15B is a data configuration diagram that shows another example.

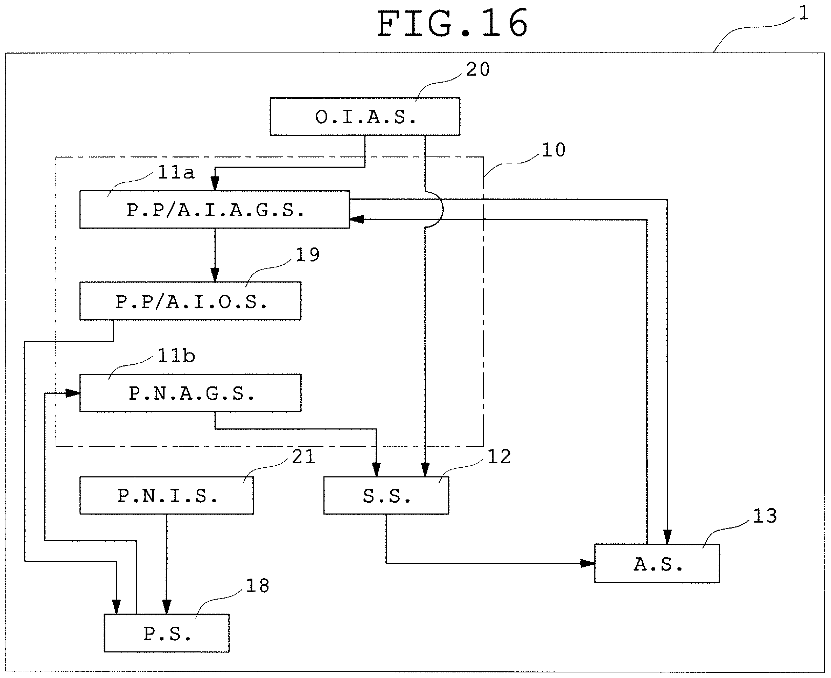

FIG. 16 is an explanatory diagram that shows the configuration of a cell observation information processing system according to a fifth embodiment mode of the present invention.

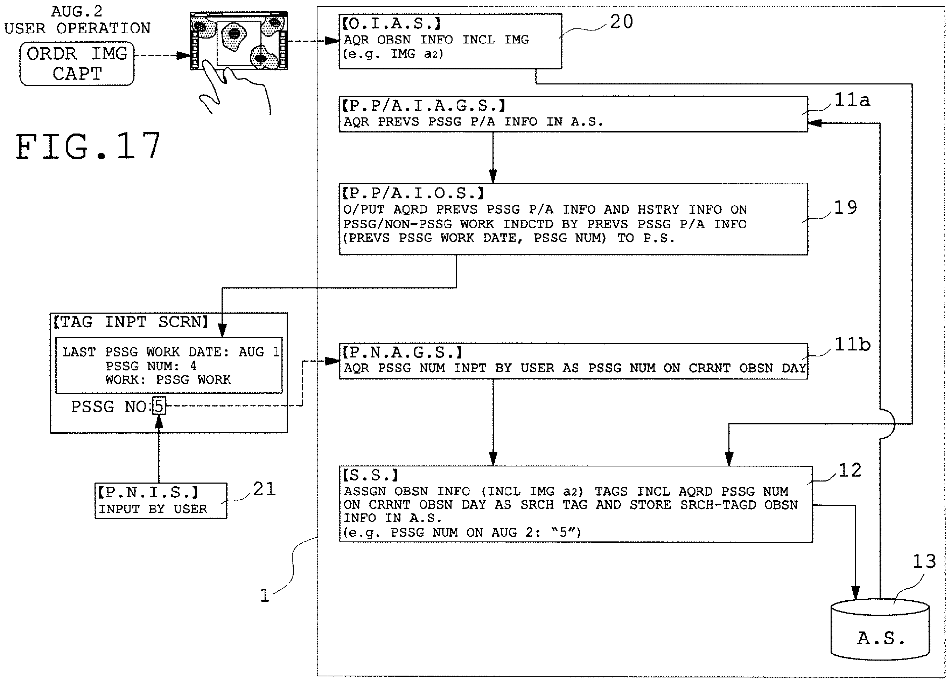

FIG. 17 is an explanatory diagram that schematically shows a flow of processes from acquisition of the observation information, to acquisition of the previous passage presence/absence information, output of the previous passage presence/absence information to the presenting section, acquisition of the passage number on the current observation day based on the passage number on the current observation day input by the user through the presenting section, and storage, in the archive section, of observation information assigned the search tag including the passage number on the current observation day, using the cell observation information processing system of FIG. 16.

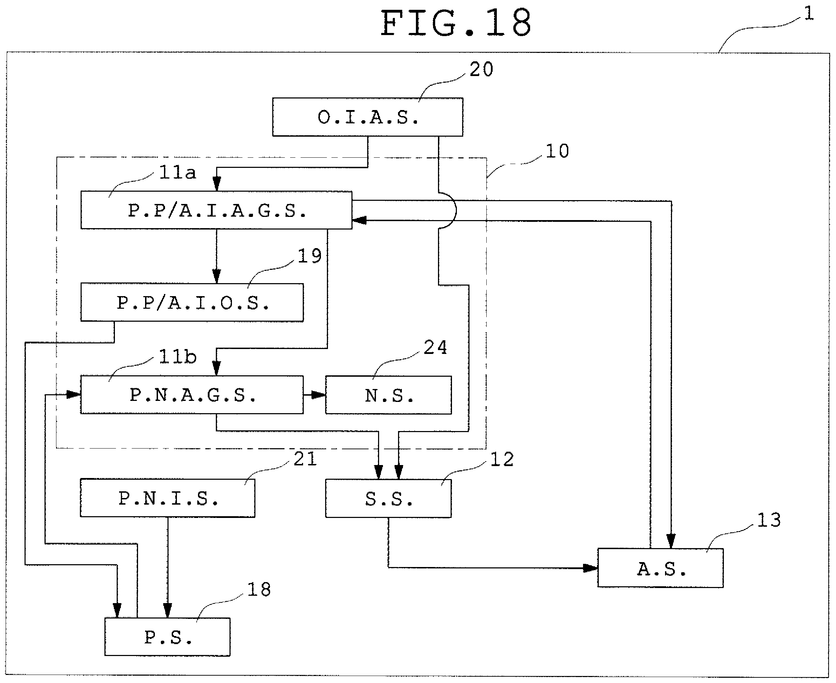

FIG. 18 is an explanatory diagram that shows the configuration of a cell observation information processing system according to a modified example of FIG. 16.

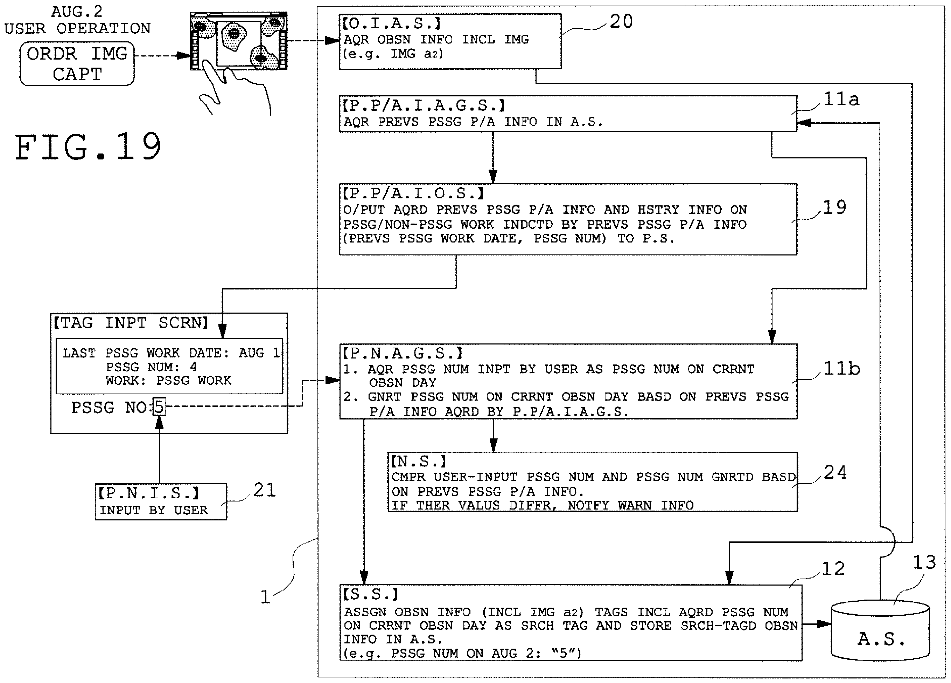

FIG. 19 is an explanatory diagram that schematically shows a flow of processes from acquisition of the observation information, to acquisition of the previous passage presence/absence information, output of the previous passage presence/absence information to the presenting section, acquisition of the passage number on the current observation day based on the passage number on the current observation day input by the user through the presenting section, automatic generation of the passage number on the current observation day based on the previous passage presence/absence information, notification on warning information based on comparison between the passage number on the current observation day input by the user and the automatically generated passage number, and storage, in the archive section, of observation information as signed the search tag including the pas sage number on the current observation day, using the cell observation information processing system of FIG. 18.

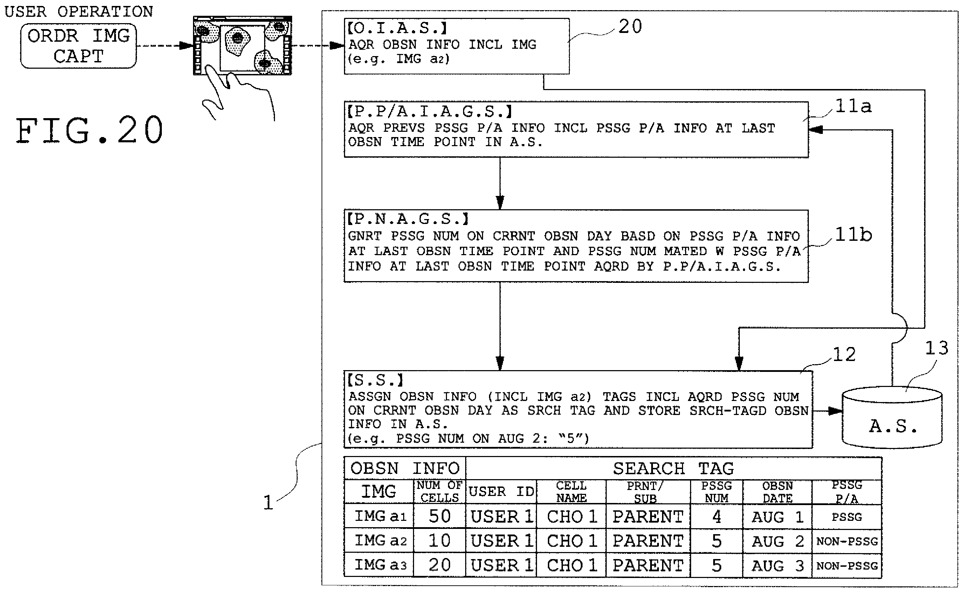

FIG. 20 is an explanatory diagram that schematically shows a flow of processes from acquisition of the observation information, to acquisition of the previous passage presence/absence information, acquisition of the passage number on the current observation day based on the previous passage presence/absence information and passage number, and storage, in the archive section, of observation information assigned the search tag including the passage number on the current observation day, using the cell observation information processing system according to a sixth embodiment mode of the present invention.

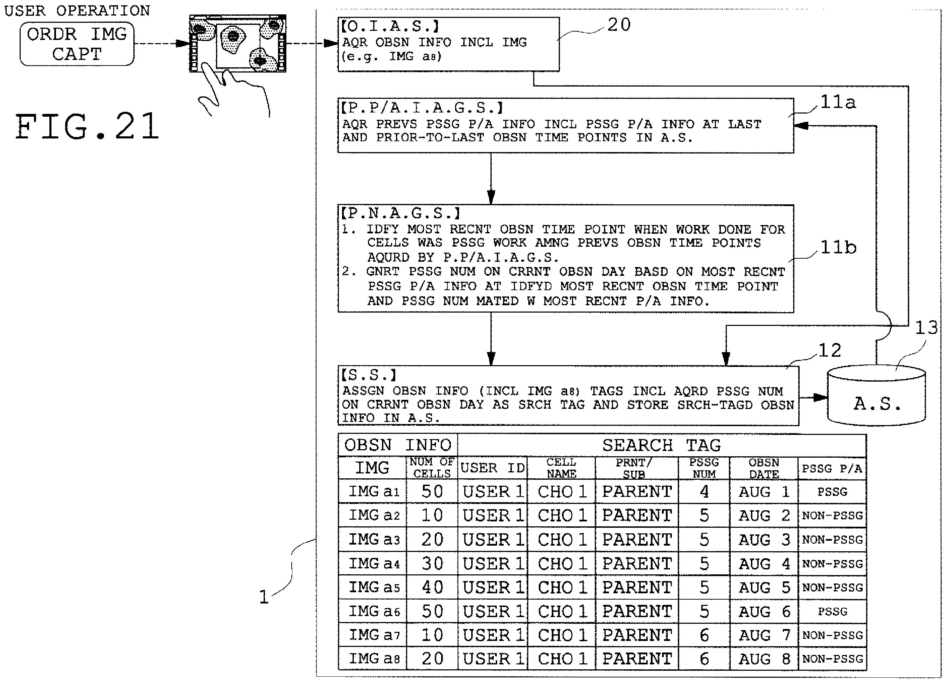

FIG. 21 is an explanatory diagram that schematically shows a flow of processes from acquisition of the observation information, to acquisition of the previous passage presence/absence information, acquisition of the passage number on the current observation day based on the previous passage presence/absence information and passage number, and storage, in the archive section, of observation information assigned the search tag including the passage number on the current observation day, using the cell observation information processing system according to a seventh embodiment mode of the present invention.

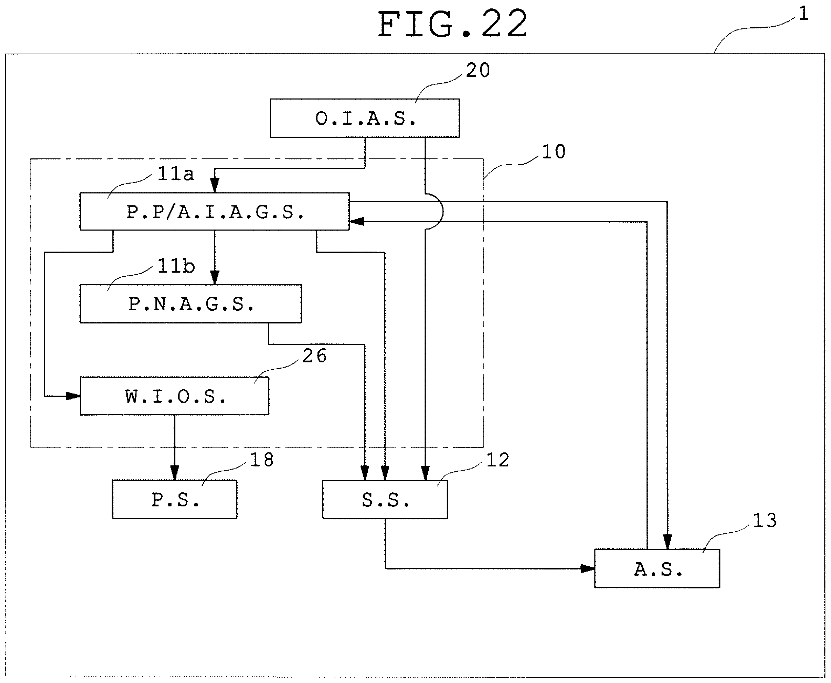

FIG. 22 is an explanatory diagram that shows the configuration of a cell observation information processing system according to an eighth embodiment mode of the present invention.

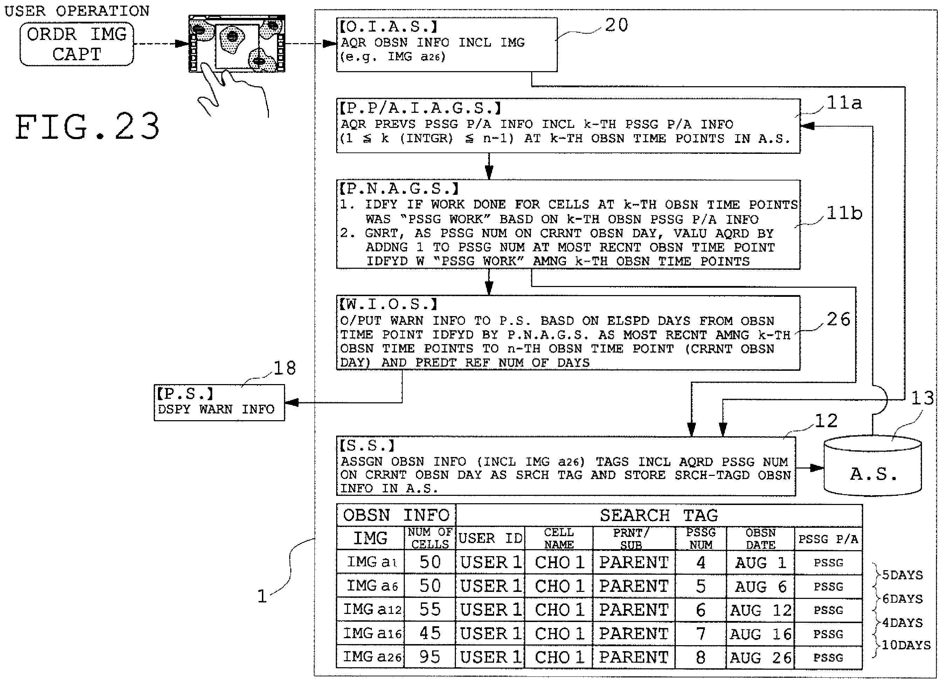

FIG. 23 is an explanatory diagram that schematically shows a flow of processes from acquisition of the observation information, to acquisition of the previous passage presence/absence information, identification of the most recent observation time point at which the passage work is conducted in the previous passage presence/absence information, acquisition of the passage number at the current observation day based on the passage number at the identified observation time point, output of warning information based on the elapsed days from the identified most recent observation time point to the current observation day and the predetermined reference number of days, and storage, in the archive section, of observation information as signed the search tag including the passage number on the current observation day, using the cell observation information processing system of FIG. 22.

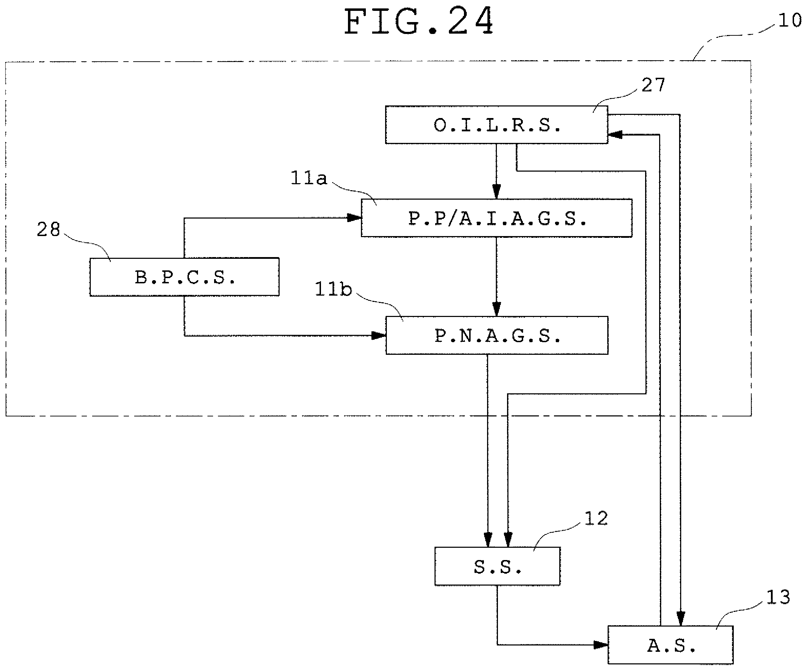

FIG. 24 is an explanatory diagram that shows the main configuration of a cell observation information processing system according to a ninth embodiment mode of the present invention.

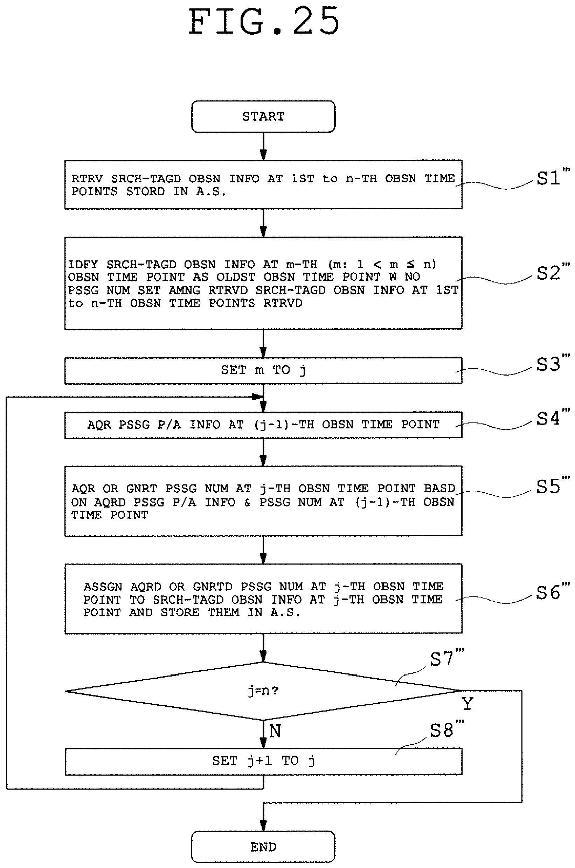

FIG. 25 is a flowchart that shows an example of processing procedures from extraction of the first-to-n-th-tagged observation information, to identification of the search-tagged observation information at the oldest observation time point with no passage number being set, batch generation of the passage number, and storage, in the archive section, of the observation information assigned tag with passage number being set, using the cell observation information processing system of FIG. 24.

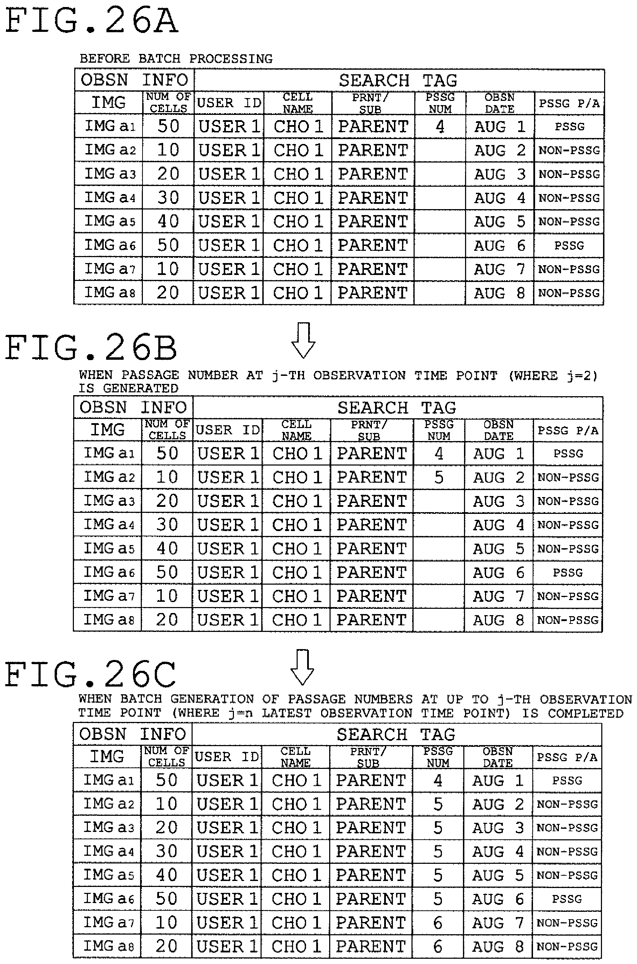

FIGs. 26A to 26C are explanatory diagrams that show an example of search-tagged observation information record where the passage number is generated in bulk using the cell observation information processing system of FIG. 24. FIG. 26A is a diagram that shows a search-tagged observation information record before the batch processing. FIG. 26B is a diagram that shows a state where the passage number is assigned to the search-tagged observation information record at the oldest observation time point with no passage number being indicated. FIG. 26C is a diagram that shows a state where the passage number is assigned to the search-tagged observation information record up to the latest observation time point.

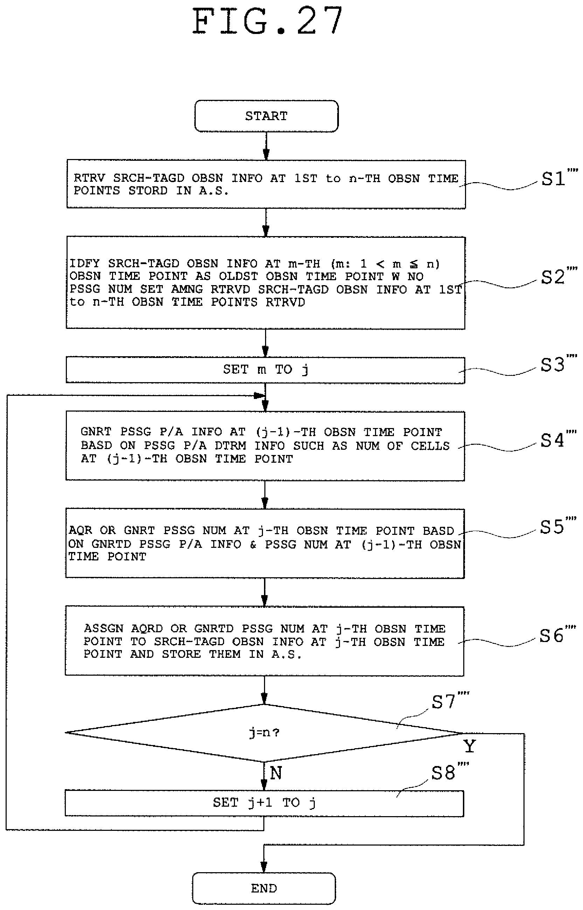

FIG. 27 is a flowchart that shows an example of processing procedures from retrieval of the first-to-n-th-tagged observation information, to identification of the search-tagged observation information at the oldest observation time point with no passage number being set, batch generation of the passage number, and storage, in the archive section, of the observation information assigned the tag of the passage number, using a cell observation information processing system according to a tenth embodiment mode of the present invention.

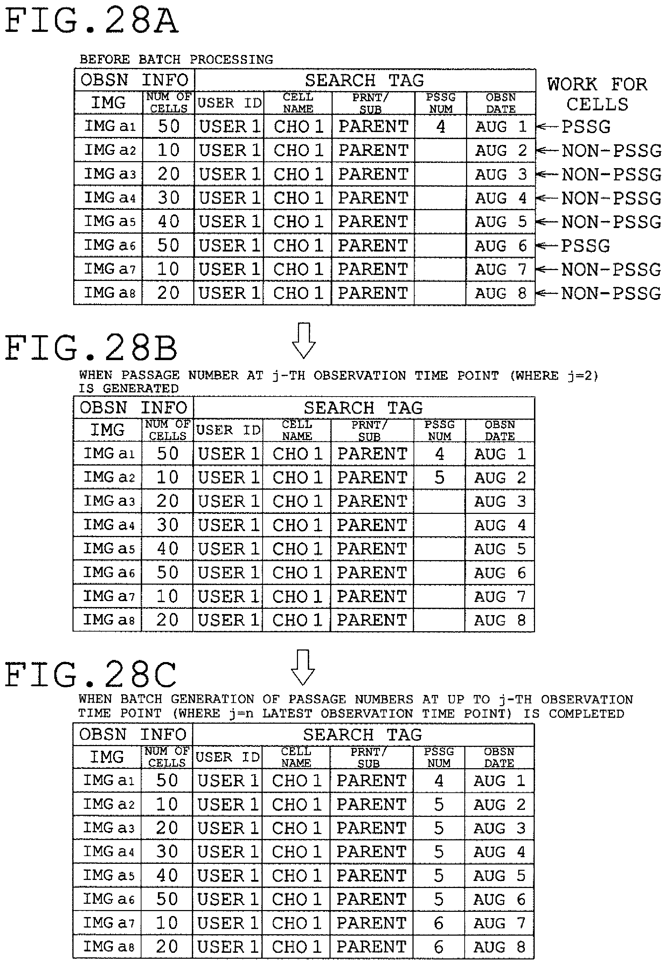

FIGs. 28A to 28C are explanatory diagrams that show an example of search-tagged observation information records where the passage number is generated in bulk using the cell observation information processing system of FIG. 27. FIG. 28A is a diagram that shows search-tagged observation information records before the batch processing. FIG. 28B is a diagram that shows a state where the passage number is assigned to the search-tagged observation information record at the oldest observation time point with no passage number being indicated. FIG. 28C is a diagram that shows a state where the passage number is assigned to the search-tagged observation information record up to the latest observation time point.

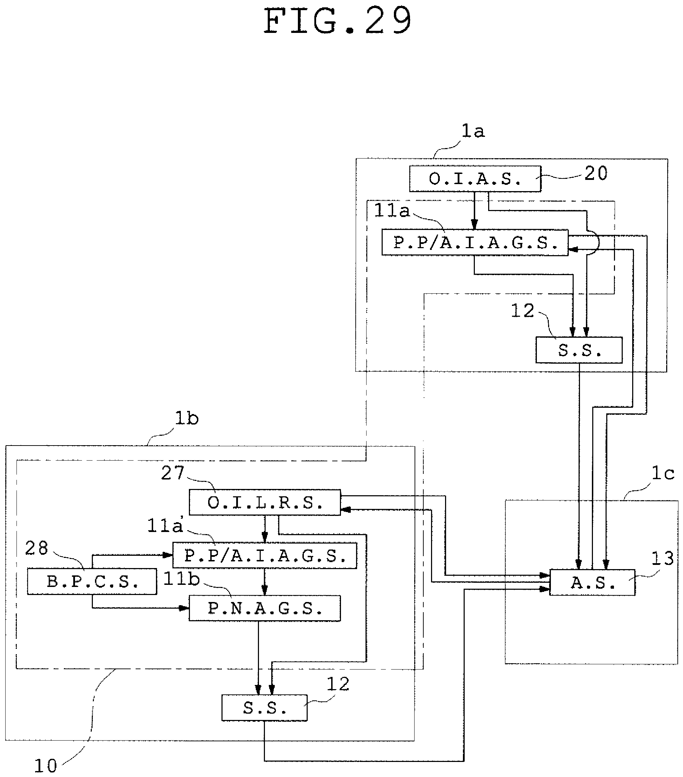

FIG. 29 is an explanatory diagram that shows the configuration of a cell observation information processing system according to a modified example of the present invention.

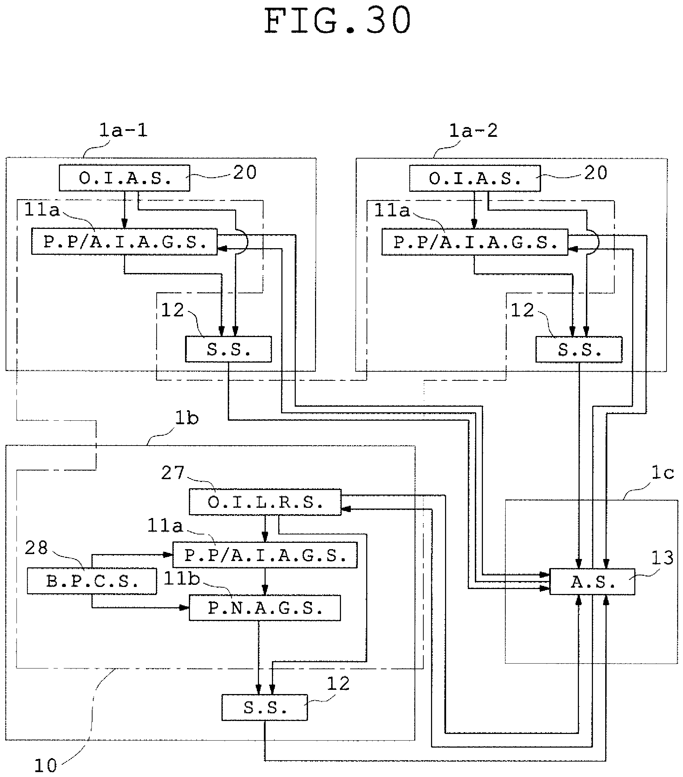

FIG. 30 is an explanatory diagram that shows the configuration of a cell observation information processing system according to another modified example of the present invention.

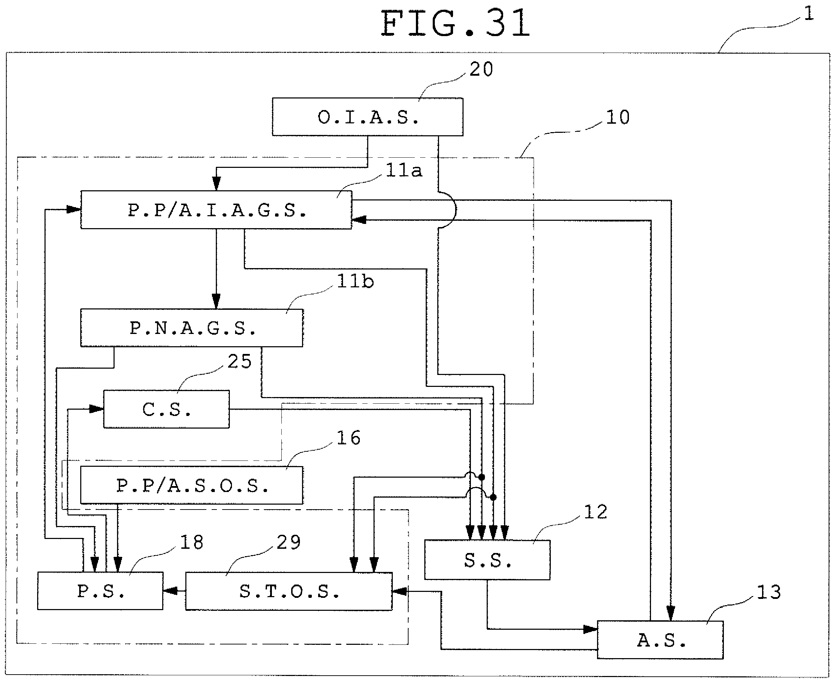

FIG. 31 is an explanatory diagram that shows the configuration of a cell observation information processing system according to still another modified example of the present invention.
FIG. 32 is an explanatory diagram that shows an example of the relationship between maintenance work for cells and timing of incrementing the passage number.

### MODE FOR CARRYING OUT THE INVENTION

Embodiment modes of the present invention will be explained below. The embodiment mode explained below does not improperly limit the contents of the present invention recited in the claimed scope for a patent. In addition, not all of the configurations explained in reference to the embodiment mode below are indispensable configurations requirement for the present invention.

A cell observation information processing system according to the embodiment mode of the present invention includes: a passage presence/absence information acquiring and generating section that acquires or generates previous passage presence/absence information including at least one passage presence/absence value among first to (n-1)-th passage presence/absence values indicating whether work for cells was passage work at first to (n-1)-th observation time points, among first to n-th observation time points at which first to n-th pieces of observation information each including items indicating a cell observation result including a cell image, activity data indicating the cell activity such as the number of cells, confluency, morphology and viability, and an observation name are acquired in a time series manner through an observation information acquiring section; and a passage number acquiring and generating section that acquires or generates at least an n-th passage number at the n-th observation time point automatically or through an operation by a user, based on the previous passage presence/absence information acquired or generated by the passage presence/absence information acquiring and generating section. Note that n is an integer equal to or greater than two, and the current observation time point is represented by the n-th observation time point.

As with the cell observation information processing system according to the embodiment mode of the present invention, in the case where the passage presence/absence information acquiring and generating section acquires or generates the previous passage presence/absence information including at least one or more passage presence/absence values among the first to (n-1)-th passage presence/absence values indicating whether the work for the cells at the first to (n-1) -th observation time points is the passage work and where the passage number acquiring and generating section acquires or generates at least an n-th passage number at the n-th observation time point based on the previous passage presence/absence information acquired or generated by the passage presence/absence information acquiring and generating section, the apparatus can support input by the user inputting the passage number. Alternatively, without input of the passage number by the user, the apparatus can appropriately, automatically update the passage number. As a result, the user's error of inputting the passage number that is one of tags to be assigned to cell observation information, such as on cells, can be prevented.

According to the embodiment mode of the present invention, the passage presence/absence information acquiring and generating section may be configured to acquire or generate the passage presence/absence information through an operation by the user. Alternatively, this section may be configured to automatically acquire or generate the information, for example.

According to the embodiment mode of the present invention, the passage number acquiring and generating section may be configured to acquire or generate the passage number through an operation by the user. Alternatively, this section may be configured to automatically acquire or generate the information, for example.

According to the embodiment mode of the present invention, as to the timing when the passage presence/absence information acquiring and generating section acquires or generates the passage presence/absence information or the timing when the passage number acquiring and generating section acquires or generates the passage number, a configuration may be adopted that achieves the acquisition or generation every time the observation information is acquired. Alternatively, a configuration may be adopted that achieves batch acquisition or generation after acquisition of the observation information.

The embodiment mode of the present invention exerts, besides the above advantageous effects, the following accompanying advantageous effects according to the configurations of the passage presence/absence information acquiring and generating section and the passage number acquiring and generating section.

### (1) Function and effect of configuration of passage presence/absence information acquiring and generating section

### (1-1) Function and effect of configuration where passage presence/absence information acquiring and generating section acquires or generates passage presence/absence information through user's operation

Preferably, the cell observation information processing system according to the embodiment mode of the present invention is configured so that the passage presence/absence information acquiring and generating section can further generate the n-th passage presence/absence value indicating whether the work for the cells at the n-th observation time point is the passage work, based on the selection order accepted from the user through the presenting section, and the presenting section can accept, from the user, the selection order on whether the work for the cells at the n-th observation time point is passage work.

As stated above, in the case where the cell observation information at each time point is compiled into the database, the image of the cells on the current observation day when the passage work is to be conducted is acquired before the passage work. As a result, the observation day on which the passage number of the cell specimen is to be incremented and stored in the database file deviates from the observation day on which the cell passage work is actually conducted. Consequently, if the user tries to input the passage number manually through a screen or the like where a search tag to be assigned to observation information is set, at the current observation day when the user conducted predetermined maintenance work for cells on the basis of memory and recognition of the last maintenance work having been conducted for cells, misunderstanding on passage number counting and misunderstanding on the details of maintenance work conducted for cells occur, and the passage number is thus prone to be erroneously input.

On the other hand, the observation day on which the user stores the work details for the cells in the database file coincides with the observation day on which the work for the cells is actually conducted. Consequently, if the user is not caused to be aware the value of the passage number to be input, the possibility that the user misunderstands the work details having conducted for the cells on the current observation day is considered to be reduced. Moreover, if the user is caused to perform an operation for confirming the details of work conducted for the cells on the current observation day, it is considered that misunderstanding of the work details can be further prevented.

As with the cell observation information processing system according to the embodiment mode of the present invention, in the case where the passage presence/absence information acquiring and generating section further generates the n-th passage presence/absence value indicating whether the work for the cells at the n-th observation time point is the passage work based on the selection order accepted from the user through the presenting section, and the presenting section accepts, from the user, the selection order on whether the work for the cells at the n-th observation time point is the passage work, assigning the n-th (i.e., on the current observation day) passage presence/absence value accepted from the user as the search tag to the n-th observation information acquired by the observation information acquiring section and storing the information in the archive section allows the passage presence/absence value stored in the archive section this time to be the previous (i.e., the (n-1)-th) passage presence/absence value at the next observation time point. Consequently, at the next observation time point, the passage presence/absence information acquiring and generating section acquires or generates the previous passage presence/absence value, and the passage number acquiring and generating section acquires or generates the passage number, based on the previous passage presence/absence value. As a result, the user can acquire the correct value as the passage number that is one of the search tags to be assigned to the observation information at the next observation time point only by a simple operation, for example, pressing a selection button, in order to confirm the work details for the cells conducted on the current observation day. This negates the need for user's inputting the passage number and, in turn, negates user's input errors of the passage number, and can prevent the user from misunderstanding the work details conducted for the cells on the current observation day.

That is,preferably,the cell observation information processing system according to the embodiment mode of the present invention further includes a storing section that assigns the n-th passage presence/absence value generated by the passage presence/absence information acquiring and generating section, as a search tag, to the n-th observation information acquired by the observation information acquiring section, and stores the n-th observation information assigned the search tag, in the archive section.

As described above, such a configuration negates user's input errors of the passage number, and furthermore, can exert an advantageous effect of preventing misunderstanding of the work details conducted for the cells at the current observation day.

Preferably, the cell observation information processing system according to the embodiment mode of the present invention further includes a control section that controls the storing section so as to execute storage of the n-th observation information assigned the search tag, in the archive section, when accepting the selection order on whether the work for the cells is the passage work, from the user through the presenting section.

Such a configuration allows the user to confirm storage of the passage presence/absence value on the current observation day in the archive section through the simple operation as described above, in addition to the advantageous effect as described above.

Preferably, in the cell observation information processing system according to the embodiment mode of the present invention, the presenting section completes accepting, from the user, the selection order on whether the work for the cells at the n-th observation time point is the passage work, by input of a confirmation order from the user.

Such a configuration allows the user to reconfirm the selection order on whether the work is the passage work before the n-th observation information assigned the search tag is stored in the archive section by the storing section, thereby enabling the user to be prevented from erroneously operating the selection order.

### (1-2) Function and effect of configuration where passage presence/absence information acquiring and generating section automatically acquires or generates passage presence/absence information

Preferably, in the cell observation information processing system according to the embodiment mode of the present invention, the passage presence/absence information acquiring and generating section includes a passage presence/absence determining section that determines whether the work for the cells at the n-th observation time point is the passage work, based on passage presence/absence determining information at the n-th observation time point, and generates, as the n-th passage presence/absence value, a determination result determined by the passage presence/absence determining section on determining whether the work is the passage work.

The activity data indicating the cell activity that includes, for example, the number of cells, confluency, passage dates of the past, the number of passage interval days, the time elapsed from the last passage date are used as the passage presence/absence determining information.

According to such a configuration, as described later, in the case where the passage presence/absence information acquiring and generating section assigns the n-th (i.e., the current observation day) passage presence/absence value automatically acquired or generated as the search tag to the n-th observation information acquired by the observation information acquiring section, based on the determination result by the passage presence/absence determining section, and stores the information in the archive section, the passage presence/absence value stored in the archive section this time is to be the previous (i.e., the (n-1)-th) passage presence/absence value at the next observation time point. Consequently, at the next observation time point, the passage presence/absence information acquiring and generating section acquires or generates the previous passage presence/absence value, and the passage number acquiring and generating section acquires or generates the passage number, based on the previous passage presence/absence value. As a result, the user can acquire the correct value as the passage number that is one of the search tags to be assigned to the observation information at the next observation time point without any operation for confirming the work details for the cells conducted on the current observation day. The need of input of the passage number by the user is then negated, which can eliminate input errors of the passage number by the user. Furthermore, the necessity of work for confirming the work details for the cells conducted on the current observation day is negated, which can reduce efforts and time for user's operation required to acquire the tag to be assigned to the observation information on the cells and the like.

That is, preferably, the cell observation information processing system according to the embodiment mode of the present invention further includes a storing section that assigns the n-th passage presence/absence value generated by the passage presence/absence information acquiring and generating section, as a search tag, to the n-th observation information acquired by the observation information acquiring section, and stores the n-th observation information assigned the search tag, in the archive section.

Such a configuration can eliminate errors of input of the passage number by the user as described above, and negates the need of work for confirming the work details for the cells conducted on the current observation day, which can exert the advantageous effect of reducing efforts and time for user's operation required to acquire the tag to be assigned to the observation information on the cells and the like.

### (1-2-1) Function and effect of configuration in which passage presence/absence determiningsection determines that work for cells at n-th observation time point is non-passage work when the number of cells or confluency at n-th observation time point is lower than predetermined upper threshold

Preferably, the cell observation information processing system according to the embodiment mode of the present invention is configured so that the activity data is the number of cells or confluency, and when the number of cells or confluency at the n-th observation time point is lower than the upper threshold, the passage presence/absence determining section determines that the work for the cells at the n-th observation time point is the non-passage work.

As stated above, typically, in a period during which the cell activity data acquired from a captured image does not reach the upper threshold of the predetermined confluency or number of cells, the user conducts the non-passage work for the cells.

Consequently, when the cell activity data on the current observation day is lower than the predetermined upper threshold of the confluency or number of cells, the possibility that the work conducted for the cells on the current observation day is the non-passage work is significantly high.

As with the cell observation information processing system according to the embodiment mode of the present invention, in the case where when the number of cells or confluency at the n-th observation time point is lower than the predetermined upper threshold, the passage presence/absence determining section determines that the work for the cells at the n-th observation time point is the non-passage work, the apparatus can automatically acquire or generate the passage presence/absence value at the n-th observation time point without intervention of user's operation.

### (1-2-2) Function and effect of configuration in which passage presence/absence determiningsection determines that work for cells at n-th observation time point is passage work when the number of cells or confluency at n-th observation time point is at least predetermined upper threshold

The cell observation information processing system according to the embodiment mode of the present invention is configured so that the activity data is the number of cells or confluency, and when the number of cells or confluency at the n-th observation time point is at least the predetermined upper threshold, the passage presence/absence determining section determines that the work for the cells at the n-th observation time point is the passage work.

As stated above, typically, when the cell activity data acquired from a captured image is confirmed to reach the upper threshold of the predetermined confluency or number of cells, the user conducts the passage work for the cells.

Consequently, when the cell activity data on the current observation day is at least the predetermined upper threshold of the confluency or number of cells, the possibility that the work conducted for the cells on the current observation day is the passage work is significantly high.

As with the cell observation information processing system according to the embodiment mode of the present invention, in the case where when the number of cells or confluency at the n-th observation time point is at least the upper threshold, the passage presence/absence determining section determines that the work for the cells at the n-th observation time point is the passage work, the apparatus can automatically acquire or generate the passage presence/absence value at the n-th observation time point without intervention of user's operation.

### (1-2-3) Function and effect of configuration in which passage presence/absence information acquiring and generating section automatically generates previous passage presence/absence information based on at least passage presence/absence determination information at n-th observation time point

Preferably, in the cell observation information processing system according to the embodiment mode of the present invention, the passage presence/absence information acquiring and generating section includes a passage presence/absence determining section that determines whether the work for the cells at the (n-1) -th observation time point is the passage work, based on passage presence/absence determining information at the n-th observation time point, and is configured to generate, as the previous passage presence/absence information, a determination result determined by the passage presence/absence determining section on determining whether the work is the passage work.

Such a configuration allows the passage presence/absence information acquiring section to acquire or generate the passage presence/absence value at the (n-1)-th observation time point even if the passage presence/absence information is not stored as the search tag in the archive section, and allows the passage number acquiring and generating section to acquire or generate the passage number at the n-th observation time point, based on the passage presence/absence value at the acquired or generated (n-1)-th observation time point. As a result, the user can acquire the correct value as the passage number that is one of the search tags to be assigned to the observation information on the current observation day. The need of input of the passage number by the user is then negated, which can eliminate input errors of the passage number by the user.

### (1-2-3-1) Function and effect of configuration in which passage presence/absence determiningsection determines that work for cells at (n-1) -th observation time point is passage work when the number of cells or confluency at n-th observation time point is lower than predetermined lower threshold

Preferably, the cell observation information processing system according to the embodiment mode of the present invention is configured so that the activity data is the number of cells or confluency, and when the number of cells or confluency at the n-th observation time point is lower than the lower threshold, the passage presence/absence determining section determines that the work for the cells at the (n-1) -th observation time point is the passage work.

As stated above, typically, when the cell activity data acquired from a captured image is confirmed to reach the upper threshold of the predetermined confluency or number of cells, the user conducts the passage work for the cells. On the next observation day of an observation day when passage work was conducted, the activity data acquired by capturing images of cells in a new culture container falls short of a lower threshold of the predetermined confluency or the number of cells. On the observation day after the next observation day of that when passage work was conducted, the activity data acquired by taking images of cells in the culture container becomes equal to or exceeds the lower threshold of the predetermined confluency or the number of cells.

Consequently, when the cell activity data on the current observation day is lower than the predetermined lower threshold of the confluency or number of cells, the possibility that the work conducted for the cells at the last observation time point is the passage work is significantly high.

As with the cell observation information processing system according to the embodiment mode of the present invention, in the case where when the number of cells or confluency at the n-th observation time point is lower than the predetermined lower threshold, the passage presence/absence determining section determines that the work for the cells at the (n-1)-th observation time point is the passage work, the passage presence/absence information acquiring section can acquire or generate the passage presence/absence value at the (n-1)-th observation time point even if the passage presence/absence information is not stored as the search tag in the archive section, and the passage number acquiring and generating section can acquire or generate the passage number at the n-th observation time point, based on the passage presence/absence value at the acquired or generated (n-1)-th observation time point.

### (1-2-3-2) Function and effect of configuration in which passage presence/absence determiningsection determines that work for cells at (n-1) -th observation time point is non-passage work when the number of cells or confluency at n-th observation time point is at least predetermined lower threshold

Preferably, the cell observation information processing system according to the embodiment mode of the present invention is configured so that the activity data is the number of cells or confluency, and when the number of cells or confluency at the n-th observation time point is at least the lower threshold, the passage presence/absence determining section determines that the work for the cells at the (n-1)-th observation time point is the non-passage work.

As stated above, on the next observation day of an observation day when passage work was conducted, the activity data acquired by capturing images of cells in a new culture container falls short of a lower threshold of the predetermined confluency or the number of cells. On the observation day after the next observation day of that when passage work was conducted, the activity data acquired by capturing images of cells in a culture container becomes equal to or exceeds the lower threshold of the predetermined confluency or the number of cells.

Consequently, when the cell activity data on the current observation day is at least the predetermined lower threshold of the confluency or number of cells, the possibility that the work conducted for the cells at the last observation time point was the non-passage work is significantly high.

As with the cell observation information processing system according to the embodiment mode of the present invention, in the case where when the number of cells or confluency at the n-th observation time point is at least the predetermined lower threshold, the passage presence/absence determining section determines that the work for the cells at the (n-1)-th observation time point is the non-passage work, the passage presence/absence information acquiring section can acquire or generate the passage presence/absence value at the (n-1)-th observation time point even if the passage presence/absence value is not stored as the search tag in the archive section, and the passage number acquiring and generating section can acquire or generate the passage number at the n-th observation time point, based on the passage presence/absence value at the acquired or generated (n-1)-th observation time point.

### (1-2-3-3) Function and effect of configuration in which the passage presence/absence determining section determines that the work for the cells at the (n-1)-th observation time point is the passage work when the number of cells or confluency at the (n-1)-th observation time point is at least a predetermined upper threshold, and the number of cells or confluency at the n-th observation time point is lower than a predetermined lower threshold

Preferably, the cell observation information processing system according to the embodiment mode of the present invention is configured so that the activity data is the number of cells or confluency, and when the number of cells or confluency at the (n-1) -th observation time point is at least the predetermined upper threshold and the number of cells or confluency at the n-th observation time point is lower than the lower threshold, the passage presence/absence determining section determines that the work for the cells at the (n-1)-th observation time point is the passage work.

As stated above, typically, when the cell activity data acquired from a captured image is confirmed to reach the upper threshold of the predetermined confluency or number of cells, the user conducts the passage work for the cells. On the next observation day of an observation day when passage work was conducted, the activity data acquired by capturing images of cells in a new culture container falls short of the lower threshold of the predetermined confluency or the number of cells. On the observation day after the next observation day of that when passage work was conducted, the activity data acquired by capturing images of cells in a culture container becomes equal to or exceeds the lower threshold of the predetermined confluency or the number of cells. Consequently, when the cell activity data on the current observation day is lower than the predetermined lower threshold of the confluency or number of cells, the possibility that the work conducted for the cells at the last observation time point was the passage work is significantly high.

However, even when the current observation day is the observation time point two times after the passage work, a case where the cell activity data is lower than the predetermined lower threshold of the confluency or number of cells can occur even with a low probability. In such a case, if the passage presence/absence determining section determines that the work for the cells at the (n-1)-th observation time point is the non-passage work when the number of cells or confluency at the n-th observation time point is at least the predetermined lower threshold, the passage presence/absence value at the last observation time point is erroneously determined.

As with the cell observation information processing system according to the embodiment mode of the present invention, in the case where when the number of cells or confluency at the (n-1)-th observation time point is at least the predetermined upper threshold and the number of cells or confluency at the n-th observation time point is lower than the predetermined lower threshold, the passage presence/absence determining section determines that the work for the cells at the (n-1) -th observation time point is the passage work, the passage presence/absence information acquiring section can acquire or generate the passage presence/absence value at the (n-1) -th observation time point even if the passage presence/absence information is not stored as the search tag in the archive section, and the passage number acquiring and generating section can more accurately acquire or generate passage number at the n-th observation time point, based on the passage presence/absence value at the acquired or generated (n-1)-th observation time point.

### (1-2-3-4) Function and effect of configuration in which passage presence/absence determining section determines that work for the cells at (n-1) -th observation time point is passage work when the number of cells or confluency at n-th observation time point is reduced by at least predetermined difference threshold in comparison with the number of cells or confluency at (n-1) -th observation time point

Preferably, the cell observation information processing system according to the embodiment mode of the present invention is configured so that the activity data is the number of cells or confluency, and when the number of cells or confluency at the n-th observation time point is reduced by at least the predetermined difference threshold in comparison with the number of cells or confluency at the (n-1)-th observation time point, the passage presence/absence determining section determines that the work for the cells at the (n-1) -th observation time point is the passage work.

As stated above, typically, when the cell activity data acquired from a captured image is confirmed to reach the upper threshold of the predetermined confluency or number of cells, the user conducts the passage work for the cells. On the next observation day of an observation day when passage work was conducted, the activity data acquired by capturing images of cells in a new culture container falls short of a lower threshold, such as the predetermined confluency and the number of cells. Consequently, when the number of cells or confluency is reduced by at least the predetermined threshold in comparison with the number of cells or confluency at the last observation time point, the possibility that the work conducted for the cells at the last observation time point is the passage work is significantly high.

As with the cell observation information processing system according to the embodiment mode of the present invention, in the case where when the number of cells or confluency at the n-th observation time point is reduced by at least the predetermined threshold in comparison with the number of cells or confluency at the (n-1)-th observation time point, the passage presence/absence determining section determines that the work for the cells at the (n-1)-th observation time point is the passage work, the passage presence/absence information acquiring section can acquire or generate the passage presence/absence value at the (n-1)-th observation time point even if the passage presence/absence information is not stored as the search tag in the archive section, and the passage number acquiring and generating section can acquire or generate at the n-th observation time point, based on the passage presence/absence value at the acquired or generated (n-1)-th observation time point. As a result, the user can acquire the correct value as the passage number that is one of the search tags to be assigned to the observation information on the current observation day. The need of input of the passage number by the user is then negated, which can eliminate input errors of the passage number by the user.

### (1-3) Function and effect of configuration of storing passage presence/absence values at first to (n-1) -th observation time points as search tags

Preferably, the cell observation information processing system according to the embodiment mode of the present invention further includes a storing section that assigns the first to (n-1) -th passage presence/absence values acquired or generated by the passage presence/absence information acquiring and generating section, as search tags, to the respective first to (n-1) -th pieces of observation information acquired by the observation information acquiring section, and stores the first to (n-1)-th pieces of observation information assigned the search tags, in the archive section.

According to such a configuration, the passage presence/absence information acquiring and generating section acquires or generates, as the previous passage presence/absence information, the first to (n-1) -th passage presence/absence values that are each one of search tags assigned to the first to (n-1)-th pieces of observation information stored in the archive section, and the passage number acquiring and generating section acquires or generates the n-th passage number based on the previous passage presence/absence information, thereby allowing the user to acquire a correct value as the passage number that is one of the search tags to be assigned to the observation information on the current observation day without any operation of confirming the work details for the cells conducted on the current observation day. The need of input of the passage number by the user is then negated, which can eliminate input errors of the passage number by the user. Furthermore, the necessity of operation for confirming the work details for the cells conducted on the current observation day is negated, which can reduce efforts and time for user's operation required to acquire the search tag to be assigned to the observation information on the cells and the like.

### (1-3-1) Function and effect of configuration of storing passage presence/absence values at first to (n-1) -th observation time points as search tags, acquiring or generating pieces of passage presence/absence information on the same cells at first to (n-1)-th observation time points, and acquiring or generating the n-th passage number based on passage presence/absence information

Preferably, the cell observation information processing system according to the embodiment mode of the present invention is configured so that the storing section further assigns first to (n-1)-th pieces of observation information the respective pieces of cell identifying information for identifying the cells indicated by the pieces of observation information, as search tags, and stores the information in the archive section, the passage presence/absence information acquiring and generating section acquires the previous passage presence/absence information on the same cells from the archive section, based on the tags of the cell identifying information, and the passage number acquiring and generating section acquires or generates the n-th passage number, based on the previous passage presence/absence information on the same cells acquired or generated by the passage presence/absence information acquiring and generating section.

The cell identifying information is identifying information for identifying the cells to be observed, and more specifically, includes at least one of the cell name, cell kind, cell level information for identifying whether the cells are of the parent cell line to be cultured or of a subculture having been separated from and cut out of the parent cell line and the passage number of the cells.

A cell name is a name freely given by a user to the cells used for experiment and research.

A cell kind is a specific category into which the cells are classified.

There is large population of cell kinds, and the number of cell kinds is so large as several hundred or more. The user sometimes sets different observation days for different cell kinds to be cultured for experiment and research. In such a case, the previous passage presence/absence information on a cell specimen belonging to a cell kind other than that of a cell specimen belonging to the cell kind to be observed on the observation day is unnecessary to acquire or generate the n-th passage number of the cell specimen belonging to the cell kind to be observed by the user on the observation day. If the cell specimen belonging to the cell kind to be acquired or generated cannot be identified when the passage presence/absence information acquiring and generating section acquires or generates the previous passage presence/absence information, it is difficult to acquire or generate the n-th passage number of the cell specimen belonging to the cell kind correctly and efficiently.

In the cell identifying information, at least the notation of cell name can be freely defined by the user in some cases. The user sometimes assigns different cell names to multiple cell specimens belonging to the same cell kind according to research and experiment. In such a case, if the cell specimen that is assigned the cell name and to be acquired or generated cannot be identified when the passage presence/absence information acquiring and generating section acquires or generates the previous passage presence/absence information, it is difficult to acquire or generate correctly and efficiently the n-th passage number of the cell specimen assigned the cell name.

In the case, as with the cell observation information processing system according to the embodiment mode of the present invention, where the storing section further assigns first to (n-1) -th pieces of observation information the respective pieces of cell identifying information for identifying the cells indicated by the pieces of observation information as search tags and stores the information in the archive section, the passage presence/absence information acquiring and generating section acquires the previous passage presence/absence information on the same cells from the archive section based on the tags of the cell identifying information, and the passage number acquiring and generating section acquires or generates the n-th passage number based on the previous passage presence/absence information on the same cells acquired or generated by the passage presence/absence information acquiring and generating section, the n-th passage number of the cell specimen belonging to the cell kind and the cell specimen assigned the cell name can be correctly and efficiently acquired or generated.

### (1-3-1-1) Function and effect of configuration of storing passage presence/absence values at first to (n-1) -th observation time points as search tags, acquiring or generating pieces of passage presence/absence information on the same cells and the same user at first to (n-1)-th observation time points, and acquiring or generating the n-th passage number based on passage presence/absence information

Preferably, the cell observation information processing system according to the embodiment mode of the present invention is configured so that the storing section further assigns first to (n-1)-th pieces of observation information the respective pieces of user identifying information for identifying the user having observed the pieces of observation information, as search tags, and stores the information in the archive section, the passage presence/absence information acquiring and generating section acquires or generates the previous passage presence/absence information corresponding to the same cells and the same user from the archive section, based on the tags of the cell identifying information and the tags of the user identifying information, and the passage number acquiring and generating section acquires or generates the n-th passage number, based on the previous passage presence/absence information corresponding to the same cells and the same user and acquired or generated by the passage presence/absence information acquiring and generating section.

The user identifying information is identifying information for identifying the user who conducts cell observation, and more specifically, this is identifying information for identifying the very person concerned who inputs cell observation result or an acting person who conducts cell observation or inputs a cell observation result in place of the very person concerned.

In observation and experiment using cells, in accordance with the conventional custom, cell management tends to be conducted such that a particular limited user manages the cells throughout their entire lifecycle from birth to killing.

Under such observation and experiment circumstances in which each user independently conducts thorough management of cells, each user, in the situation of assigning search tags to acquired cell observation information, shows his or her particularity in notation of the search tags. Search tags with notations varying with particularities of individual users are convenient in use for the individual users who are accustomed to them.

As stated above, in the cell identifying information, at least the notation of cell name can be freely defined by each user in some cases. In such cases, the notations of cell names are sometimes customized according to users and the different users may assign different cell names even for cell specimens belonging to the same cell kind. On the contrary, different users can sometimes assign the same cell name to different cell specimens. In such cases, the previous passage presence/absence information on a cell specimen under another user's management other than the cell specimen under management by the very user concerned is unnecessary to acquire or generate the n-th passage number of the cell specimen to be observed by the user on the observation day irrespective of the cell name assigned to the cell specimen. In such cases, if the cell specimen that is under the user's management and is a target to be acquired or generated cannot be identified when the passage presence/absence information acquiring and generating section acquires or generates the previous passage presence/absence information, it is difficult to acquire or generate correctly and efficiently the n-th passage number of the cell specimen under the user's management if different users have assigned the same cell name to different cell specimens, for example.

In the case, as with the cell observation information processing system according to the embodiment mode of the present invention, where the storing section further assigns first to (n-1) -th pieces of observation information the respective pieces of user identifying information for identifying users having observed the pieces of observation information as search tags and stores the information in the archive section, the passage presence/absence information acquiring and generating section acquires or generates the previous passage presence/absence information corresponding to the same cells and the same user from the archive section based on the tags of the cell identifying information and the tags of the user identifying information, and the passage number acquiring and generating section acquires or generates the n-th passage number based on the previous passage presence/absence information corresponding to the same cells and the same user acquired or generated by the passage presence/absence information acquiring and generating section, the n-th passage number of the cell specimen under management by the very user concerned can be correctly and efficiently acquired or generated even if different users assign the same cell name to the different cell specimens.

### (2) Function and effect of configuration of passage number acquiring and generating section

### (2-1) Function and effect of configuration in which previous passage presence/absence information is presented on screen to assist input operation of passage number by user and passage number acquiring and generating section acquires or generates passage number input by user as n-th passage number

Preferably, the cell observation information processing system according to the embodiment mode of the present invention further includes a passage presence/absence information output section that outputs, to the presenting section, the previous passage presence/absence information acquired or generated by the passage presence/absence information acquiring and generating section. The presenting section is configured to present, to the user, the previous passage presence/absence information output by the passage presence/absence information output section, and to accept an input of the passage number from the user. The passage number acquiring and generating section is configured to acquire, as the n-th passage number, the passage number input through the presenting section from the user referring to the previous passage presence/absence information presented by the presenting section.

As stated above, in the case where the cell observation information at each time point is compiled into a database, the image of the cells on the current observation day on which the passage work is to be conducted is acquired before the passage work. As a result, the observation day on which the passage number of the cell specimen is to be incremented and stored in the database file deviates from the observation day on which the cell passage work is actually conducted. Consequently, if the user tries to input the passage number manually through a screen or the like where a search tag to be assigned to observation information is set, on the current observation day when the user conducted predetermined maintenance work for cells, on the basis of memory and recognition of the maintenance work having been conducted for cells at the last observation time point, misunderstanding on passage number counting and misunderstanding on the details of maintenance work conducted for the cells occur, and the passage number is thus prone to be erroneously input.

If the user is allowed to confirm information on maintenance work conducted for the cells at the last observation time point when he or she manually inputs the passage number on a screen or the like where a search tag to be assigned to the observation information is set, it is considered that misunderstanding on passage number counting and misunderstanding on the details of maintenance work conducted for the cells can be prevented and erroneous input of the passage number can be prevented.

In the case of configuration, as with the cell observation information processing system according to the embodiment mode of the present invention, where the system further includes a passage presence/absence information output section that outputs, to the presenting section, the previous passage presence/absence information acquired or generated by the passage presence/absence information acquiring and generating section, the presenting section is configured to present, to the user, the previous passage presence/absence information output by the passage presence/absence information output section, and to accept an input of the passage number from the user, and the passage number acquiring and generating section is configured to acquire, as the n-th passage number, the passage number input through the presenting section from the user referring to the previous passage presence/absence information presented by the presenting section, the passage presence/absence information in the information on the maintenance work conducted to the cells at the last observation time point can be confirmed by the user, which can prevent the user from misunderstanding the details of the maintenance work conducted to the cells and from erroneously inputting the passage number.

### (2-1-1) Function and effect of configuration in which previous passage presence/absence information and history information on passage/non-passage work is presented on screen to assist input operation of passage number by user and passage number acquiring and generating section acquires or generates passage number input by user as n-th passage number

Preferably, in the cell observation information processing system according to the embodiment mode of the present invention, the configuration is made so that the passage presence/absence information output section further outputs, to the presenting section, history information on passage/non-passage work indicated by the previous passage presence/absence information, that the presenting section further presents, to the user, the history information output by the passage presence/absence information output section, and that the passage number acquiring and generating section acquires, as the n-th passage number, the passage number input through the presenting section from the user referring to the previous passage presence/absence information and the history information presented by the presenting section.

The history information here contains, for example, at least one of dates identified with previous passage work for cells and passage numbers on the dates identified with the previous passage work for cells.

In the case of configuration, as with the cell observation information processing system according to the embodiment mode of the present invention, where the passage presence/absence information output section further outputs, to the presenting section, history information on passage/non-passage work indicated by the previous passage presence/absence information, the presenting section further presents, to the user, the history information output by the passage presence/absence information output section, and the passage number acquiring and generating section acquires, as the n-th passage number, the passage number input through the presenting section from the user referring to the previous passage presence/absence information and the history information presented by the presenting section, the user is allowed to confirm not only the passage presence/absence information but also history information that contains dates identified with previous passage work for the cells and passage numbers on the dates identified with the previous passage work for the cells, as information on the last maintenance work conducted for the cells. This configuration can thus prevent the user from misunderstanding the details of the maintenance work conducted to the cells, from misunderstanding passage number counting, and from erroneously inputting the passage number.

### (2-1-2) Function and effect of configuration in which presentation of previous passage presence/absence information on screen supports input operation of passage number by user, passage number acquiring and generating section automatically generates passage number for checking n-th input, and notification of warning information on input of passage number can be issued to user based on the passage number input by the user and on the n-th passage number for checking input automatically generated

Preferably, in the cell observation information processing system according to the embodiment mode of the present invention, the passage number acquiring and generating section further automatically generates the passage number for checking the n-th input based on the previous passage presence/absence information, besides the passage number input from the user, and the system further includes a notification section that can notify the user of warning information on an input of the passage number, based on the passage number input by the user and on the n-th passage number for checking input automatically generated by the passage number acquiring and generating section.

Preferably, in the cell observation information processing system according to the embodiment mode of the present invention, the notification section notifies the user of the warning information when the passage number input by the user and the n-th passage number for checking the input automatically generated by the passage number acquiring and generating section are different from each other.

According to such a configuration, if a doubt arises about user' s erroneous input of the passage number, the user is allowed to confirm the passage number, thereby further preventing the user from erroneously input the passage number.

### (2-2) Function and effect of configuration in which passage number acquiring and generating section automatically generates n-th passage number based on passage presence/absence value at previous observation time point and previous passage number

Preferably, in the cell observation information processing system according to the embodiment mode of the present invention, the passage number acquiring and generating section generates, as previous passage numbers, passage numbers corresponding to passage presence/absence values at respective observation time points included in previous passage presence/absence information acquired or generated by the passage presence/absence information acquiring and generating section, and further generates the n-th passage number based on the previous passage presence/absence information and the previous passage numbers.

Such a configuration negates the need for user's operation of inputting the passage number, and can prevent the user from erroneously inputting the passage number.

### (2-2-1) Function and effect of configuration in which passage number acquiring and generating section automatically generates n-th passage number using passage presence/absence value at last ((n-1)-th) observation time point

Preferably, in the cell observation information processing system according to the embodiment mode of the present invention, the previous passage presence/absence information contains the (n-1) -th passage presence/absence value at the (n-1) -th observation time point, the previous passage number contains the (n-1)-th passage number at the (n-1) -th observation time point, and the passage number acquiring and generating section generates the n-th passage number basedonthe (n-1)-th passage presence/absence value and the (n-1)-th passage number.

As stated above, the cell passage number as the search tag to be stored in the database file on the current observation day is defined according to the passage presence/absence value and the passage number stored in the database file at the last observation time point.

In the case, as with the cell observation information processing system according to the embodiment mode of the present invention, where the previous passage presence/absence information contains the (n-1)-th passage presence/absence value at the (n-1)-th observation time point, the previous passage number contains the (n-1)-th passage number at the (n-1)-th observation time point, and the passage number acquiring and generating section generates the n-th passage number based on the (n-1)-th passage presence/absence value and the (n-1)-th passage number, the passage number on the current observation day can be automatically calculated.

Preferably, in the cell observation information processing system according to the embodiment mode of the present invention, the passage number acquiring and generating section generates, as the n-th passage number, a value acquired by adding one to the (n-1)-th passage number, when the (n-1)-th passage presence/absence value indicates the passage work.

Preferably, in the cell observation information processing system according to the embodiment mode of the present invention, the passage number acquiring and generating section generates a number identical to the (n-1)-th passage number as the n-th passage number, when the (n-1) -th passage presence/absence value indicates the non-passage work.

### (2-2-2) Function and effect of configuration in which passage number acquiring and generating section can automatically generate n-th passage number using passage presence/absence value at second last or earlier ((n-2)-th or smaller ordinal-numbered) observation time point

Preferably, in the cell observation information processing system according to the embodiment mode of the present invention, the previous passage presence/absence information contains the (n-1) -th passage presence/absence value at the (n-1) -th observation time point and an (n-2)-th or smaller ordinal-numbered passage presence/absence value at an (n-2)-th or earlier observation time point, and the passage number acquiring and generating section identifies the observation time point at which the most recent passage work was conducted for the cells among the previous observation time points and generates the n-th passage number based on the passage number at the observation time point identified by the most recent passage work.

As stated above, in the case where the cell observation information at each time point is compiled into a database, the image of the cells on the current observation day when the passage work is to be conducted is acquired before the passage work. As a result, the observation day on which the passage number of the cell specimen is to be incremented and stored in the database file deviates from the observation day on which the cell passage work is actually conducted. Consequently, the passage number at the observation time point after the most recent observation day when the passage presence/absence value indicating the passage work was stored has a value larger by one than the passage number on the most recent observation day when the passage presence/absence value indicating the passage work was stored.

In the case where, as with the cell observation information processing system according to the embodiment mode of the present invention, where the previous passage presence/absence values include the (n-1)-th passage presence/absence value at the (n-1)-th observation time point and an (n-2)-th or smaller ordinal-numbered passage presence/absence value at an (n-2) -th or earlier observation time point, and the passage number acquiring and generating section identifies the observation time point at which the last passage work was conducted for the cells among the previous observation time points and generates the n-th passage number based on the passage number at the observation time point identified by the most recent passage work, the passage number on the current observation day can be automatically calculated without using the passage presence/absence value or the passage number at the (n-1)-th observation time point.

Preferably, in the cell observation information processing system according to the embodiment mode of the present invention, the passage number acquiring and generating section generates, as the n-th passage number, a value acquired by adding one to the passage number at the observation time point identified by the most recent passage work.

### (2-2-3) Function and effect of configuration in which passage number acquiring and generating section presents warning information to user based on result of comparison between elapsed days and predetermined reference number of days, the elapsed days being from the observation time point identified as a time point at which the most recent passage work was conducted to the n-th observation time point among k-th (k is one or more integers ranging from one to n-1, inclusive) observation time points

Preferably, in the cell observation information processing system according to the embodiment mode of the present invention, the previous passage presence/absence information contains each of k-th (k is one or more integers ranging from one to n-1, inclusive) passage presence/absence values at corresponding one of one or more k-th observation time point, the passage number acquiring and generating section identifies whether the work for the cells at each of the k-th observation time points is passage work, based on the corresponding each of the k-th passage presence/absence values, the system further includes a warning information output section that can output warning information to the presenting section based on elapsed days and the predetermined reference number of days, the elapsed days being from the most recent observation time point identified by the passage number acquiring and generating section that the passage work was conducted at this time point to the n-th observation time point among the k-th observation time points, and the presenting section presents, to the user, the warning information output by the warning information output section.

Preferably, in the cell observation information processing system according to the embodiment mode of the present invention, the predetermined reference number of days is the reference value of the cell passage interval days.

Preferably, in the cell observation information processing system according to the embodiment mode of the present invention, the reference value of the cell passage interval days is defined based on the previous cell passage interval days. Such a reference value of the cell passage interval days may be, for example, the average value of the previous cell passage interval days.

Preferably, in the cell observation information processing system according to the embodiment mode of the present invention, the reference value of the cell passage interval days is defined for each of kinds of cells.

A case may arise where even though the passage work was actually conducted at a previous observation time point, the user forgets to store the passage presence/absence value as the search tag together with the observation information in the database file.

Typically, the interval of passage work for cells is a substantially constant number of days. Consequently, if the number of elapsed days from the most recent observation time point at which the previous passage presence/absence information was stored to the current observation day is identified, use of the interval of passage work conducted for the cells as the reference value of the cell passage interval days allows for estimating presence or absence of an observation day on which the user forgot to store the passage presence/absence value on the working day in the database file even though the passage work was conducted in a period to the current observation day.

In the case, as with the cell observation information processing system according to the embodiment mode of the present invention, where the passage number acquiring and generating section identifies whether the work for the cells at each of the k-th observation time points is passage work, based on the corresponding each of the k-th passage presence/absence values, the system further includes a warning information output section that can output warning information to the presenting section based on elapsed days and the predetermined reference number of days, the elapsed days being from the most recent observation time point identified by the passage number acquiring and generating section that the passage work was conducted at this time point to the n-th observation time point among the k-th observation time points, and the presenting section presents, to the user, the warning information output by the warning information output section, the user's attention is drawn to prevention of forgetting storage into the database file and also to whether the value of the passage number on the current observation day is correct in case the user did not store the passage presence/absence value as the search tag together with the observation information in the database file at a previous observation time point.

The reference value of the cell passage interval days as the predetermined reference number of days may be the number of days freely designated by the user.

### (2-2-3-1) Function and efect of configuration in which when elapsed days from most recent observation time point at which the passage work was conducted to n-th observation time point is at least the predetermined reference number of days, warning information is presented to user

Preferably, in the cell observation information processing system according to the embodiment mode of the present invention, the warning information output section outputs the warning information to the presenting section, when the elapsed days from the most recent observation time point at which the passage work is identified to have been conducted by the passage number acquiring and generating section to the n-th observation time point is at least the predetermined reference number of days.

As stated above, if the predetermined reference number of days elapsed from the most recent observation time point identified that the passage work was conducted from the previous passage presence/absence information acquired by the passage presence/absence information acquiring and generating section to the current observation day, it can be assumed that even though the passage work was conducted at the predetermined observation time point before the current observation day, the fact that the passage work was conducted has not been stored in the database file.

In this case, in the database file on the current observation day, the passage number is required to be added to the passage number at the most recent observation time point that can be identified that the passage work was conducted from the previous passage presence/absence information acquired by the passage presence/absence information acquiring and generating section.

However, there can be a case where according to some value of the elapsed days from the most recent observation time point that can be identified that the passage work was conducted from the previous passage presence/absence value acquired by the passage presence/absence value acquiring and generating section to the most recent observation time point, there can be multiple observation time points at which, even though the passage work was conducted, the fact of passage work was not stored in the database file before the current observation day. Consequently, the correct passage number cannot be calculated only and simply by adding one to the value of the passage number.

In the case, as with the cell observation information processing system according to the embodiment mode of the present invention, where the warning information output section presents the warning information to the user when the number of elapsed days from the observation time point identified by the passage number acquiring and generating section that the most recent passage work was conducted to the n-th observation time point is at least the predetermined reference number of days, notification of presence of the observation day on which the fact of conducting the passage work was not stored in the database file can be issued to the user to draw the attention of whether the value of the passage number on the current observation day is correct, and can draws the user's attention for preventing lack of storage into the database file.

### (3) Function and efect of configuration on timing of acquiring and generating passage presence/absence information and passage number (3-1) Function and efect of configuration of generating passage number in real time every time observation information is acquired

Preferably, in the cell observation information processing system according to the embodiment mode of the present invention, the passage number acquiring and generating section sequentially acquires or generates the n-th passage number based on the previous passage presence/absence information, every time the current observation information is sequentially acquired by the observation information acquiring section (updated so as to increment the n value by one).

Preferably, in the cell observation information processing system according to the embodiment mode of the present invention, the passage presence/absence information acquiring and generating section acquires or generates in bulk at least one among the first to (n-1) -th passage presence/absence values included in the previous passage presence/absence information.

### (3-2) Function and efect of configuration of generating passage numbers in bulk after observation information is acquired

Preferably, in the cell observation information processing system according to the embodiment mode of the present invention, the passage number acquiring and generating section acquires or generates in bulk at least one of the first to n-th passage numbers at at least one of the first to n-th observation time points, based on the previous passage presence/absence information.

Thus, in the case where the time point of assigning the tag of the passage number is configured to be different from the time point at which the observation information is acquired, is assigned the search tag and is stored in the archive section, the process for acquiring or generating the passage number to be assigned as the search tag to the observation information acquired at the time when the cell observation information is acquired can be omitted. Consequently, the time period during which the cells are left out of an incubator can be reduced, which can reduce damage to be applied to the cells when the observation information is acquired to the minimum. In the case of intervention of user's operation for acquiring or generating the passage numbers in bulk, the time point of user's operation is different from the time point for acquiring the observation information. The difference prevents the cells from being damaged. Consequently, the user can perform an operation where input errors of the passage number are checked in a sufficient time.

### (4) Functions and effects of other configurations

Preferably, the cell observation information processing system according to the embodiment mode of the present invention, further includes a storing section that assigns the n-th passage number acquired or generated by the passage number acquiring and generating section, as a search tag, to the n-th observation information, and stores the n-th observation information assigned the search tag, in the archive section.

According to such a configuration, as stated above, in the case where the n-th (i.e., on the current observation day) passage presence/absence value accepted from the user is assigned as the search tag to the n-th observation information acquired by the observation information acquiring section and stored in the archive section, the passage presence/absence value and passage number stored in the archive section this time can be used as the previous (i.e., the updated (n-1)-th) passage presence/absence value and passage number at the next observation time point. Consequently, at the (i.e., the updated n-th) observation time point at the next time, the passage number acquiring and generating section can automatically acquire or generate the n-th passage number based on the (n-1)-th passage presence/absence value and passage number.

Preferably, the cell observation information processing system according to the embodiment mode of the present invention further includes a search tag output section that outputs the search tag to the presenting section, and a presenting section that presents, to the user, the search tag output by the search tag output section.

In the case where the cell observation information processing system thus includes the presenting section, the user can confirm the search tag to be assigned to the observation information displayed through the presenting section included in the cell observation information processing system, without using a presenting section other than the cell observation information processing system.

Embodiment modes of the present invention will be explained below with reference to the drawings.

### First embodiment mode

FIG. 1 is a block diagram that conceptually shows the basic configuration of a cell observation information processing system according to the present invention. FIG. 2 is an explanatory diagram that shows the configuration of cell observation information processing system according to a first embodiment mode of the present invention.

As shown FIGs. 1 and 2, the cell observation information processing system 10 according to the first embodiment mode includes a passage presence/absence information acquiring and generating section 11a, a passage number acquiring and generating section 11b, and a control section 25. In FIGs. 1 and 2, reference numeral 1 denotes a cell observation information processing installation, reference numeral 12 denotes a storing section, reference numeral 13 denotes an archive section, reference numeral 18 denotes a monitor serving as a presenting section, reference numeral 16 denotes passage presence/absence selecting section, and reference numeral 20 denotes an observation information acquiring section. In FIG. 2, the cell observation information processing system 10 is configured as a stand-alone system with the passage presence/absence information acquiring and generating section 11a, the passage number acquiring and generating section 11b, and the control section 25, together with the storing section 12, the archive section 13, the presenting section 18, the passage presence/absence selecting and ordering section 16, and the observation information acquiring section 20. The cell observation information processing installation 1 is configured, for example, of a microscope apparatus provided with an image capture apparatus and a computer provided with a database.

The passage presence/absence information acquiring and generating section 11a acquires or generates previous passage presence/absence information via the observation information acquiring section 20 including previous passage presence/absence information that contains at least one passage presence/absence value among the first to (n-1)-th passage presence/absence values that indicate whether work for the cell at the first to (n-1) -th observation time points among the first to n-th observation time points (n is an integer at least two) at which the first to n-th pieces of observation information including items indicating a cell observation result including a cell images, activity data indicating the cell activity, such as the number of cells, confluency, morphology, and a viability, and an observation name are acquired in a time series manner.

In the following description of each embodiment mode of the present invention, the n-th observation time point is the current observation day.

The observation information acquiring section 20 is configured to acquire the first to n-th pieces of observation information (n is an integer at least two) that includes items indicating cell observation results including cell images, the activity data indicating the cell activity, such as the number of cells, confluency, morphology and a viability, and the observation name, in response to acquisition orders at the first to n-th observation time points. More specifically, the observation information acquiring section 20 is actuated when a user inputs an observation name and pushes an order button for observation information acquisition on an image-capture order screen (not shown in the drawings) provided in the installation 1, makes an image capture apparatus (not shown in the drawings) provided in the installation 1 image-capture a specimen containing cells placed at an observation position, detects activity data that show cell activity in terms of cell confluency, morphology, a viability or so by subjecting a cell image as captured to image processing and analysis processing via an image processing section and an image analyzing section (not shown in the drawings), and temporarily stores, in a storage area (not shown in the drawings) in the installation 1, a piece of observation information including the cell image, the activity data and the observation name.

The passage presence/absence information acquiring and generating section 11a generates the n-th passage presence/absence value based on the selection order accepted from the user via the presenting section 18.

The passage number acquiring and generating section 11b at least acquires or generates the n-th passage number at the n-th observation time point automatically or through an operation by the user, based on the previous passage presence/absence information acquired or generated by the passage presence/absence information acquiring and generating section 11a.

For example, the presenting section 18 displays a passage work presence/absence button 18e (passage work presence button 18e-1, and passage work absence button 18e-2) on a screen as shown in FIG. 3A, and accepts a selection order on whether the work for the cells at the n-th observation time point is passage work from the user through the passage presence/absence selecting and ordering section 16. In the example of FIG. 3A, the presenting section 18 is configured to complete the selection order through the passage work presence/absence button 18e when the user selects any of the passage work presence button 18e-1 and the passage work absence button 18e-2 through the passage presence/absence selecting and ordering section 16. For example, as shown in FIG. 3B, the presenting section 18 includes a confirmation button 18f for the user in addition to the passage work presence/absence button 18e (passage work presence button 18e-1 and passage work absence button 18e-2), and may be configured to complete the selection order through the passage work presence/absence button 18e when the user presses the confirmation button 18f through the passage presence/absence selecting and ordering section 16.

The passage presence/absence selecting and ordering section 16 includes, for example, a mouse, keyboard, touch panel and the like, and has a function of allowing for selecting or pressing of the passage work presence/absence button 18e (and confirmation button 18f in the example of FIG. 3B) displayed on the presenting section 18.

In FIGs. 3A and 3B, reference numeral 18a denotes a display item of user identifying information, reference numeral 18b denotes a display item of date and time information, reference numeral 18c denotes an activity data display screen, and reference numeral 18d denotes a search tag input screen.

The search tag input screen 18d is configured to allow the user to input, for example, cell identifying information including the cell name, cell level information, and passage number, as search tags. The search tag input screen 18d is configured to initially display the cell name and the cell level information at the last observation time point stored in the archive section 13, the n-th passage presence/absence value generated by the passage presence/absence information acquiring and generating section 11a, and the n-th passage number generated by the passage number acquiring and generating section 11b.

The cell observation information processing system 10 of the first embodiment mode includes a search tag output section not shown in the drawings, and is configured to cause the search tag output section to output these pieces of information to the search tag input screen 18d.

The storing section 12 assigns the n-th passage presence/absence value acquired by the passage presence/absence information acquiring and generating section 11a and the n-th passage number acquired or generated by the passage number acquiring and generating section 11b, as search tags, to the n-th observation information acquired by the observation information acquiring section 20, and stores the n-th observation information assigned the search tag in the archive section 13.

When the control section 25 accepts, via the presenting section 18, the selection order on whether the work is the passage work through the passage presence/absence selecting and ordering section 16 from the user, the control section 25 controls the storing section 12 to store the n-th observation information assigned the search tag into the archive section 13. In the example of FIG. 3A, the selection order on whether the work is the passage work is completed when any passage work presence/absence button 18e (passage work presence button 18e-1 and passage work absence button 18e-2) is selected. On the other hand, in the example of FIG. 3B, the selection order on whether the work is the passage work is not completed when only any passage work presence/absence button 18e (passage work presence button 18e-1 and passage work absence button 18e-2) is selected but is completed when the confirmation button 18f is pressed.

Although illustration in the diagrams is omitted for convenience' sake, the cell observation information processing system 10 of the first embodiment mode includes a tag acquiring section for acquiring a search tag to be assigned to observation information, besides the passage presence/absence information acquiring and generating section 11a and the passage number acquiring and generating section 11b.

The tag acquiring section acquires, as the search tag for retrieving the observation information including items indicating the cell observation result, such as the cell image, the activity data indicating the cell activity, such as the number of cells, confluency, morphology, and a viability, the observation name acquired through the observation information acquiring section 20 in response to the acquisition order at one time point, at least one of tags among user identifying information for identifying the user observing the cells, the cell identifying information for identifying the cells to be observed, the date and time information for identifying the date and time when the observation information is acquired, information on work by the user for maintaining the cells, apparatus identifying information for identifying the apparatus used to acquire the observation information, activity data variation information, and an image tag indicating a cell image.

To be specific, the tag acquiring section is configured to allow for acquiring the user ID selected and input by the user on a user ID input screen (not shown in the drawings) provided for the cell observation in formation processing installation 1, and the cell level information (parent cell line or subculture), and the cell name and the like input by the user into the tag input field on the search tag input screen 18d shown in FIGs. 3A and 3B, as search tags. The tag acquiring section may be configured to acquire, as a search tag, the passage number input by the user into the tag input field on the search tag input screen 18d. In each embodiment mode of the present invention, for convenience' sake, the description is made assuming that the passage number acquired or generated by the passage number acquiring and generating section 11b is used as the search tag.

Furthermore, the tag acquiring section acquires the observation date and time automatically generated by the apparatus as date and time information for identifying the date and time when the observation information was acquired; the date and time may be the image capture date and time recorded as a log in the cell image in each piece of the observation information acquired through the observation information acquiring section 20. Moreover, the tag acquiring section is configured to allow for acquiring the image tag from the cell image in each of the pieces of observation information acquired through the observation information acquiring section 20.

Furthermore, the storing section 12 is configured to assign the tag acquired from the tag acquiring section, as the search tag for retrieving the observation information, to the observation information acquired through the observation information acquiring section 20 in response to the acquisition order at each time point, and record the observation information assigned the search tag in the archive section 13.

More specifically, the information on user's work for cell maintenance contains at least one of the work detail, dilution rate, container's type or size, and container address where the cells are stored.

The dilution rate is defined as an amount of thinning in a situation where cells cultured in a container are thinned for passage or experiment.

The image tag is a tag for indicating a cell image, and more specifically, an image displayable in a small size in an image selection and input screen (not shown in the drawings) or information containing an image and its corresponding name integrally combined together, for example.

The activity data variation information is expressed, for example by an amount of variation (that is, a rate of variation) between respective activity data (for example, cell confluency, the number of cells or a viability) at a plurality of time points, and can be shown, for example by a slope of a plotted line in a graph.

The archive section 13 has, as shown in FIG. 4, one or more records of the search-tagged observation information. Each record is created in response to image capture of cell images at each time point, where observation information corresponding to the image capture of the cell image at one time point is related with search tags for retrieving the observation information.

While the user ID is used as the user identifying information in this embodiment mode for convenience' sake, the user identifying information is not necessarily limited to the user ID; for example, any letters or symbols that can distinguish a certain user from other users, such as a name or nickname of the user, may work.

The cell observation information processing installation 1 is provided with, for example, a system log-in screen not shown in the drawings. If a user inputs a log-in ID and a password of the system in the system log-in screen, a user-ID input screen not shown in the drawings is displayed. In the user-ID input screen, if the user inputs, by selection, his or her user ID as the user identifying information, the user ID is temporarily stored in the storage area (not shown in the drawings) in the installation 1 and an image-capture order screen (not shown in the drawings) is displayed so that the user can perform operation for image-capture order. The user-ID input screen not shown in the drawings may be configured to function as a system log-in screen also by letting a user perform selection and input operation for a log-in ID and a password of the system together with input operation by selection of a user ID.

In reference to FIGs. 5 and 6, an explanation is made on the processing procedure from acquisition of the observation information, to acquisition of previous passage presence/absence information, generation of the passage number on the current observation day based on the previous passage presence/absence information, a selection order by the user through the presenting section 18 on whether the work for the cells on the current observation day is the passage work, generation of the passage presence/absence value on the current observation day based on the selection order, and storage, in the archive section 13, of the passage presence/absence value on the current observation day, and observation information to which the search tag including the passage number is assigned, using the cell observation information processing system 10 according to the first embodiment mode thus configured.

The procedure of condition management of cells cultured by users is divided into a step of storing observation information on the cells under culture into the archive section 13 and a step of searching and retrieving desired observation information on cells from the archive section 13 and checking conditions of the cells. The acquisition of previous passage presence/absence information, the generation of the passage number on the current observation day based on the previous passage presence/absence information, the selection order by the user through the presenting section 18 on whether the work for the cells on the current observation day is the passage work, the generation of the passage presence/absence value on the current observation day based on the selection order are performed at a step of storing the observation information on the cells under culture in the archive section 13.

First, the user inputs a log-in ID and a password of the system in the system log-in screen (not shown in the drawings). Then, the user ID input screen not shown in the drawings is displayed. Then, the user inputs his or her own user ID (in the example of FIG. 5, "USER 1") by selection. The user ID is temporarily stored in the storage area in the installation 1 and an image-capture order screen (not shown in the drawings) is displayed, to allow the user to perform operation for image-capture order.

Then, in the image-capture order screen (not shown in the drawings), the user inputs an observation name and pushes an order button for ordering acquisition of observation information. Then, the observation information acquiring section 20 makes the image capture apparatus (not shown in the drawings) image-capture a specimen containing cells placed at the observation position, and detects activity data indicating the cell activity in terms of number of cells, confluency, morphology, a viability or so, for example by subj ecting a cell image as captured to image processing and analysis processing via the image processing section and the image analyzing section (not shown in the drawings). This section then stores, in the temporal storage area (not shown in the drawings), observation information including the cell image, the activity data, and the observation name in the installation 1. In this way, the observation information at one time point is acquired (Step S1). It is noted that acquisition of observation information can be repeated a plurality of times for different acquisition time points.

After completion of input and observation image acquisition order by the user from the image-capture order screen and acquisition of observation information by the observation information acquiring section 20, the passage presence/absence information acquiring and generating section 11a acquires the previous passage presence/absence information including at least one passage presence/absence value (here, for convenience' sake, it is assumed that (n-1)-th passage presence/absence value at the last time) among the first to (n-1)-th passage presence/absence values indicating whether the work for the cells at the first to (n-1)-th observation time points stored in the archive section 13 (here, for convenience' sake, it is assumed that (n-1) -th observation time point at the last time) (Step S2).

Next, the passage number acquiring and generating section 11b generates the passage number on the current observation day based on the previous passage presence/absence information acquired (Steps S3 to S5).

To be specific, when the passage presence/absence value at the last observation time point is a value indicating "passage work" (Step S3), the passage number acquiring and generating section 11b generates a value acquired by adding one to the passage number at the last observation time point as the passage number at the current observation day (Step S4). When the passage presence/absence value at the last observation time point is a value indicating "non-passage work" (Step S3), the passage number acquiring and generating section 11b generates a value identical to the passage number at the last observation time point as the passage number at the current observation day (Step S5).

The presenting section 18 initially displays the cell level information at the last observation time point stored in the archive section 13, the cell name, and the passage number on the current observation day generated by the passage number acquiring and generating section 11b, and also displays a work presence/absence button 18e, on the search tag input screen 18d as shown in FIGs. 3A and 3B (Step S6).

The user inputs the cell level information for identifying whether the cells is of a parent cell line or of a subculture, and the cell name (and the passage number in some cases) on the search tag input screen 18d as shown in FIGs. 3A and 3B. The user selects the pas sage work presence/absence button 18e that is any of the passage work presence button 18e-1 and the passage work absence button 18e-2.

Upon input of the search tag onto the search tag input screen 18d, the tag acquiring section acquires, the cell level information for identifying whether the cells are of the parent cell line or of the subculture, the cell name (and the passage number in some cases) input by the user on the search tag input screen 18d, and the user ID, as search tags.

Upon selection of the passage work presence/absence button 18e (Step S7), the passage presence/absence information acquiring and generating section 11a generates the passage presence/absence value on the current observation day as search tags, based on the passage presence/absence button 18e selected by the user (Steps S8 to S10) .

To be specific, upon user's selection of the passage work presence button 18e-1 (Step S8), the passage presence/absence information acquiring and generating section 11a generates the passage presence/absence value that indicates that the work for the cells on the current observation day is "passage work" (Step S9). On the contrary, upon user's selection of the passage work absence button 18e-2 (Step S8), the passage presence/absence information acquiring and generating section 11a generates the passage presence/absence value that indicates that the work for the cells on the current observation day is "non-passage work" (Step S10).

At this time, with completion of user's selection and order operation on the passage work presence/absence button 18e (i.e., in the example of FIG. 3A, e.g., selection of any passage work presence/absence button 18e achieves completion; in the example of FIG. 3B, selection of any passage work presence/absence button 18e and subsequent pressing of the confirmation button 18f achieve completion) (Step S11), the control section 25 controls the storing section 12 to assigns not only the search tags acquired from the tag acquiring section but also the passage presence/absence value on the current observation day generated by the passage presence/absence information acquiring and generating section 11a and the passage number on the current observation day generated by the passage number acquiring and generating section 11b as search tags to the observation information, and stores the information in the archive section 13 (Step S12). In the example of FIG. 3B, until the user's selection and order operation for the passage work presence/absence button 18e is completed (i.e., the confirmation button 18f is pressed) with the search tag input screen 18d being displayed, the storing section 12 does not operate and the processes of Steps S7 to S11 are repeated according to the control by the control section 25. At this time, the user inputs the cell level information for identifying whether the cells are of the parent cell line or of the subculture, and the cell name, and performs a selection and order operation for the passage work presence/absence button 18e that is any of the passage work presence button 18e-1 and passage work absence button 18e-2.

In the example of FIG. 6, it is configured to switch display of the presenting section 18 so as to allow the user to input the tag and select the passage work presence/absence button 18e through the passage work presence/absence button 18 and the search tag input screen 18d every time the observation information acquiring section 20 acquires the observation information. Alternatively, it may be configured to switch display of the presenting section 18 so as to allow the user to input the tag and select the passage work presence/absence button 18e through the passage work presence/absence button 18e and the search tag input screen 18d after the observation information acquiring section 20 repeats acquisition of the observation information multiple times. As to timing of assigning the search tag, it may be configured to switch display of the presenting section 18 so as to allow the user to input the tag and select the passage work presence/absence button 18e through the search tag input screen 18d on which the passage work presence/absence button 18e is displayed, in real time after the observation information acquiring section 20 acquires the observation information. Alternatively, it may be configured to switch display of the presenting section 18 so as to allow the user to input the tag and select the passage work presence/absence button 18e through the passage work presence/absence button 18e and the search tag input screen 18d a certain period after the observation information acquiring section 20 acquires the observation information.

Here, generation of the passage presence/absence value and the passage number on the current observation day in the cell observation information processing system 10 according to the first embodiment mode is described further in detail using the example of observation information and search tags shown in a lower part of FIG. 5.

In the example in FIG. 5, the observation day on Aug. 1 is a day on which the passage work is conducted. On the observation day when the passage work is conducted (Aug. 1), the search tag is assigned to the cell observation information before passage work, and the information is stored in the database file that serves as the archive section 13.

Consequently, on Aug. 1, the observation information acquiring section 20 acquires observation information including an image (here, image a1) and the number of cells with respect to the cells having not been subjected to passage work. The user inputs information that includes the cell level information for identifying whether the cells are of a parent cell line or of a subculture, and the cell name, on the search tag input screen 18d. The passage number at the observation time point on Aug. 1 generated based on the passage presence/absence information at the observation time point before Aug. 1 stored in the archive section 13 is initially displayed on the search tag input screen 18d, as described later. Although not shown here, it is assumed that Aug. 1 is an observation day on which the passage work is conducted, and the passage presence/absence value on the observation time point immediately before Aug. 1 thus indicates "non-passage work" and the passage number is "4". The passage presence/absence information acquiring and generating section 11a acquires the passage presence/absence value "non-passage work" on the observation time point immediately before Aug. 1. Next, the passage number acquiring and generating section 11b generates the passage number on Aug. 1 based on the passage presence/absence value on the observation time point immediately before Aug. 1. The passage presence/absence value on the observation time point immediately before Aug. 1 indicates "non-passage work". Consequently, the value "4" identical to the passage number on the observation time point immediately before Aug. 1 is generated as the passage number on Aug. 1.

Aug. 1 is the observation day on which the passage work is conducted. Consequently, the user selects the passage work presence button 18e-1. Upon selection of the passage work presence button 18e-1, the passage presence/absence information acquiring and generating section 11a generates the value indicating "passage work" as the passage presence/absence value on Aug. 1.

When the user completes the selection order through the passage work presence/absence button 18e (here, the passage work presence button 18e-1), the storing section 12 assigns the cell name, the cell level information, the date of the current observation day and the like input by the user through the search tag input screen 18d and acquired by the tag acquiring section, and the passage presence/absence value and the passage number on the current observation day (Aug. 1) generated by the passage presence/absence information acquiring and generating section 11a and the passage number acquiring and generating section 11b, as search tags, to the observation information, and stores the information in the archive section 13.

Subsequently, the user conducts actual passage work.

The observation day, Aug. 2, is a day on which the non-passage work is conducted. On the observation day, Aug. 2, the observation time point, Aug. 1, is a previous (last) observation time point. On Aug. 2, which is the next observation time point of the observation day (Aug. 1) on which the passage work was conducted, the search tag is assigned to the cell observation information after passage work, and the information is stored in the database file that serves as the archive section 13.

Consequently, on Aug. 2, the observation information acquiring section 20 acquires observation information including an image (here, image a2) and the number of cells with respect to the cells having been subjected to passage work. The user inputs information that contains the cell level information for identifyingwhether the cells are of a parent cell line or of a subculture, and the cell name, on the tag input screen 18d. The passage number "5" at the observation time point on Aug. 2 generated based on the passage presence/absence value at the observation time point on Aug. 1 stored in the archive section 13 is initially displayed on the search tag input screen 18d, as described later. The passage presence/absence information acquiring and generating section 11a acquires the passage presence/absence value "passage work" on the observation time point, Aug. 1. Next, the passage number acquiring and generating section 11b generates a value "5", which is acquired by adding one to the passage number "4" at the observation time point on Aug. 1 as the passage number on Aug. 2, based on the passage presence/absence value "passage work" at the observation time point on Aug. 1.

Aug. 2 is the observation day on which the non-passage work is conducted. Consequently, the user selects the passage work absence button 18e-2. Upon selection of the passage work absence button 18e-2, the passage presence/absence information acquiring and generating section 11a generates the value indicating "non-passage work" as the passage presence/absence value on Aug. 2.

When the user completes the selection order through the passage work presence/absence button 18e (here, the passage work absence button 18e-2), the storing section 12 assigns the cell name, the cell level information, the date of the current observation day and the like input by the user through the search tag input screen 18d, and the passage presence/absence value and the passage number on the current observation day (Aug. 2) generated by the passage presence/absence information acquiring and generating section 11a and the passage number acquiring and generating section 11b, as search tags, to the observation information, and stores the information in the archive section 13.

Subsequently, the user conducts actual non-passage work.

According to the cell observation information processing system 10 of the first embodiment mode, the passage presence/absence information acquiring and generating section 11a acquires or generates the previous passage presence/absence information that contains at least one passage presence/absence value among the first to (n-1) -th passage presence/absence values indicating whether the work at the previous (first to (n-1)-th) observation time points are passage work, the passage number acquiring and generating section 11b acquires or generates at least n-th passage number at at least the n-th observation time point based on the previous passage presence/absence information acquired or generated by the passage presence/absence information acquiring and generating section, the passage presence/absence information acquiring and generating section11a further generates the n-th passage presence/absence value on the current observation day based on the selection order accepted from the user through the presenting section 18, and the presenting section 18 accepts, from the user, the selection order of whether the work for the cells at the n-th observation time point is the passage work. Consequently, the user can acquire a correct value as the passage number that is one of search tags to be assigned to the observation information at the next observation time point only by a simple operation, for example, pressing the selection button or the like to confirm the work details conducted to the cells on the current observation day. This negates the need for user's inputting the passage number and, in turn, negates user's input errors of the passage number, and can prevent the user from misunderstanding the work details conducted for the cells on the current observation day.

The cell observation information processing system 10 of the first embodiment mode further includes a control section that controls the storing section 12 to execute storage of the n-th observation information assigned the search tag into the archive section 13 when the selection order of whether the work is the passage work is accepted from the user through the presenting section 18. Consequently, in addition to the aforementioned advantageous effects, the user is allowed to confirm storage of the passage presence/absence value on the current observation day into the archive section 13 through the simple operation as described above.

Furthermore, in the cell observation information processing system 10 of the first embodiment mode, according to the example of FIG. 3B, the presenting section 18 allows acceptance of the selection order of whether the work for the cells at the n-th observation time point is the passage work from the user to be completed by an input of the confirmation button 18f. Consequently, before storage of the n-th observation information assigned the search tag by the storing section 12 into the archive section 13 is executed, the user can reconfirm the selection order of whether the work is the passage work. Therefore, user's misoperation of the selection order can be prevented.

The cell observation information processing installation 1 of FIG. 1 further includes a search criterion designation screen, not shown, besides the above configuration elements. Consequently, items can be designated as search criteria, among search tags assigned to the search-tagged observation information; the items are, for example at least one of the user ID as user identifying information, the cell name as cell identifying information, the cell level information (parent cell line or subculture), work details as information pertaining to user's work for maintaining the cells, the apparatus ID as apparatus identifying information, the amount of variation in activity as activity data variation information and the like.

The cell observation information processing system 10 includes a search criterion acquiring section and an observation data retrieving section, not shown. The search criterion acquiring section acquires, as search criteria, for example a combination of the activity data, date and time information and the like in addition to the items input by the user on the search criterion designation screen. The observation data retrieving section searches the archive section 13 using the search criteria acquired by the search criterion acquiring section, and retrieves, from the archive section, data that contains search-tagged observation information assigned the search tags matching with the search criteria. The data that contains the search-tagged observation information retrieved by the observation data retrieving section is displayed on a display screen, not shown, in a display manner according to the search criteria acquired by the search criterion acquiring section.

After user' s input of the search criteria on the search criterion designation screen, acquisition of the search criteria by the search criterion acquiring section, retrieval of data containing the search-tagged observation information by the observation data retrieving section, and display on the display screen, desired cell observation information can be searched for and retrieved from the archive section 13, and the process in a stage of checking the cell condition can be executed.

In the example of FIG. 2, the configuration of the cell observation information processing system 10 includes the passage presence/absence information acquiring and generating section 11a, the passage number acquiring and generating section 11b, and the control section 25. The configuration is not limitedto this example. Alternatively, for example, the configuration may further include the storing section 12.

### Second embodiment mode

FIG. 7 is an explanatory diagram that shows the configuration of a cell observation information processing system according to a second embodiment mode of the present invention. FIG. 8 is an explanatory diagram that conceptually shows a method of determining whether the work for the cells on the current observation day by a passage presence/absence determining section in the cell observation information processing system in FIG. 7 is passage work.

In the cell observation information processing system 10 according to the second embodiment mode, the passage presence/absence information acquiring and generating section 11a includes a passage presence/absence determining section 11a1.

The passage presence/absence determining section 11a1 determines whether the work for cells on the current observation day (i.e., the n-th observation time point) is passage work, based on the passage presence/absence determining information. The activity data (e.g., the number of cells or confluency) indicating the cell activity, for example may be used as the passage presence/absence determining information.

To be specific, for example, the passage presence/absence determining section 11a1 determines that the work for the cells on the current observation day is non-passage work when the number of cells or confluency on the current observation day is less than a predetermined upper threshold. Furthermore, for example, the passage presence/absence determining section 11a1 determines that the work for the cells on the current observation day is passage work when the number of cells or confluency on the current observation day is at least a predetermined upper threshold.

The passage presence/absence information acquiring and generating section 11a automatically creates a determination result by the passage presence/absence determining section 11a1 as the passage presence/absence value on the current observation day, not based on the selection order accepted from the user through the presenting section 18 as in the embodiment mode of FIG. 2.

The configuration of the passage number acquiring and generating section 11b is substantially identical to that of the cell observation information processing system 10 according to the first embodiment mode.

In reference to FIGs. 9 and 10, an explanation is made on the processing procedure from acquisition of the observation information, to acquisition of previous passage presence/absence information, generation of the passage number on the current observation day based on the previous passage presence/absence information, determination of whether the work for the cells on the current observation day is the passage work by the passage presence/absence determining section 11a1, generation of the passage presence/absence value on the current observation day based on the determination result, and storage, in the archive section 13, of the observation information to which the search tag including the passage presence/absence value and the passage number on the current observation day is assigned, using the cell observation information processing system10 according to the second embodiment mode thus configured.

For convenience' sake, description is made using an example where the cell activity data used as passage presence/absence determination data is the number of cells, and the predetermined upper threshold is the number of cells "45". For convenience' sake, description of the configuration and operation for acquiring the search tags, such as the cell name and cell level, is omitted.

Steps up to the input and observation image acquisition order by the user from the image-capture order screen, acquisition of observation information by the observation information acquiring section 20 (Step S1'), acquisition of previous passage presence/absence information by the passage presence/absence information acquiring and generating section 11a (Step S2'), generation of the passage number on the current observation day based on the previous passage presence/absence information by the passage number acquiring and generating section 11b (Steps S3' to S5') are substantially identical to those of the cell observation information processing system 10 according to the first embodiment mode.

Next, the passage presence/absence information acquiring section 11a causes the passage presence/absence determining section 11a1 to determine whether the work for the cells at the observation time point on the current observation day is passage work, based on the passage presence/absence determining information, and then generates the determination result as the passage presence/absence value (Steps S6' to S8').

To be specific, as shown in FIG. 8, in the example of the observation information and the search tag indicated in a lower part of FIG. 9, the number of cells is 50 that exceeds the predetermined upper threshold of 45 in the case where the current observation day is Aug. 1. In this case, the passage presence/absence determining section 11a1 determines that the work for the cells on the current observation day is passage work. The passage presence/absence information acquiring and generating section 11a generates the passage presence/absence value that indicates that the work for the cells on the current observation day (Aug. 1) is "passage work" (Steps S6' and Step S8').

In the case where the current observation day is Aug. 2, the number of cells is 10 that is less than the predetermined upper threshold of 45. In this case, the passage presence/absence determining section 11a1 determines that the work for the cells on the current observation day is non-passage work. The passage presence/absence information acquiring and generating section 11a generates the passage presence/absence value that indicates that the work for the cells on the current observation day (Aug. 2) is "non-passage work" (Steps S6' and S7').

Next, the storing section 12 assigns the passage presence/absence value and the passage number on the current observation day generated by the passage presence/absence information acquiring and generating section 11a and the passage number acquiring and generating section 11b as search tags to the observation information, and stores the information in the archive section 13 (Step S9').

According to the cell observation information processing system 10 of the second embodiment mode, the passage presence/absence information acquiring and generating section 11a includes the passage presence/absence determining section 11a1 that determines whether the work for the cells at the n-th observation time point, based on the passage presence/absence determining information at the n-th observation time point is passage work, and generates the determination result on whether the work is the passage work by the passage presence/absence determining section 11a1 as the n-th observation time point. Consequently, the n-th (i.e., current observation day) passage presence/absence value acquired or generated based on the determination result by the passage presence/absence determining section 11a1 is assigned as the search tag to the n-th observation information 1acquired by the observation information acquiring section 20, and stored in the archive section 13, thereby causing the passage presence/absence value stored in the archive section 13 this time to be the previous (i.e., updated (n-1)-th) passage presence/absence value at the next observation time point. Consequently, at the next observation time point, the passage presence/absence information acquiring and generating section 11a acquires or generates the previous passage presence/absence information, and the passage number acquiring and generating section 11b acquires or generates the passage number, based on the previous passage presence/absence information. As a result, the user can acquire the correct value as the passage number that is one of the search tags to be assigned to the new observation information at the next observation time point without any operation for confirming the work details for the cells conducted on the current observation day. The need of input of the passage number by the user is thus negated, which can eliminate input errors of the passage number by the user. Furthermore, the necessity of work for confirming the work details for the cells conducted on the current observation day is negated, which can reduce efforts and time for user' s operation required to acquire the tag to be assigned to the observation information on the cells and the like.

In the example of FIG. 7, the cell observation information processing system 10 includes the passage presence/absence information acquiring and generating section 11a, and the passage number acquiring and generating section 11b. However, the configuration is not limited to this example. Alternatively, for example, the configuration may further include the storing section 12.

### Third embodiment mode

FIG. 11 is an explanatory diagram that shows the configuration of a cell observation information processing system according to a third embodiment mode of the present invention. FIG. 12 is an explanatory diagram that conceptually shows a method of determining whether the work for the cells on the last observation time point by the passage presence/absence determining section in the cell observation information processing system in FIG. 11 is passage work.

In the cell observation information processing system 10 according to the third embodiment mode, the passage presence/absence information acquiring and generating section 11a includes a passage presence/absence determining section 11a1'.

The passage presence/absence determining section 11a1' determines whether the work for cells on the (n-1)-th observation time point is passage work, based on at least the passage presence/absence determining information on the current observation day (i.e., the n-th observation time point). As with the cell observation information processing system according to the second embodiment mode, the activity data (e.g., the number of cells or confluency) indicating the cell activity, for example may be used as the passage presence/absence determining information.

To be specific, for example, the passage presence/absence determining section 11a1' determines that the work for the cells at the last ((n-1)-th) observation time point is passage work when the number of cells or confluency on the current observation day is less than a predetermined lower threshold. For example, the passage presence/absence determining section 11a1' determines that the work for the cells at the last ((n-1)-th) observation time point is non-passage work when the number of cells or confluency on the current observation day is at least the predetermined lower threshold. Preferably, for example, the passage presence/absence determining section 11a1' determines that the work for the cells at the last ((n-1) -th) observation time point is the passage work when the number of cells or confluency at the last ((n-1) -th) observation time point is at least the predetermined upper threshold, and the number of cells or confluency on the current observation day is lower than the predetermined lower threshold.

The configuration of the passage number acquiring and generating section 11b is substantially identical to that of the cell observation information processing system 10 according to the first embodiment mode.

In reference to FIGs. 13 and 14, an explanation is made on the processing procedure from acquisition of the observation information, to determination by the passage presence/absence determining section 11a1' on whether the work for the cells on the previous observation time point is the passage work, generation of the previous passage presence/absence information based on the determination result, generation of the passage number on the current observation day based on the previous passage presence/absence information, and storage, in the archive section 13, of observation information assigned the search tag including the passage number on the current observation day, using the cell observation information processing system 10 according to the third embodiment mode thus configured.

Steps up to the input and observation image acquisition order by the user from the image-capture order screen, and acquisition of the observation information by the observation information acquiring section 20 (Step S1'') are substantially identical to those of the cell observation information processing system 10 according to the first embodiment mode.

Next, the passage presence/absence information acquiring section 11a causes the passage presence/absence determining section 11a1' to determine whether the work for the cells at the last observation time point is passage work, based on the passage presence/absence determining information on the current observation day, and then generates the determination result as the passage presence/absence value (Steps S2'' to S6'').

To be specific, as shown in FIG. 12, in the case where the current observation day is Aug. 1, the number of cells or confluency is at least the predetermined lower threshold. In this case, the passage presence/absence determining section 11a1' determines that the work for the cells at the last observation time point (the observation time point immediately before Aug. 1) is non-passage work. The passage presence/absence information acquiring and generating section 11a generates the passage presence/absence value that indicates that the work for the cells on the last observation time point (observation time point immediately before Aug. 1) is "non-passage work" (Steps S2'' and S6'').

In the case where the current observation day is Aug. 2, the number of cells or confluency is less than the predetermined lower threshold. The number of cells or confluency at the last observation time point (Aug. 1) stored in the archive section 13 is at least the predetermined upper threshold. In this case, the passage presence/absence determining section 11a1' determines that the work for the cells on the last observation time point (Aug. 1) is passage work. The passage presence/absence information acquiring and generating section 11a generates the passage presence/absence value that indicates that the work for the cells on the last observation time point (Aug. 1) is "passage work" (Steps S2'' to S5").

Although not applied to the example of FIG. 12, when the number of cells or confluency on the current observation day is less than the predetermined lower threshold and the number of cells or confluency at the last observation time point is less than the predetermined upper threshold, the passage presence/absence determining section 11a1' determines that the work for the cells at the last observation time point is non-passage work. The passage presence/absence information acquiring and generating section 11a generates a passage presence/absence value that indicates that the work for the cells on the last observation time point is "non-passage work" (Steps S2" to S4" and S6''.

Next, in a manner substantially identical to that of the cell observation information processing system 10 according to the first embodiment mode, the passage number on the current observation day is generated based on the previous passage presence/absence information by the passage number acquiring and generating section 11b (Steps S7' to S9').

Next, the storing section 12 assigns the passage presence/absence value and the passage number on the current observation day generated by the passage number acquiring and generating section 11b as search tags to the observation information, and stores the information in the archive section 13 (Step S10').

According to the cell observation information processing system 10 according to the third embodiment mode, the passage presence/absence information acquiring and generating section 11a includes the passage presence/absence determining section 11a1, that determines whether the work for the cells at the (n-1) -th observation time point is passage work, based on at least the passage presence/absence determining information at the n-th observation time point, and generates the determination result on whether the work is the passage work by the passage presence/absence determining section11a1', as the previous passage presence/absence information. Consequently, even if the passage presence/absence information is not recorded as the search tag in the archive section 13, the passage presence/absence value at the (n-1)-th observation time point can be acquired or generated by the passage presence/absence information acquiring section 11a, and the passage number at the n-th observation time point can be acquired or generated by the passage number acquiring and generating section 11b, based on the acquired or generated pas sage presence/absence value at the (n-1)-th observation time point. As a result, the user can acquire the correct value as the passage number that is one of the search tags to be assigned to the observation information on the current observation day. The need of input of the passage number by the user is then negated, which can eliminate input errors of the passage number by the user.

In the cell observation information processing system 10 according to the third embodiment mode, it is configured so that when the number of cells or confluency at the (n-1)-th observation time point is at least the predetermined upper threshold and the number of cells or confluency at the n-th observation time point is lower than the lower threshold, the passage presence/absence determining section 11a1' determines that the work for the cells at the (n-1) -th observation time point is the passage work, thereby allowing the passage number acquiring and generating section 11b to acquire or generate more accurately the passage number at the n-th observation time point by the passage number acquiring and generating section 11b.

Based on the determination result of whether the work for the cells at the (n-1) -th observation time point is passage work through the passage presence/absence determining section 11a1' of the passage presence/absence information acquiring and generating section 11a as shown in Steps S2'' to S6'' of FIG. 14 in the observation information processing system 10 in the third embodiment mode, the configuration for acquiring or generating the passage presence/absence value is applicable as a configuration for acquiring or generating the passage presence/absence value on the (n-1)-th observation time point as shown in Step S2 of FIG. 6 and Step S2' of FIG. 10 in the observation information processing systems 10 in the first and second embodiment modes, for example.

In the example of FIG. 11, the cell observation information processing system 10 includes the passage presence/absence information acquiring and generating section 11a, and the passage number acquiring and generating section 11b. The configuration is not limited to this example. Alternatively, for example, the configuration may include the storing section 12.

### Fourth embodiment mode

FIGs. 15A and 15B are explanatory diagrams that conceptually show examples of a method of selecting data to be subjected to acquisition or generation of the passage presence/absence value at the last time by the passage presence/absence information acquiring section, acquisition or generation of the passage number by the passage number acquiring and generating section, in a cell observation information processing system according to a fourth embodiment mode of the present invention. FIG. 15A is a data configuration diagram that shows an example thereof. FIG. 15B is a data configuration diagram that shows another example.

The cell observation information processing system 10 of the fourth embodiment mode includes any of the cell observation information processing systems 10 in the first to third embodiment modes, and the storing section 12.

The storing section 12 is configured so as to assign the first to (n-1)-th passage presence/absence values acquired or generated by the passage presence/absence information acquiring and generating section 11a, as search tags, to the respective first to (n-1)-th pieces of observation information acquired by the observation information acquiring section 20, and to further assign the first to (n-1)-th pieces of observation information respective pieces of cell identifying information for identifying the cells indicated by the pieces of the observation information as the search tag and store the information in the archive section 13.

For example, as shown in FIG. 15A, the passage presence/absence information acquiring and generating section 11a acquires the previous passage presence/absence information on the same cells from the archive section 13, based on the tags of the cell identifying information.

The passage number acquiring and generating section 11b acquires or generates the n-th passage number, based on the previous passage presence/absence information on the same cells acquired or generated by the passage presence/absence information acquiring and generating section 11a.

In the example of FIG. 15A, the passage presence/absence information acquiring and generating section 11a uses the cell name "CHO1" as a search key, to search through the search-tagged observation information stored in the archive section 13. The passage presence/absence value of the retrieved search-tagged observation information at the last observation time point (here, Aug. 1) is acquired. The passage number acquiring and generating section 11b generates the passage number on the current observation day (here, Aug. 2), based on the passage presence/absence value acquired by the passage presence/absence information acquiring and generating section 11a.

As another example of the cell observation information processing system 10 according to the fourth embodiment mode, the storing section 12 may be configured so as to assign the first to (n-1)-th pieces of observation information pieces of user identifying information for identifying users having observed the respective pieces of the observation information, as search tags, and to store the information in the archive section 13.

In this case, for example, as shown in FIG. 15B, the passage presence/absence information acquiring and generating section 11a is configured to acquire the previous passage presence/absence information on the same cells and the same user from the archive section 13, based on the tags of the cell identifying information and the user identifying information.

The passage number acquiring and generating section 11b is configured to acquire or generate the n-th passage number, based on the previous passage presence/absence information on the same cells and the same user acquired or generated by the passage presence/absence information acquiring and generating section 11a.

In the example of FIG. 15B, the passage presence/absence information acquiring and generating section 11a uses a cell name "CHO1" and a user ID "User 1" as the search key and searches for the search-tagged observation information stored in the archive section 13. The passage presence/absence value of the retrieved search-tagged observation information at the last observation time point (here, Aug. 1) is acquired. The passage number acquiring and generating section 11b generates the passage number on the current observation day (here, Aug. 2), based on the passage presence/absence information acquired by the passage presence/absence information acquiring and generating section 11a.

Another configuration is substantially identical to the configuration of any of the cell observation information processing systems 10 according to the first to third embodiment modes.

According to the cell observation information processing system 10 of the example of FIG. 15A in the fourth embodiment mode of the present invention, the storing section 12 further assigns first to (n-1)-th pieces of observation information the respective pieces of cell identifying information for identifying the cells indicated by the pieces of observation information as search tags and stores the information in the archive section 13, the passage presence/absence information acquiring and generating section 11a acquires the previous passage presence/absence information on the same cells from the archive section 13 based on the tags of the cell identifying information, and the passage number acquiring and generating section 11b acquires or generates the n-th passage number based on the previous passage presence/absence information on the same cells acquired or generated by the passage presence/absence information acquiring and generating section 11a. Consequently, the n-th passage number of the cell specimen belonging to the cell kind and the cell specimen assigned the cell name can be correctly and efficiently acquired or generated.

According to the cell observation information processing system 10 of the example in FIG. 15B, the storing section 12 further assigns first to (n-1)-th pieces of observation information the respective pieces of user identifying information for identifying users having observed the pieces of observation information as search tags and stores the information in the archive section 13, the passage presence/absence information acquiring and generating section 11a acquires or generates the previous passage presence/absence information associated with the same cells and the same user from the archive section 13 based on the tags of the cell identifying information and the tags of the user identifying information, and the passage number acquiring and generating section 11b acquires or generates the n-th passage number based on the previous passage presence/absence information associated with the same cells and the same user acquired or generated by the passage presence/absence information acquiring and generating section 11a. Consequently, the n-th passage number of the cell specimen under management by the very user concerned can be correctly and efficiently acquired or generated even if different users assigns the same name to the different cell specimens.

### Fifth embodiment mode

FIG. 16 is an explanatory diagram that shows the configuration of a cell observation information processing system according to a fifth embodiment mode of the present invention.

The cell observation information processing system 10 according to the fifth embodiment mode includes a passage presence/absence information acquiring and generating section 11a, a passage number acquiring and generating section 11b, and a passage presence/absence information output section 19.

The configuration of the passage presence/absence information acquiring and generating section 11a is substantially identical to that of the passage presence/absence information acquiring and generating section 11a of the first embodiment mode.

The passage presence/absence information output section 19 outputs the previous passage presence/absence information acquired or generated by the passage presence/absence information acquiring and generating section 11a, and further, history information on the passage work or non-passage work indicated by the previous passage presence/absence information (information that contains at least one of previous passage work date, and passage number), to the presenting section 18.

The presenting section 18 is configured to present, to a user, the previous passage presence/absence information output by the passage presence/absence information output section 11a, and accepts an input of a passage number from the user through a passage number input section 21.

The passage number acquiring and generating section 11b acquires the passage number input at the presenting section 18 through the passage input section 21 by the user having referred to the previous passage presence/absence information presented by the presenting section 18, as the n-th passage number (on the current observation day).

In addition, the presenting section 18 includes the tag acquiring screen 18d shown in FIGs. 3A and 3B, and a tag acquiring section analogous to that of the first embodiment mode.

A flow of processes from acquisition of the observation information, to acquisition of the previous passage presence/absence information, output of the previous passage presence/absence information to the presenting section 18, acquisition of the passage number on the
current observation day based on the passage number on the current observation day input by the user through the presenting section 18, and storage, in the archive section 13, of observation information assigned the search tag including the passage number on the current observation day, using the cell observation information processing system 10 according to the fifth embodiment mode with reference to FIG. 17.

It is herein assumed that the current observation day is Aug. 2, and the previous observation time point is Aug. 1 which is the last observation time point.

Steps up to the input and observation image acquisition order by the user from the image-capture order screen, acquisition of the observation information by the observation information acquiring section 20, and acquisition of the previous passage presence/absence information by the passage presence/absence information acquiring and generating section 11a are substantially identical to those of the cell observation information processing system 10 according to the first embodiment mode.

Next, the passage presence/absence information output section 19 outputs the previous passage presence/absence information acquired by the passage presence/absence information acquiring and generating section 11a, and further, the history information on the passage work or non-passage work indicated by the previous passage presence/absence information to a tag input screen of the presenting section 18.

In the example of FIG. 17, the previous passage presence/absence value "passage work", the previous passage work date "Aug. 1", and the passage number on Aug. 1 "4" are displayed on the tag input screen.

The user inputs the passage number on the current observation day "5" into the passage number input item provided in the presenting section, through the passage number input section 21, while referring to these pieces of information displayed on the tag input screen of the presenting section 18.

In the example of FIG. 17, the passage presence/absence value at the last observation time point "passage work", and the passage number at the last observation time point "4" can be referred to through the presenting section 18. Consequently, the user can recognize that the passage number on the current observation day that is to be input is a value "5" obtained by adding one to the passage number "4" on the last observation time point. When "non-passage work" is displayed as the passage presence/absence value on the last observation time point on the presenting section 18, the user can recognize that the passage number on the current observation day that is to be input is the same value as the value of the passage number on the last observation time point.

After input of the passage number on the current observation day by the user is completed, the passage number acquiring and generating section 11b acquires the passage number input by the user as the passage number on the current observation day.

Next, the storing section 12 assigns the observation information the cell name, cell level information, date on the current observation day and the like, which have been input by the user on the tag input screen and acquired by the tag acquiring section, and the passage number on the current observation day acquired by the passage number acquiring and generating section 11b, as search tags, and stores these items in the archive section 13.

According to the cell observation information processing system 10 of the fifth embodiment mode, the system further includes a passage presence/absence information output section 19 that outputs, to the presenting section 18, the previous passage presence/absence information acquired or generated by the passage presence/absence information acquiring and generating section 11a, the presenting section 18 is configured to present, to the user, the previous passage presence/absence information output by the passage presence/absence information output section 19, and to accept an input of the passage number from the user, and the passage number acquiring and generating section 11b is configured to acquire, as the n-th passage number, the passage number input through the presenting section 18 from the user referring to the previous passage presence/absence information presented by the presenting section 18. Consequently, the passage presence/absence information in the information on the maintenance work conducted to the cells at the last observation time point can be confirmed by the user, which can prevent the user from misunderstanding the details of the maintenance work conducted to the cells and from erroneously inputting the passage number.

Furthermore, according to the cell observation information processing system 10 of the fifth embodiment mode, the passage presence/absence information output section 19 further outputs, to the presenting section 18, history information on passage/non-passage work indicated by the previous passage presence/absence information, the presenting section 18 further presents, to the user, the history information output by the passage presence/absence information output section 19, and the passage number acquiring and generating section 11b acquires, as the n-th passage number, the passage number input through the presenting section 18 from the user referring to the previous passage presence/absence information presented by the presenting section 18 and to the history information. Consequently, the user is allowed to confirm not only the passage presence/absence information but also history information that contains the day identified as the day when the passage work was conducted according to the previous passage presence/absence information, and the passage number on the day identified as the day when the passage work was conducted, as information on the last maintenance work conducted for the cells. This configuration can thus prevent the user from misunderstanding the details of the maintenance work conducted to the cells, from misunderstanding passage number counting, and from erroneously inputting the passage number.

Preferably, the cell observation information processing system 10 according to the fifth embodiment mode automatically generates the passage number based on the previous passage presence/absence information besides the passage number input by the user, and can check the passage number input by the user based on comparison with the automatically generated passage number.

FIG. 18 is an explanatory diagram that shows the configuration of a cell observation information processing system according to a modified example of FIG. 16.

The cell observation information processing system 10 in FIG. 18 includes a passage presence/absence information acquiring and generating section 11a, a passage number acquiring and generating section 11b, a passage presence/absence information output section 19, and a notification section 24.

The passage number acquiring and generating section 11b has not only the configuration of the passage number acquiring and generating section 11b in the example of FIG. 16 but also a configuration that automatically generates the n-th passage number for checking input based on the previous passage presence/absence information acquired by the passage presence/absence information acquiring and generating section 11a besides the passage number input by the user.

The notification section 24 is configured to compare the passage number input by the user with the n-th passage number for checking input automatically generated by the passage number acquiring and generating section 11b and when the values of compared numbers are different from each other notify the user of warning information on input of the passage number. The notification section 24 may have any configuration only if this section can issue notification to the user; the notification may be any of output of a warning message onto the presenting section 18, generation of a warning sound and the like.

Another configuration is substantially identical to the configuration of the cell observation information processing system 10 of the example in FIG. 16.

A flow of processes from acquisition of the observation information, to acquisition of the previous passage presence/absence information, output of the previous passage presence/absence information to the presenting section 18, acquisition of the passage number on the current observation day based on the passage number on the current observation day input by the user through the presenting section 18, automatic generation of the passage number on the current observation day based on the previous passage presence/absence information, notification on warning information based on comparison between the passage number at the current observation day input by the user and the automatically generated passage number, and storage, in the archive section 13, of observation information assigned the search tag including the passage number on the current observation day, using the cell observation information processing system 10 of FIG. 18 is described with reference to FIG. 19.

Steps up to the input and observation image acquisition order by the user from the image-capture order screen, acquisition of observation information by the observation information acquiring section 20, acquisition of previous passage presence/absence information by the passage presence/absence information acquiring and generating section 11a, output of the previous passage presence/absence information by the passage presence/absence information output section 19, output of history information on the passage work or non-passage work indicated by the previous passage presence/absence information onto the tag input screen of the presenting section 18, and user's input of the passage number on the current observation day are substantially identical to those of the cell observation information processing system 10 of the example in FIG. 16.

After completion of user's input of the passage number on the current observation day, the passage number acquiring and generating section 11b acquires the passage number input by the user as the passage number on the current observation day, and automatically generates the n-th passage number for checking input based on the previous passage presence/absence information acquired by the passage presence/absence information acquiring and generating section 11a besides the passage number input by the user.

Next, the notification section 24 compares the passage number input by the user with the n-th passage number for checking input automatically generated by the passage number acquiring and generating section 11b, and when the values of compared numbers are different from each other, notifies the user of warning information on input of the passage number.

The flow of processing thereafter is substantially identical to that of the cell observation information processing system 10 of the example in FIG. 16.

The cell observation information processing system 10 of the example in FIG. 18 is configured so as to notify the user of warning information when the passage number input by the user is different from the n-th passage number for checking input automatically generated by the passage number acquiring and generating section. Consequently, when a doubt of an error of the passage number input by the user occurs, the input error of passage number by the user can be further prevented by causing the user to confirm the passage number.

In the case of the example in FIG. 18, a confirmation button for confirming completion of input of the passage number through the passage number input section 21 on the tag input screen may be provided. Thus, when the warning information is notified by the notification section 24, the user can input the passage number again.

### Sixth embodiment mode

FIG. 20 is an explanatory diagram that schematically shows a flow of processes from acquisition of the observation information, to acquisition of the previous passage presence/absence information, acquisition of the passage number on the current observation day based on the previous passage presence/absence information and passage number, and storage, in the archive section, of observation information assigned the search tag including the passage number on the current observation day, using the cell observation information processing system according to a sixth embodiment mode of the present invention.

The basic configuration of the cell observation information processing system 10 according to the sixth embodiment mode is substantially identical to the configuration shown in FIG. 1.

The passage presence/absence information acquiring and generating section 11a acquires the previous passage presence/absence information that contains the passage presence/absence value at the last ((n-1)-th) observation time point in the archive section 13.

The passage number acquiring and generating section 11b generates the n-th passage number on the current observation day basedonthe (n-1)-th passage presence/absence value and the (n-1)-th passage number at the (n-1)-th observation time point.

To be specific, the passage number acquiring and generating section 11b generates, as the passage number on the current observation day, a value acquired by adding one to the (n-1) -th passage number, when the (n-1) -th passage presence/absence value indicates the "passage work". The passage number acquiring and generating section 11b generates, as the passage number on the current observation day, a value identical to the (n-1)-th passage number, when the (n-1)-th passage presence/absence value indicates the "non-passage work".

For example, in the example of FIG. 20, in the case where the current observation day is Aug. 2, the passage presence/absence information acquiring and generating section 11a acquires the previous passage presence/absence information (on or before Aug. 1) that contains the passage presence/absence value at the last observation time point on Aug. 1 and is stored in the archive section 13. The passage presence/absence value on Aug. 1 indicates "passage work". The passage number in the record of the search-tagged observation information on Aug. 1 indicates "4" . The passage number acquiring and generating section 11b thus generates, as the passage number on Aug. 2 that is the current observation day, the "5" acquired by adding one to the passage number "4" on Aug. 1.

For example, in the example of FIG. 20, in the case where the current observation day is Aug. 3, the passage presence/absence information acquiring and generating section 11a acquires the previous passage presence/absence information (on or before Aug. 2) that contains the passage presence/absence value on Aug. 2 at the last observation time point and is stored in the archive section 13. The passage presence/absence value on Aug. 2 indicates "non-passage work". The passage number in the record of the search-tagged observation information on Aug. 2 indicates "5". The passage number acquiring and generating section 11b thus generates the passage number "5" on Aug. 2 as the passage number on Aug. 3 that is the current observation day.

Next, the storing section 12 assigns the passage number generated by the passage number acquiring and generating section 11b as the search tag to the observation information, and stores the information in the archive section 13.

According to the cell image information processing system 10 of the sixth embodiment mode, the previous passage presence/absence information contains the (n-1)-th passage presence/absence value at the (n-1) -th observation time point, the previous passage number contains the (n-1)-th passage number at the (n-1)-th observation time point, and the passage number acquiring and generating section 11b generates the n-th passage number based on the (n-1) -th passage presence/absence value and the (n-1)-th passage number. Consequently, the passage number on the current observation day can be automatically calculated.

### Seventh embodiment mode

FIG. 21 is an explanatory diagram that schematically shows a flow of processes from acquisition of the observation information, to acquisition of the previous passage presence/absence information, acquisition of the passage number on the current observation day based on the previous passage presence/absence information and passage number, and storage, in the archive section, of observation information assigned the search tag including the passage number on the current observation day, using the cell observation information processing system according to a seventh embodiment mode of the present invention.

The basic configuration of the cell observation information processing system 10 according to the seventh embodiment mode is substantially identical to the configuration shown in FIG. 1.

The passage presence/absence information acquiring and generating section 11a acquires the previous passage presence/absence information that contains a passage presence/absence value at the last ((n-1)-th) observation time point and a passage presence/absence value at a second last or earlier ((n-2)-th or earlier) observation time point in the archive section 13.

The passage number acquiring and generating section 11b identifies an observation time point at which the most recent passage work for the cells was conducted among the previous observation time points. The passage number acquiring and generating section 11b generates the n-th passage number on the current observation day based on the passage number at the observation time point identified by the most recent passage work. For example, the passage number acquiring and generating section 11b generates, as the passage number on the current observation day, a value acquired by adding one to the passage number at the observation time point identified by the most recent passage work, as described above.

Here, in the example of FIG. 21, a case where the current observation day is Aug. 8 is described in detail.

The passage presence/absence information acquiring and generating section 11a acquires the passage presence/absence values at the observation time points on and before Aug. 7 (here, Aug. 7 to Aug. 1).

The passage number acquiring and generating section 11b identifies Aug. 6 as the most recent observation time point at which the passage presence/absence value is "passage work" among observation time points on and before Aug. 7. Next, the passage number acquiring and generating section 11 thus generates, as the passage number on the current observation day (Aug. 8), the "6" acquired by adding one to the same passage number "5" on Aug. 6.

In the example of FIG. 21, a case where the current observation day is Aug. 6 is described in detail.

The passage presence/absence information acquiring and generating section 11a acquires the passage presence/absence information on the observation time point on and before Aug. 5 (here, Aug. 5 to Aug. 1).

The passage number acquiring and generating section 11b identifies the Aug. 1 as the most recent observation time point at which the passage presence/absence value is "passage work" among observation time points on and before Aug. 5. Next, the passage number acquiring and generating section 11 thus generates, as the passage number on the current observation day (Aug. 6), the "5" acquired by adding one to the same passage number "4" on Aug. 1.

Next, the storing section 12 assigns the passage number generated by the passage number acquiring and generating section 11b as the search tag to the observation information, and stores the information in the archive section 13.

The cell observation information processing system 10 according to the seventh embodiment mode can automatically calculates the passage number at the observation time point without using the passage presence/absence value and the passage number at the last (n-1) observation time point.

### Eighth embodiment mode

FIG. 22 is an explanatory diagram that shows the configuration of a cell observation information processing system according to an eighth embodiment mode of the present invention.

The cell observation information processing system 10 according to the eighth embodiment mode further includes a warning information output section 26, in addition to the basic configuration shown in FIG. 1.

The passage presence/absence information acquiring and generating section 11a acquires the previous passage presence/absence information that contains each of the k-th (k is at least one integer that is at least one and less than or equal to n-1) passage presence/absence values at the corresponding k-th observation time points in the archive section 13.

The passage number acquiring and generating section 11b identifies whether work for the cells at each of the k-th observation time points is the passage work, based on the corresponding k-th passage presence/absence values. The passage number acquiring and generating section 11b generates, as the passage number on the current observation day, a value acquired by adding one to the passage number at the most recent observation time point at which the passage presence/absence value indicates "passage work" among the k-th observation time points.

The warning information output section 26 is configured so as to output the warning information to the presenting section 18 based on the elapsed days identified by the passage number acquiring and generating section 11b from the most recent observation time point at which the passage presence/absence value indicates "passage work" to the n-th observation time point (current observation day) among the k-th observation time points and on the predetermined reference number of days.

To be specific, in the case where the elapsed days from the most recent observation time point at which the passage presence/absence value identified by the passage number acquiring and generating section 11b indicates "passage work" to the n-th observation time point (current observation day) is less than the predetermined reference number of days, the warning information output section 26 determines that no passage work was conducted from the observation time point identified that the most recent passage work was conducted to the current observation day. In this case, the warning information output section 26 outputs no warning information to the presenting section 18.

On the contrary, in the case where the elapsed days from the most recent observation time point at which the passage presence/absence value identified by the passage number acquiring and generating section 11b indicates "passage work" to the n-th observation time point (current observation day) is at least the predetermined reference number of days, the warning information output section 26 determines that, even though another passage work was conducted during a period from the observation time point identified that the most recent passage work was conducted to the current observation day, a passage presence/absence value on the observation day on which the another passage work was conducted has not been stored in the archive section 13, or the passage presence/absence value stored in the archive section 13 has an error. In this case, the warning information output section 26 outputs the warning information to the presenting section 18.

The predetermined reference number of days may be, for example, the average value of previous cell passage interval days automatically calculated by the system using a calculation means, not shown. Alternatively, this number may be the number of days designated by the user through a designation means, not shown. The predetermined reference number of days may be a number defined according to each cell kind.

The presenting section 18 presents, to the user, the warning information output by the warning information output section 26.

A flow of processes from acquisition of the observation information, to acquisition of the previous passage presence/absence information, identification of the observation time point at which the most recent passage work was conducted based on the previous passage presence/absence information, acquisition of the passage number on the current observation day based on the passage number at the identified observation time point when the most recent passage work was conducted, output of warning information based on the elapsed days from the identified most recent observation time point when the most recent passage work was conducted to the current observation day and on the predetermined reference number of days, and storage, in the archive section, of observation information assigned the search tag including the passage number on the current observation day, using the thus configured cell observation information processing system 10 of FIG. 22 is described with reference to FIG. 23.

It is herein assumed that the current observation day is Aug. 26, and the predetermined reference number of days is the average number of passage days of five. For convenience' sake, only data with the passage work having been conducted is extracted as the record of the search-tagged observation information and shown in the lower field of FIG. 23.

Steps up to the input and observation image acquisition order by the user from the image-capture order screen, and acquisition of the observation information by the observation information acquiring section 20 are substantially identical to those of the cell observation information processing system 10 according to each embodiment mode.

The passage presence/absence information acquiring and generating section 11a acquires the passage presence/absence information on the observation time point on and before Aug. 25 (here, Aug. 25 to Aug. 1).

The passage number acquiring and generating section 11b identifies whether work for the cells at each of observation time points from Aug. 25 to Aug. 1 is the passage work. In the example of FIG. 23, as described above, only data at the observation time point when the passage work was conducted is shown. The passage number acquiring and generating section 11b generates, as the passage number on the current observation day Aug. 26 that is the current observation day, a value "8" acquired by adding one to the passage number "7" at the most recent observation time point of Aug. 16 at which the passage presence/absence value indicates "passage work".

Here, the warning information output section 26 compares the elapseddays (10 days) fromAug. 16 which is the most recent observation time point at which the passage presence/absence value indicates "passage work" according to the passage number acquiring and generating section 11b to the n-th observation time point (current observation day) Aug. 26 with the predetermined reference number of days (here, the average passage interval days for the cells is "5 days"). In the example of FIG. 23, the elapsed days from the most recent observation time point at which the passage presence/absence value indicates "passage work" to the current observation day exceeds the predetermined reference number of days. Consequently, the warning information output section 26 outputs the warning information to the presenting section 18.

The storing section 12 assigns the passage number generated by the passage number acquiring and generating section 11b as the search tag to the observation information, and stores the information in the archive section 13.

According to the cell observation information processing system 10 of the eighth embodiment mode of the present invention, the warning information output section 26 is configured to output the warning information to presenting section 18 when the elapsed days from the observation time point identified by the passage number acquiring and generating section 11b that the most recent passage work was conducted to the n-th observation time point is at least the predetermined reference number of days. Consequently, notification of presence of an observation day on which a passage work was conducted in fact but not stored in the database file can be output to the user to draw the attention of whether the value of the passage number on the current observation day is correct, and can draw the user's attention for preventing failure of storage into the database file.

### Ninth embodiment mode

FIG. 24 is an explanatory diagram that shows the main configuration of a cell observation information processing system according to a ninth embodiment mode of the present invention. FIG. 25 is a flowchart that shows an example of processing procedures from extraction of the first-to-(n-th)-tagged observation information, to identification of the search-tagged observation information at the oldest observation time point with no passage number being set, generation of passage numbers in bulk, and storage, in the archive section, of the observation information assigned tags of the passage numbers, using the cell observation information processing system of FIG. 24. FIGs. 26A to 26C are explanatory diagrams that show an example of search-tagged observation information record where passage numbers are generated in bulk using the cell observation information processing system of FIG. 24. FIG. 26A is a diagram that shows a search-tagged observation information record before the batch processing. FIG. 26B is a diagram that shows a state where the passage number is assigned to the search-tagged observation information record at the oldest observation time point with no passage number being indicated. FIG. 26C is a diagram that shows a state where the passage number is assigned to the search-tagged observation information record up to the latest observation time point.

The cell observation information processing system 10 according to the ninth embodiment mode is configured to generate and assign passage numbers in bulk posteriorly to the search-tagged observation information assigned the passage presence/absence information having already been stored in the archive section 13.

To be specific, the cell observation information processing system 10 according to the ninth embodiment mode includes not only the passage presence/absence information acquiring and generating section 11a and the passage number acquiring and generating section 11b but also an observation information list retrieving section 27 and a batch processing control section 28.

The observation information list retrieving section 27 is configured so as to retrieve pieces of search-tagged observation information at the first to n-th observation time points stored in the archive section 13.

The passage presence/absence information acquiring and generating section 11a identifies the search-tagged observation information at the m-th (m: 1 < m ≤ n) observation time point that is the oldest observation time point with no passage number being set, among the pieces of search-tagged observation information at the first to n-th observation time points retrieved from the observation information list retrieving section 27. Under control by the batch processing control section 28 described later, the passage presence/absence values at the (m-1)-th to (n-1)-th observation time points are acquired.

Under control by the batch processing control section 28, described later, the passage number acquiring and generating section 11b acquires or generates the passage numbers at the m-th to n-th observation time points, based on the passage presence/absence values and the passage numbers at the (m-1) -th to (n-1) -th observation time points.

The batch processing control section 28 is configured to control the operation of the passage presence/absence information acquiring and generating section 11a and the passage number acquiring and generating section 11b (and the storing section 13) for acquiring or generating the passage numbers at the m-th to n-th observation time points.

A processing procedures from retrieval of the first-to-(n-th)-tagged observation information, to identification of the search-tagged observation information at the oldest observation time point with no passage number being set, batch generation of the passage numbers, and storage, in the archive section, of the observation information assigned tags of the passage number, using the thus configured cell observation information processing system 10 of FIG. 24 is described with reference to FIGs. 25 and 26.

Here, as shown in FIG. 26A, the pieces of search-tagged observation information at the observation time points on Aug. 1 to Aug. 8 are stored in the archive section 13. Among the pieces of the information, the passage number is set in the search-tagged observation information at the observation time point on the Aug. 1, and the passage numbers are not set in the pieces of search-tagged observation information at the observation time points on Aug. 2 to Aug. 8.

As with the example in FIG. 26A, the data mode according to which the passage presence/absence information is assigned but the passage number is not assigned and storage is made into the archive section 13 may occur, for example in a case where another apparatus that includes the observation information acquiring section 20, the passage presence/absence information acquiring and generating section 11a and the storing section 12 is separately provided, and a search tag other than the passage number is assigned by the user from the apparatus to the observation information and stored in the archive section 13.

First, the observation information list retrieving section 27 retrieves pieces of search-tagged observation information at the first to n-th observation time points stored in the archive section 13 (Step S1"').

Next, the passage presence/absence information acquiring and generating section 11a identifies the search-tagged observation information at the m-th (m: 1 < m ≤ n) observation time point that is the oldest observation time point with no passage number being set, among the pieces of search-tagged observation information at the first to n-th observation time points retrieved by the observation information list retrieving section 27 (Step S2"'). In the example of FIG. 26A, the search-tagged observation information on Aug. 2 is identified as the search-tagged observation information at the m-th observation time point.

Here, the batch processing control section 28 sets the identified observation time point of Aug. 2 as a value indicating the ongoing processing time point in the batch processing ("j" in the example of FIG. 26A) (Step S3"').

Next, the passage presence/absence information acquiring and generating section 11a acquires the passage presence/absence value at the (j-1)-th observation time point before and nearest the j-th processing time point (Step 4"'). In the example of FIG. 26, the initial value of "j" at the processing time point is Aug. 2. In the search-tagged observation information on Aug. 1, which is the (j-1)-th observation time point before and nearest the j-th observation time point of August 2, the passage presence/absence value indicates "passage work".

Next, the passage number acquiring and generating section 11b acquires or generates the passage number at the j-th processing time point, based on the passage presence/absence value and the passage number at the (j-1)-th observation time point, which is before and nearst the j-th processing time point (Step 5"'). In the example of FIG. 26A, at the (j-1)-th observation time point on Aug. 1, the passage presence/absence value indicates "passage work", and the passage number is "4". In this case, as with each of the aforementioned embodiment modes, the passage number acquiring and generating section 11b generates a value "5" acquired by adding one to the passage number as the passage number at the j-th processing time point.

Next, the storing section 12 assigns the passage number generated by the passage number acquiring and generating section 11b, as the search tag, to the observation information, and stores the information in the archive section 13 (Step S6"'). The state of the search-tagged observation information record at this time is shown in FIG. 26B.

Next, the batch processing control section 28 checks whether the j-th processing time point reaches the latest n-th observation time point (Step S7"'). The batch processing control section 28 updates the next (j+1)-th observation time point to the j-th observation time point (Step S8"') and then repeats the processes in Steps S4"' to S7"' until the j-th processing time point reaches the latest n-th observation time point. A state where the process of generating the passage number in bulk is finished, and the passage number is assigned to the search-tagged observation information record up to the latest observation time point is shown in FIG. 26C.

According to the cell observation information processing system 10 of the ninth embodiment mode, in the case where the time point of as signing the tag of the passage number is configured to be different from the time point at which the observation information is acquired, is assigned the search tag and is stored in the archive section, the process for acquiring or generating the passage number as the search tag to be assigned to the observation information acquired at the time when the cell observation information is acquired can be omitted. Consequently, the time period during which the cells is left out of an incubator can be reduced, which can reduce damage to be appliedto the cells when the observation information is acquired. In the case of intervention of user's operation for acquiring or generating the passage numbers in bulk, the time point of user's operation is different from the time point for acquiring the observation information. The difference prevents the cells from being damaged. Consequently, the user can perform an operation where input errors of the passage number are checked in a sufficient time.

The configuration of the cell observation information processing system according to the ninth embodiment mode is only shown as a part of configuration of the cell observation information processing system of the present invention. It is a matter of course that a configuration combined with the cell observation information processing system of another embodiment mode may be adopted.

FIG. 27 is a flowchart that shows an example of processing procedures from extraction of the first-to-n-th-tagged observation information, to identification of the search-tagged observation information at the oldest observation time point with no passage number being set, batch generation of the passage numbers, and storage, in the archive section, of the observation information assigned tags of the passage number, using the cell observation information processing system according to a tenth embodiment mode of the present invention. FIGs. 28A to 28C are explanatory diagrams that show an example of search-tagged observation information record where the passage numbers are generated in bulk using the cell observation information processing system of FIG. 27. FIG. 28A is a diagram that shows a search-tagged observation information record before the batch processing. FIG. 28B is a diagram that shows a state where the passage number is assigned to the search-tagged observation information record at the oldest observation time point with no passage number being indicated. FIG. 28C is a diagram that shows a state where the passage number is added to the search-tagged observation information record up to the latest observation time point.

The cell observation information processing system 10 according to the tenth embodiment mode is configured to generate and assign passage numbers in bulk posteriorly to search-tagged observation information in which the passage presence/absence information is not adopted as a data item or not prepared as data and which has already been stored in the archive section 13.

The cell observation information processing system 10 according to the tenth embodiment mode is different from the cell observation information processing system 10 according to the ninth embodiment mode, in the method of acquiring and generating the passage presence/absence values at the (m-1)-th to the (n-1)-th observation time points by the passage presence/absence information acquiring and generating section 11a. That is, the passage presence/absence information acquiring and generating section 11a identifies the search-tagged observation information at the m-th (m: 1 < m ≤ n) observation time point that is the oldest observation time point with no passage number being set, among the pieces of search-tagged observation information at the first to n-th observation time points retrieved from the observation information list retrieving section 27. Under control by the batch processing control section 28, the passage presence/absence values at the (m-1)-th to (n-1)-th observation time points are generated based on the cell activity data, such as the number of cells or confluency, at the (m-1)-th to (n-1)-th observation time points.

The other configuration and operation are substantially identical to the configuration and operation of the cell observation information processing system 10 according to the ninth embodiment mode shown in FIG. 24.

The cell observation information processing system 10 of the tenth embodiment mode can generate and assign passage numbers in bulk posteriorly to search-tagged observation information in which the passage presence/absence information is not adopted as a data item or not prepared as data and which has already been stored in the archive section 13. Consequently, in addition to the advantageous effects of the cell observation information processing system 10 according to the ninth embodiment mode, an advantageous effect of omitting the process for acquiring or generating the passage presence/absence information as the search tag that is to be assigned to the observation information acquired at the time of acquiring the cell observation information. Consequently, the time period during which the cells are left out of an incubator can be reduced, which can reduce damage to be applied to the cells when the observation information is acquired.

As described above, according to some embodiment modes of the present invention, attainable are the cell observation information processing system, the cell observation information processing method, the cell observation information processing program, the archive section provided for the cell observation information processing system, and apparatuses provided for the cell observation information processing system that can prevent erroneous input of the passage number, which is one of tags to be assigned to observation information, such as on cells, by the user.

The embodiment modes of the cell observation information processing systems of the embodiment mode of the present invention have thus been described above. Alternatively, the cell observation information processing system may be con figured by a cell observation information processing program provided for causing a computer provided in the installation 1 to function as: a passage presence/absence information acquiring and generating means for acquiring or generating,asa previouspassage presence/absence value, at least one passage presence/absence value among first to (n-1) -th passage presence/absence values indicating whether work for cells was passage work at first to (n-1) -th observation time points, among first to n-th observation time points at which first to n-th pieces of observation information including items indicating a cell observation result including a cell image, and activity data indicating the cell activity, such as the number of cells, confluency, morphology and a viability, and an observation name are acquired in a time series manner through an observation information acquiring section; and a passage number acquiring and generating means for acquiring or generating the n-th passage number at the n-th observation time point, automatically or through an operation by a user, based on the previous passage presence/absence information acquired by the passage presence/absence information acquiring and generating means.

The cell observation information processing system according to the embodiment mode of the present invention may thus have the configuration where the cell observation information processing program is included in the computer provided in the installation 1. Alternatively, for example, a configuration of being recorded in a recording medium that is readable by a computer provided in the installation 1 may be adopted.

The cell observation information processing system according to the embodiment mode of the present invention is not limited to the configuration where the passage presence/absence information acquiring and generating section 11a and the passage number acquiring and generating section 11b are included together with the storing section 12, the archive section 13, the presenting section 18, the passage presence/absence selecting and ordering section 16 and the control section 25 in the single installation 1, as shown in FIG. 2, for example. For example, as shown in FIGs. 29 and 30 as modified examples, a configuration may be adopted where the passage presence/absence information acquiring and generating section and the passage number acquiring and generating section are separately provided in multiple apparatuses in a distributed manner.

FIG. 29 is an explanatory diagram that shows the configuration of a cell observation information processing system according to a modified example of the present invention.

In the cell observation information processing system 10 according to this modified example, for example, a passage presence/absence information acquiring and generating section 11a configured in a manner analogous to the configuration in the cell observation information processing system 10 of the second embodiment mode is provided together with the observation information acquiring section 20 and the storing section 12 in an observation processing apparatus 1a, an observation information list retrieving section 27, a passage presence/absence information acquiring and generating section 11a', a passage number acquiring and generating section 11b and a batch processing control section 28 which are configured in a manner analogous to the configuration in the cell observation information processing system 10 of the tenth embodiment mode are provided together with the storing section 12 in a passage number assigning apparatus 1b, and the archive section 13 is provided in the archive processing apparatus 1c.

The observation processing apparatus 1a is configured, for example, of a microscope apparatus provided with an image capture apparatus and a computer.

The passage number assigning apparatus 1b is configured, for example, by internally including a computer provided with database software, and further includes a presenting apparatus, not shown.

The archive processing apparatus 1c is configured, for example, of a storing apparatus that stores a database file.

The observation processing apparatus 1a and the archive processing apparatus 1c, and the passage number assigning apparatus 1b and the archive processing apparatus 1c are connected via a network to each other.

The configurations of the storing section 12, the archive section 13, and the observation information acquiring section 20 are substantially identical to the configurations of the first embodiment mode shown in FIG. 2, for example.

In the thus configured cell observation information processing system 10 in FIG. 29, the observation information acquiring section 20 included in the observation processing apparatus 1a acquires observation information in response to an acquisition order at one time point by the user, in the step of storing, in the archive section 13, the observation information on the cultured cells. Next, the passage presence/absence information acquiring and generating section 11a acquires the passage presence/absence value on the current observation day, based on the activity data (the number of cells, and confluency) calculated based on the data acquired by the observation information acquiring section 20, for example. Next, the storing section 12 provided for the observation processing apparatus 1a assigns, as a search tag, the passage presence/absence value acquired by the passage presence/absence information acquiring section 11a to the observation information, and stores the search-tagged observation information in the archive section 13 in the archive apparatus 1c via the network line.

The passage number assigning apparatus 1b generates the passage numbers in bulk and assigns the numbers to the search-tagged observation information record which has already been stored in the archive section 13 of the archive apparatus 1c via the observation processing apparatus 1a and in which multiple passage numbers are not set, according to procedures analogous to those described in the tenth embodiment mode.

According to the cell observation information processing system 10 in FIG. 29, the passage presence/absence information acquiring and generating section 11a that acquires or generates the passage presence/absence information to be assigned as the search tag to the observation information during acquisition of the observation information, and the passage presence/absence information acquiring and generating section 11a' for generating the passage numbers in bulk to the search-tagged observation information having been stored in the archive section 13 with no passage number being set and the passage number acquiring and generating section 11b, which constitute the cell observation information processing system 10, are provided separately in the multiple apparatuses 1a and 1b. Consequently, the individual apparatuses can be reduced in size and simplified. Furthermore, observation information acquisition, and passage number acquisition and generation are separately supported by the different apparatuses. Consequently, the process of acquiring or generating the passage number as the search tag to be assigned to the observation information acquired at the time of acquiring the cell observation information can be omitted. Consequently, the time period during which the cells is left out of an incubator can be reduced, which can reduce damage to be applied to the cells when the observation information is acquired. In the case of intervention of user's operation for acquiring or generating the passage numbers in bulk, the time point of user's operation is different from the time point for acquiring the observation information. The difference prevents the cells from being damaged. Consequently, the user can perform an operation where input errors of the passage number are checked in a sufficient time.

FIG. 30 is an explanatory diagram that shows the configuration of a cell observation information processing system according to another modified example of the present invention.

The cell observation information processing system 10 of this modified example causes multiple observation processing apparatuses 1a-1 to 1a-n (n = 2 in FIG. 30 for convenience' sake) to acquire respective pieces of observation information, assigns pieces of passage presence/absence information as search tags to the acquired pieces of observation information, and stores the information in the archive section 13 of the archive processing apparatus 1c. A configuration is adopted where the passage number assigning apparatus 1b generates the passage numbers in bulk and assigns the numbers to the observation data assigned the tags of passage presence/absence information stored in the archive section 13.

To be specific, in the cell observation information processing system 10 in FIG. 30, the multiple observation processing apparatuses 1a-1 to 1a-n each include the passage presence/absence information acquiring and generating section 11a configured in a manner analogous to the configuration in the cell observation information processing system 10 of the second embodiment mode, together with the observation information acquiring section 20 and the storing section 12.

Another configuration is substantially identical to the configuration of the cell observation information processing system 10 in FIG. 29.

According to the thus configured cell observation information processing system 10 in FIG. 30, in the step of storing the observation information on the cultured cells in the archive section, in each of the observation processing apparatuses 1a-1 to 1a-n, the observation information acquiring section 20 acquires the observation information in response to the acquisition order by the user at one time point, the passage presence/absence information acquiring and generating section 11a acquires, for example, the passage presence/absence value on the current observation day based on the activity data (the number of cells, confluency) calculated based on the data acquired by the observation information acquiring section 20, and the storing section 12 provided for the corresponding one of the observation processing apparatuses 1a-1 to 1a-n assigns the passage presence/absence information acquired by the passage presence/absence information acquiring section 11a, as the search tag, to the observation information, and stores the search-tagged observation information in the archive section 13 of the archive apparatus 1c via the network line.

The passage number assigning apparatus 1b generates the passage numbers in bulk and assigns the numbers to the search-tagged observation information record which has already been stored in the archive section 13 of the archive apparatus 1c via the observation processing apparatuses 1a-1 to 1a-n and in which multiple passage numbers are not set, according to procedures analogous to those described in the tenth embodiment mode.

The cell observation information processing system 10 in FIG. 30 is configured so that the multiple observation processing apparatuses 1a-1 to 1a-n (n = 2 in FIG. 30, for convenience' sake) each acquire observation information, assigns the passage presence/absence information as the search tag to the corresponding observation information acquired, stores the information in the archive section 13 of the archive processing apparatus 1c, and the passage number assigning apparatus 1b generates the passage numbers in bulk and assigns the numbers to the observation data assigned the passage presence/absence information stored in the archive section 13. Consequently, the user can acquire the observation information through the observation processing apparatus provided at a desired place without being limited to the arrangement of a single observation processing apparatus 1a-x (x is an integer from 1 to n), and record, in the archive section 13, the search-tagged observation information assigned the passage presence/absence information acquired through the corresponding observation processing apparatus as the search tag in a centralized manner, thereby further improving the user-friendliness.

The multiple observation processing apparatuses 1a-1 to 1a-n have the configuration that generates the passage numbers in bulk and assigns the numbers to the tagged-observation data on passage presence/absence information stored in the archive section 13. Consequently, each of the observation processing apparatuses is reduced in size and weight, and dedicated observation processing apparatuses are configured for the respective users in a portable manner, which allows each user to acquire cell observation information at any place and can improve user-friendliness.

The other configuration and functions and effects are substantially identical to those of the modified example in FIG. 29.

FIG. 31 is an explanatory diagram that shows the configuration of a cell observation information processing system according to still another modified example of the present invention.

For example, the cell observation information processing system 10 according to this modified example has a configuration that includes the configuration of cell observation information processing system 10 in FIG. 2, and further includes the search tag output section 29 and the presenting section 18.

The search tag output section 29 outputs, to the presenting section 18, search tags that are passage presence/absence information acquired or generated by the passage presence/absence information acquiring and generating section 11a and the passage numbers acquired or generated by the passage number acquiring and generating section 11b, and further for example, search tags that are the cell level information at the last observation time point stored in the archive section 13, and the cell name.

The presenting section 18 presents the search tag output by the search tag output section 29, on the search tag input screen 18d as shown in FIGs. 3A and 3B.

The other configuration and function and effect are, for example, substantially identical to the configuration and function and effect in the first embodiment mode shown in FIG. 2.

Here, the example applied to the cell observation information processing system in FIG. 2 has been described. The configuration where the cell observation information processing system 10 according to the present invention includes the search tag output section 29 and the presenting section 18 is applicable to the cell observation information processing systems of the other embodiment modes.

In the aforementioned embodiment modes, the presenting section is the screen interface. Alternatively, in another modified example, the presenting section may be any of other user interfaces, such as an audio interface, a line-of-sight interface, and a gesture interface. For example, in the case where the presenting section is the audio interface, the presenting section may use an audio analysis technique as disclosed in JP KOKAI No. 2012-252181 to accept a selection order on whether the work is passage work, or a confirmation order where the acceptance of the selection order is confirmed, from the user by means of audio. Furthermore, the presenting section may output the passage number acquired or generated by the passage number acquiring and generating section, by means of audio.

For example, in the case where the presenting section is the line-of-sight interface, a configuration may be adopted where association information that associates the direction in which the user's line of sight moves with a character for the direction is stored in the presenting section, the presenting section causes a line-of-sight detecting section, such as a line-of-sight detecting sensor, to detect the direction of user's line of sight, and the character associated with the direction may be acquired as the order by the user, based on the detected user's line of sight and the association information.

Furthermore, for example, in the case where the presenting section is the gesture interface, a configuration may be adopted where association information that associates the direction in which user's body parts, such as a hand, foot or trunk, moves with a character for the direction is stored in the presenting section, the presenting section causes a gesture detecting section, such as a gesture detecting sensor, to detect the direction of movement of user's body part, and the character associated with the direction may be acquired as the order by the user, based on the detected user's movement of body part and the association information.

This modified example has exemplified the audio interface and the line-of-sight interface or the like as other user interfaces. However, the configuration is not limited thereto. For example, a configuration may be adopted where an instruction is acquired through an electroencephalography interface.

The embodiment modes of the present invention and their modified examples have thus been described. However, the present invention is not limited to the configurations as described in the embodiment modes and the modified examples. In an implementation stage, the configuration elements may be modified and embodied in the range without changing the gist of the present invention. Appropriate combination of configuration elements described in the embodiment modes and their modified examples can derive various aspects of invention. For example, some configuration elements may be removed from the entire configuration elements described in each of the embodiment modes or their modified examples. Alternatively, configuration elements described in different embodiment modes or their modified examples may be appropriately combined. Thus, various modifications or applications may be made in a range without changing the gist of the invention.

### INDUSTRIAL APPLICABILITY

The cell observation information processing system, cell observation information processing method, cell observation information processing program, archive section provided for the cell observation information processing system, and apparatuses provided for the cell observation information processing system according to the present invention are useful for the field that requires condition management of cells and the like to be used for research of a living object through use of observation information on the cells acquired by an observation information acquiring apparatus such as a microscope, and for the field that requires condition management of a living object varying in a time series manner.

### DESCRIPTION OF THE REFERENCE SYMBOLS

1 cell observation information processing installation
1a, 1a-1, 1a-2 observation processing apparatus
1b passage number assigning apparatus
1c archive processing apparatus
10 cell observation information processing system
11a, 11a' passage presence/absence information acquiring and generating section
11a1, 11a1' passage presence/absence determining section
11b passage number acquiring and generating section
12 storing section
13 archive section
16 passage presence/absence selecting and ordering section
18 presenting section (monitor)
19 passage presence/absence information output section
20 observation information acquiring section
21 passage number input section
24 notification section
25 control section
26 warning information output section
27 observation information list retrieving section
28 batch processing control section
29 search tag output section

## Claims

1. A cello observation information processing system, comprising:
a passage presence/absence information acquiring and generating section that acquires or generates previous passage presence/absence information including at least one passage presence/absence value among first to (n-1) -th passage presence/absence values indicating whether work for cells was passage work at first to (n-1)-th observation time points, among first to n-th observation time points, at which first to n-th pieces of observation information each indicating a cell observation result including at least one of a cell image, and activity data indicating an activity of cells by at least one of the number of cells, confluency, morphology, and viability are acquired in a time series manner through an observation information acquiring section, where n is an integer equal to or greater than two and the n-th observation time point represents a current observation time point; and
a passage number acquiring and generating section that acquires or generates at least an n-th passage number at the n-th observation time point automatically or through an operation by a user, based on the previous passage presence/absence information acquired or generated by the passage presence/absence information acquiring and generating section.

2. The cell observation information processing system according to claim 1,
wherein the passage presence/absence information acquiring and generating section further generates an n-th passage presence/absence value indicating whether work for the cells at the n-th observation time point is the passage work, based on a selection order accepted from the user through a presenting section, and
the presenting section accepts, from the user, the selection order on whether the work for the cells at the n-th observation time point is the passage work.

3. The cell observation information processing system according to claim 2, further comprising
a storing section that assigns the n-th passage presence/absence value generated by the passage presence/absence information acquiring and generating section, as a search tag, to the n-th observation information acquired by the observation information acquiring section, and stores the n-th observation information assigned the search tag, in an archive section.

4. The cell observation information processing system according to claim 3, further comprising
a control section that controls the storing section so as to execute storing of the n-th observation information assigned the search tag, in the archive section, when accepting, from the user, the selection order on whether the work for the cells is the passage work.

5. The cell observation information processing system according to claim 4,
wherein the presenting section completes accepting, from the user, the selection order on whether the work for the cells at the n-th observation time point is the passage work, by input of a confirmation order from the user.

6. The cell observation information processing system according to claim 1,
wherein the passage presence/absence information acquiring and generating section comprises a passage presence/absence determining section that determines whether the work for the cells at the n-th observation time point is the passage work based on passage presence/absence determining information at the n-th observation time point, and generates a result of determination by the passage presence/absence determining section on whether the work is the passage work, as an n-th passage presence/absence value.

7. The cell observation information processing system according to claim 6,
wherein the passage presence/absence determining information is the activity data indicating the activity of the cells.

8. The cell observation information processing system according to claim 7,
wherein the activity data is the number of cells or confluency, and
the passage presence/absence determining section determines that the work for the cells at the n-th observation time point is non-passage work when the number of cells or confluency at the n-th observation time point is lower than a predetermined upper threshold.

9. The cell observation information processing system according to claim 7,
wherein the activity data is the number of cells or confluency, and
the passage presence/absence determining section determines that the work for the cells at the n-th observation time point is the passage work when the number of cells or confluency at the n-th observation time point is at least a predetermined upper threshold.

10. The cell observation information processing system according to claim 6, further comprising
a storing section that assigns the n-th passage presence/absence value acquired by the passage presence/absence information acquiring and generating section, as a search tag, to the n-th observation information generated by the observation information acquiring section, and stores the n-th observation information assigned the search tag, in the archive section.

11. The cell observation information processing system according to claim 1,
wherein the passage presence/absence information acquiring and generating section comprises a passage presence/absence determining section that determines whether the work for the cells at the (n-1)-th observation time point is the passage work based on passage presence/absence determining information at the n-th observation time point, and generates a result of determination by the passage presence/absence determining section on whether the work is the passage work, as the previous passage presence/absence information.

12. The cell observation information processing system according to claim 11,
wherein the passage presence/absence determining information is the activity data indicating an activity of the cells.

13. The cell observation information processing system according to claim 12,
wherein the activity data is the number of cells or confluency, and
the passage presence/absence determining section determines that the work for the cells at the (n-1)-th observation time point is the passage work when the number of cells or confluency at the n-th observation time point is lower than a predetermined lower threshold.

14. The cell observation information processing system according to claim 12,
wherein the activity data is the number of cells or confluency, and
the passage presence/absence determining section determines that the work for the cells at the (n-1)-th observation time point is non-passage work when the number of cells or confluency at the n-th observation time point is at least a predetermined lower threshold.

15. The cell observation information processing system according to claim 12,
wherein the activity data is the number of cells or confluency, and
the passage presence/absence determining section determines that the work for the cells at the (n-1)-th observation time point is the passage work when the number of cells or confluency of the cells at the (n-1)-th observation time point is at least a predetermined upper threshold and the number of cells or confluency at the n-th observation time point is lower than a predetermined lower threshold.

16. The cell observation information processing system according to claim 12,
wherein the activity data is the number of cells or confluency, and
the passage presence/absence determining section determines that the work for the cells at the (n-1)-th observation time point is the passage work when the number of cells or confluency of the cells at the n-th observation time point is decreased by at least a predetermined threshold of difference in comparison with the number of cells or confluency at the (n-1)-th observation time point.

17. The cell observation information processing system according to claim 1, further comprising
a storing section that assigns the first to (n-1)-th passage presence/absence values acquired or generated by the passage presence/absence information acquiring and generating section, as search tags, to the respective first to (n-1)-th pieces of observation information acquired by the observation information acquiring section, and stores the first to (n-1)-th pieces of observation information assigned the search tags in the archive section.

18. The cell observation information processing system according to claim 17,
wherein the storing section assigns the first to (n-1) -th pieces of observation information respective pieces of cell identifying information for identifying cells indicated by the pieces of the observation information, as search tags, and stores the first to (n-1)-th pieces of observation information assigned the search tags in the archive section,
the passage presence/absence information acquiring and generating section acquires the previous passage presence/absence information on same cells from the archive section, based on tags carrying the cell identifying information, and
the passage number acquiring and generating section acquires or generates the n-th passage number, based on the previous passage presence/absence information on the same cells acquired or generated by the passage presence/absence information acquiring and generating section.

19. The cell observation information processing system according to claim 18,
wherein the storing section further assigns the first to (n-1)-th pieces of observation information respective pieces of user identifying information for identifying users having observed the piece of the observation information, as search tags, and stores the first to (n-1)-th pieces of observation information assigned the search tags in the archive section,
the passage presence/absence information acquiring and generating section acquires or generates the previous passage presence/absence information associated with the same cells and the same user from the archive section, based on tags carrying the cell identifying information and tags carrying the user identifying information, and
the passage number acquiring and generating section acquires or generates the n-th passage number, based on the previous passage presence/absence information associated with the same cells and same user and acquired or generated by the passage presence/absence information acquiring and generating section.

20. The cell observation information processing system according to claim 1, further comprising
a passage presence/absence information output section that outputs, to the presenting section, the previous passage presence/absence information acquired or generated by the passage presence/absence information acquiring and generating section,
wherein the presenting section is configured to present, to a user, the previous passage presence/absence information output by the passage presence/absence information output section, and accepts an input of a passage number from the user, and
the passage number acquiring and generating section acquires the passage number input through the presenting section by the user having referred to the previous passage presence/absence information presented by the presenting section, as the n-th passage number.

21. The cell observation information processing system according to claim 20,
wherein the passage presence/absence information output section further outputs, to the presenting section, history information on passage/non-passage work indicated by the previous passage presence/absence information,
the presenting section further presents, to the user, the history information output by the passage presence/absence information output section, and
the passage number acquiring and generating section acquires the passage number input through the presenting section by the user having referred to the previous passage presence/absence information and the history information presented by the presenting section, as the n-th passage number.

22. The cell observation information processing system according to claim 21,
wherein the history information includes at least either of dates indicated by the previous passage presence/absence information as dates when the passage work was conducted and passage numbers at the dates indicated by the previous passage presence/absence information as the dates when the passage work was conducted.

23. The cell observation information processing system according to claim 20,
wherein the passage number acquiring and generating section automatically generates the n-th passage number for checking an input, based on the previous passage presence/absence information besides the passage number input by the user, and
the cell observation information processing system further comprises a notification section that can notify the user of warning information on the input of the passage number, based on the passage number input by the user and the n-th passage number automatically generated by the passage number acquiring and generating section for checking the input.

24. The cell observation information processing system according to claim 23,
wherein the notification section notifies the user of the warning information when the passage number input by the user and the n-th passage number automatically generated by the passage number acquiring and generating section for checking the input are different from each other.

25. The cell observation information processing system according to claim 1,
wherein the passage number acquiring and generating section generates previous passage numbers including passage numbers at previous observation time points, the passage presence/absence values contained in the previous passage presence/absence information at the previous observation time points having been acquired or generated by the passage presence/absence information acquiring and generating section, and
further generates the n-th passage number, based on the previous passage presence/absence information and the previous passage numbers.

26. The cell observation information processing system according to claim 25,
wherein the previous passage presence/absence information includes the (n-1)-th passage presence/absence value at the (n-1)-th observation time point,
the previous passage number includes the (n-1)-th passage number at the (n-1)-th observation time point, and
the passage number acquiring and generating section generates the n-th passage number based on the (n-1)-th passage presence/absence value and the (n-1)-th passage number.

27. The cell observation information processing system according to claim 26,
wherein the passage number acquiring and generating section generates, as the n-th passage number, a value acquired by adding one to the (n-1)-th passage number, when the (n-1)-th passage presence/absence value indicates the passage work.

28. The cell observation information processing system according to claim 26,
wherein the passage number acquiring and generating section generates, as the n-th passage number, a value identical to the (n-1)-th passage number, when the (n-1)-th passage presence/absence value indicates non-passage work.

29. The cell observation information processing system according to claim 25,
wherein the previous passage presence/absence information includes the (n-1)-th passage presence/absence value at the (n-1)-th observation time point and an (n-2)-th or smaller ordinal-numbered passage presence/absence value at an (n-2) -th or earlier observation time point, and
the passage number acquiring and generating section identifies an observation time point at which a most recent passage work for the cells was conducted among the previous observation time points, and
generates the n-th passage number, based on the passage number at the observation time point identified by the most recent passage work.

30. The cell observation information processing system according to claim 29,
wherein the passage number acquiring and generating section generates, as the n-th passage number, a value acquired by adding one to the passage number at the observation time point identified by the most recent passage work.

31. The cell observation information processing system according to claim 25,
wherein the previous passage presence/absence information includes at least one passage presence/absence value each represented by a k-th passage presence/absence value at each observation time point represented by a k-th observation time point on or before the (n-1)-th observation time point,
the passage number acquiring and generating section identifies whether work for the cells at each k-th observation time point is the passage work, based on each k-th passage presence/absence value, and
the cell observation information processing system further comprises a warning information output section that can output warning information to the presenting section, based on elapsed days from a most recent observation time point at which the passage work is identified to have been conducted by the passage number acquiring and generating section among the k-th observation time points to the n-th observation time point, and a predetermined reference number of days, and
the presenting section presents, to the user, the warning information output by the warning information output section.

32. The cell observation information processing system according to claim 31,
wherein the warning information output section outputs the warning information to the presenting section, when the elapsed days from the most recent observation time point at which the passage work is identified to have been conducted by the passage number acquiring and generating section to the n-th observation time point is at least the predetermined reference number of days.

33. The cell observation information processing system according to claim 31,
wherein the predetermined reference number of days is a reference value of cell passage interval days.

34. The cell observation information processing system according to claim 33,
wherein the reference value of the cell passage interval days is defined based on previous cell passage interval days.

35. The cell observation information processing system according to claim 33,
wherein the reference value of the cell passage interval days is predefined according to each kind of the cells.

36. The cell observation information processing system according to claim 1,
wherein the passage number acquiring and generating section acquires or generates the n-th passage number based on the previous passage presence/absence information every time the n-th observation information is sequentially acquired by the observation information acquiring section.

37. The cell observation information processing system according to claim 1,
wherein the passage presence/absence information acquiring and generating section acquires or generates in bulk at least one among the first to (n-1)-th passage presence/absence values included in the previous passage presence/absence information.

38. The cell observation information processing system according to claim 1,
wherein the passage number acquiring and generating section acquires or generates in bulk at least one of the first to n-th passage numbers at at least one of the first to n-th observation time points, based on the previous passage presence/absence information.

39. The cell observation information processing system according to claim 1, further comprising
a storing section that assigns the n-th passage number acquired or generated by the passage number acquiring and generating section, as a search tag, to the n-th observation information, and stores the n-th observation information assigned the search tag, in the archive section.

40. A cell observation information processing method, comprising:
a passage presence/absence information acquiring and generating step of acquiring or generating previous passage presence/absence information including at least one passage presence/absence value among first to (n-1) -th passage presence/absence values indicating whether work for cells was passage work at first to (n-1)-th observation time points, among first to n-th observation time points at which first to n-th pieces of observation information each indicating a cell observation result including at least one of a cell image, and activity data indicating an activity of cells by at least one of the number of cells, confluency, morphology, and viability are acquired in a time series manner through an observation information acquiring section, where n is an integer equal to or greater than two and a current observation time point is represented by the n-th observation time point; and
a passage number acquiring and generating step of acquiring or generating at least an n-th passage number at the n-th observation time point automatically or through an operation by a user, based on the acquired or generated previous passage presence/absence information.

41. A cell observation information processing program causing a computer to function as:
a passage presence/absence information acquiring and generating means for acquiring or generating previous passage presence/absence information including at least one passage presence/absence value among first to (n-1) -th passage presence/absence values indicating whether work for cells was passage work at first to (n-1)-th observation time points, among first to n-th observation time points at which first to n-th pieces of observation information each indicating a cell observation result including at least one of a cell image, and activity data indicating an activity of cells by at least one of the number of cells, confluency, morphology, and viability are acquired in a time series manner through an observation information acquiring section; and
a passage number acquiring and generating means for acquiring or generating an n-th passage number at the n-th observation time point automatically or through an operation by a user, based on the previous passage presence/absence information acquired by the passage presence/absence information acquiring and generating means.

42. An archive section provided for the cell observation information processing system according to claim 3, containing one or more records of the search-tagged observation information,
wherein each of the records is created according to image capture of a cell image at a corresponding time point, and
observation information corresponding to the image capture of the cell image at one time point is associated with the search tag for retrieving the observation information.

43. An archive section provided for the cell observation information processing system according to claim 10, including one or more records of the search-tagged observation information,
wherein each of the records is created according to image capture of a cell image at a corresponding time point, and
observation information corresponding to the image capture of the cell image at one time point is associated with the search tag for retrieving the observation information.

44. An archive section provided for the cell observation information processing system according to claim 17, including one or more records of the search-tagged observation information,
wherein each of the records is created according to image capture of a cell image at a corresponding time point, and
observation information corresponding to the image capture of the cell image at one time point is associated with the search tag for retrieving the observation information.

45. An archive section provided for the cell observation information processing system according to claim 39, including one or more records of the search-tagged observation information,
wherein each of the records is created according to image capture of a cell image at a corresponding time point, and
observation information corresponding to the image capture of the cell image at one time point is associated with the search tag for retrieving the observation information.

46. A plurality of apparatuses provided for the cell observation information processing system according to claim 1,
wherein the passage presence/absence information acquiring and generating section and the passage number acquiring and generating section are separately arranged in the plurality of the apparatuses.

47. The cell observation information processing system according to claim 3, further comprising:
a search tag output section that outputs the search tag to the presenting section; and
the presenting section that presents, to the user, the search tag output by the search tag output section.

48. The cell observation information processing system according to claim 10, further comprising:
a search tag output section that outputs the search tag to the presenting section; and
the presenting section that presents, to the user, the search tag output by the search tag output section.

49. The cell observation information processing system according to claim 17, further comprising:
a search tag output section that outputs the search tag to the presenting section; and
the presenting section that presents, to the user, the search tag output by the search tag output section.

50. The cell observation information processing system according to claim 39, further comprising
a search tag output section that outputs the search tag to the presenting section; and
the presenting section that presents, to the user, the search tag output by the search tag output section.
